# EUROPEAN PATENT APPLICATION

(11) **EP 4 141 033 A1**
(43) Date of publication of application: **01.03.2023**
(21) Application number: 21792313.5
(22) Date of filing: 22.04.2021
(51) Int. Cl.: C07K 16/46, A61K 39/395, A61P 35/00

(54) **ANTI-CD73-ANTI-PD-1 BISPECIFIC ANTIBODY AND USE THEREOF**

(30) Priority: 22.04.2020 CN 202010324783; 12.03.2021 CN 202110270671
(71) Applicant: Akeso Biopharma Inc., Zhongshan, Guangdong 528437 (CN)
(72) Inventor: ZHANG, Peng, Zhongshan Guangdong 528437 (CN); LI, Baiyong, Zhongshan Guangdong 528437 (CN); XIA, Yu, Zhongshan Guangdong 528437 (CN); WANG, Zhongmin, Zhongshan Guangdong 528437 (CN)
(74) Representative: Nederlandsch Octrooibureau
(86) International application number: PCT/CN2021/089059
(87) International publication number: WO 2021/213475

(57) **Abstract**

Provided are an anti-CD73-anti-PD-1 bispecific antibody, a pharmaceutical composition thereof and a use thereof.

## Description

### TECHNICAL FIELD

The present invention relates to the fields of tumor treatment and molecular immunology, and particularly to an anti-CD73/anti-PD-1 bispecific antibody, a pharmaceutical composition thereof, and use thereof.

### BACKGROUND

Ecto-5'-nucleotidase, namely CD73 protein, is a multifunctional glycoprotein encoded by NT5E gene and having a molecular weight of 70 KD, which is anchored on a cell membrane by glyocsyl phosphatidy linositol (GPI) (Zimmermann H., 5'-Nucleotidase: molecular structure and functional aspects., Biochem J., 1992; 285:345-365).

CD73 is widely distributed on the surface of human tissue cells, and it has been found in research that CD73 is highly expressed in various solid tumors, specifically in cancer cells, dendritic cells, regulatory T cells (Tregs), natural killer cells (NK cells), myeloid-derived suppressor cells (MDSCs), tumor-associated macrophages (TAMs) and the like in a tumor micro environment. The expression of CD73 is regulated by TGF-β, EGFR, AKT, β-catenin and other molecules, especially HIF-1, which exerts the function of a transcription factor, is the most critical. An important feature of the tumor microenvironment is hypoxia, which induces the up-regulation of hypoxia-inducible factor-1 (HIF-1) and other molecules, thereby leading to the widespread expression of CD73 in the tumor micro environment (Synnestvedt K, et al., Ecto-5'-nucleotidase (CD73) regulation by hypoxia-inducible factor-1 mediates permeability changes in intestinal epithelia. J Clin Invest., 2002; 110:993-1002.). Analysis of clinical tumor samples has shown that high expression of CD73 is a potential biomarker and is closely related to adverse prognosis of various types of tumors, including breast cancer, lung cancer, ovarian cancer, kidney cancer, gastric cancer, head and neck cancer and the like. CD73 has hydrolase activity and non-hydrolase activity. The enzyme and non-enzyme functions of CD73 simultaneously work in the related process in tumors, and mutually promote and maintain the progression of tumors. More and more studies have found that CD73 is a key regulatory molecule for tumor cell proliferation, metastasis and invasion *in vitro*, and tumor angiogenesis and tumor immune escape mechanism *in vivo*, wherein an important mechanism of immune suppression is mediated by CD73-adenosine metabolic signaling pathway. CD39 at the upstream of CD73 can catalyze ATP to generate adenosine monophosphate (AMP), the generated AMP is converted into adenosine by CD73, and adenosine binds to a downstream adenosine receptor (A2AR). A2AR inhibits a series of signaling pathways related to immune activation, such as LCK, MAPK, PKC, and inhibits the immune killing effect of T cells by activating protein kinase A (PKA) and Csk kinase, thereby playing an immune suppression role to enable the tumor to achieve immune escape (Antonioli L, et al., Immunity, inflammation and cancer: a leading role for adenosine. Nat Rev Cancer., 2013; 13:842-857). Preclinical animal model studies have shown that CD73 expressed in immune cells and non-immune cells can promote the immune escape, development and metastasis of tumors, wherein the inhibition of cytotoxic T cell (CTL) and NK cell functions by Treg cell-related CD73-adenosine signals is the most significant.

For the treatment of solid tumors, one important aspect of overcoming drug resistance and improving the therapeutic effect is to relieve the inhibition effect of the tumor micro environment (TME) on immune effector cells. TME is a very complex system composed of various cells, intercellular matrix, enzymes, cytokines, metabolites, etc. It features significant low hydrogen, low pH and high pressure, and is greatly different from normal tissues. Hypoxia or ATP enrichment caused by chemoradiotherapy for killing tumor cells promotes the cascade reaction of CD39-CD73 adenosine signals, which is beneficial to the proliferation and function of various cancer-promoting cells, but is not beneficial to cancer-inhibiting cells. (Regateiro, F. S., Cobbold, S. P. & Waldmann, H., CD73 and adenosine generation in the creation of regulatory microenvironments. Clin. Exp. Immunol., 2013; 171:1-7).

The use of antibodies targeting CD73 or gene knock-out of CD73 in animal models can effectively block the growth and metastasis of tumors. Recently, the use of CD73 monoclonal antibody, small interfering RNA technology, specific inhibitor APCP and the like has achieved remarkable therapeutic effect in anti-tumor treatment of animal experiments, providing a new way for anti-tumor treatment. Evidence from *in vivo* studies has shown that targeted blockade of CD73 will be an effective treatment means for tumor patients.

The relation between CD73 overexpression and tumor subtype, prognosis and response in patients has shown that CD73 can be an important marker for future tumor treatment and detection of individuals. Therefore, the study of the CD73 target is indispensable.

The transmembrane receptor PD-1 (programmed cell death protein 1) is a member of the CD28 family, and is expressed in activated T cells, B cells and myeloid cells. The receptors of PD-1, PDL1 and PDL2, are members of the B7 superfamily. PDL1 is expressed in a variety of cells including T cells, B cells, endothelial cells and epithelial cells, and PDL2 is expressed only in antigen presenting cells such as dendritic cells and macrophages.

PD-1 plays a very important role in down-regulating the activation of T cells, and the PD-1-mediated down-regulation of T cells is one of the important mechanisms of tumor immune escape. PDL-1 expressed on the surface of tumors can bind to PD-1 on the surface of immune cells, thereby inhibiting the killing of tumor tissues by the immune cells through the PD-1/PDL-1 signaling pathway, and tumors with high expression of PD-L1 are associated with cancers that are difficult to detect (Hamanishi et al., Proc. Natl. Acad. Sci. USA, 2007; 104:3360-5). An effective way to antagonize PD-1 and thus inhibit PD-1/PDL-1 signaling pathway is injection of anti-PDL-1 antibody.

Due to the broad anti-tumor prospect and surprising efficacy of PD-1 antibodies, it is widely accepted that antibodies targeting the PD-1 pathway will bring about breakthroughs in the treatment of a variety of tumors: non-small cell lung cancer, renal cell carcinoma, ovarian cancer and melanoma (Hornet M. B., Parisi G., et al., Anti-PD-1 therapy in melanoma. Semin Oncol., 2015 Jun; 42(3):466-473), and hematological tumor and anemia (Held SA, Heine A, et al., Advances in immunotherapy of chronic myeloid leukemia CML. Curr Cancer Drug Targets, 2013 Sep; 13(7):768-74).

Bifunctional antibodies, also known as bispecific antibodies, are specific antibody drugs that target two different antigens simultaneously, and can be produced by immunosorting and purification, or can be obtained by genetic engineering. The genetic engineering has flexibility in aspects of binding site optimization, synthetic form, yield and the like, thus having certain advantages. Currently, over 45 forms of the bispecific antibody have been demonstrated (Müller D, Kontermann RE. Bispecific antibodies for cancer immunotherapy: current perspectives. BioDrugs 2010; 24:89-98). The IgG-ScFv form, namely the Morrison form (Coloma MJ, Morrison SL. Design and production of novel tetravalent bispecific antibodies. Nat Biotechnol. Nature Biotechnology, 1997; 15:159-163), has been demonstrated to be an ideal form of the bifunctional antibody due to its similarity to the naturally existing IgG form and advantages in antibody engineering, expression and purification (Miller BR, Demarest SJ, et al., Stability engineering of scFvs for the development of bispecific and multivalent antibodies. Protein Eng Des Sel 2010; 23:549-57; Fitzgerald J, Lugovskoy A. Rational engineering of antibody therapeutics targeting multiple oncogene pathways. MAbs 2011; 3:299-309).

ADCC (antibody-dependent cell-mediated cytotoxicity) refers to killing of a target cell by a killer cell (NK cell, macrophage, etc.) that is mediated by binding of the Fab fragment of an antibody to an epitope of a virus-infected cell or a tumor cell and binding of the Fc fragment of the antibody to an Fc receptor (FcR) on the surface of the killer cell.

CDC (complement dependent cytotoxicity) refers to a lytic effect on target cells by a membrane-attacking complex that is formed by the bindings of an antibody to a corresponding antigen on a cell membrane surface and later to the complement C1q and activation of C2-C9.

The IgG family comprises four members, IgG1, IgG2, IgG3 and IgG4, which differ in amino acids in the fragment crystallizable (Fc) region of the heavy chain constant region, resulting in their varying affinities for FcyRs. Wild-type IgG1 can bind to various FcyRs and elicit ADCC and CDC effects. Zhang et al. (Zhang T et al, Cancer Immunol Immunother., 2018; 67(7): 1079-1090.) and Dahan et al. (Dahan R et al., Cancer cell, 2015, 28(3):285-95.) reported that the binding of Fc fragments of antibodies targeting immune checkpoints such as PD-1 to Fc receptors negatively affects antibody-mediated anti-cancer activity, possibly because Fc-dependent effector function-induced immune cell damage, including antibody-dependent cell-mediated cytotoxicity, is an important mechanism leading to immune cell damage.

Interleukin-8 (IL-8) is a chemotactic cytokine and is mainly secreted by monocytes and the like. IL-8 plays an important role in the proliferation of normal cells and tumor cells, especially in promoting the development and progression of tumors. Studies have shown that IL-8 can promote the development of tumors; and the tumor cells themselves also secrete IL-8 to promote tumor growth and metastasis (Lo MC et al., Cancer letters, 2013, 335(1):81-92). Therefore, IL-8 has become an important inflammatory factor indispensable in the tumor micro environment.

As a pro-inflammatory factor, IL-8 is closely related to the development and progression of tumors. During the methylarsonate-induced malignant transformation of non-renal cancer cells, the expression of IL-8 gene is increased. Gene silencing of IL-8 can significantly inhibit the growth of transplanted tumors in mice, and in addition, the reduction of IL-8 level can inhibit the expression of matrix metalloproteinase-9, cyclin D1, pro-apoptotic protein Bcl-2 and vascular endothelial growth factor (VEGF), which are related to the growth and metastasis of tumors (Escudero-Lourdes C et al., Toxicology and applied pharmacology, 2012, 258(1): 10-18). Inoue et al. found that IL-8 can induce malignant transformation of non-neoplastic bladder cell line (233JP) and increases its aggressiveness, while the incidence of malignant transformation of 233JP cells is significantly reduced in IL-8 knock-out mice (Inoue K et al., Cancer Res, 2000, 60(8):2290-2299). Furthermore, in prostate cancer, IL-8 can promote the development of castration-resistant prostate cancer (CRPC) in patients (Chen K et al., Cancer research, 2015, 75(10):1992-2004), and is associated with drug resistance to tumor treatment (Araki S et al., Cancer Res, 2007, 67(14):6854-6862); gene silencing of IL-8 or its receptor can induce cell cycle arrest in tumor cells and inhibit tumor proliferation (Singh RK, Lokeshwar BL., Molecul Cancer, 2009, 8:57). The above studies have shown that the level of IL-8 is closely related to the development and progression of tumors. Further studies (Mian BM et al. Clin Cancer Res, 2003, 9(8):3167-3175) have shown that IL-8 can be a novel target for tumor treatment. In a tumor model of bladder cancer, the use of anti-IL-8 antibodies can significantly inhibit the growth of tumors.

IL-6 is mainly produced rapidly by macrophages in response to pathogen-associated molecular patterns (PAMPs) or damage-associated molecular patterns (DAMPs), and plays a protective role by removing infectious agents and inducing acute phase and immune responses to cure the damaged tissues. Although IL-6 plays an important role in infection and the resistance to and repair of tissue damage, a high level of IL-6 can activate the coagulation pathway and vascular endothelial cells, thereby inhibiting myocardial function, and can even cause a "cytokine storm", resulting in severe acute systemic inflammatory responses. Cytokine storm is a fatal complication and adverse effect in viral infection, tumor immunotherapy and the like.

Immune-related adverse effect is a common and dangerous adverse effect in the anti-tumor treatment with immune checkpoint inhibitors (ICIs) (Spain L et al., Cancer Treat Rev., 2016; 44:51-60). In recent years, immune checkpoint inhibitors have achieved great success in tumor immunotherapy, but also led to a brand-new toxicity profile due to off-target effects, among which the severe immune-related adverse events (irAEs) in major organs (including heart, lung and brain) are especially life-threatening (Bergqvist V, et al., Cancer Immunol Immunother., 2017; 66(5):581-592; Gomatou G et al., Respiration., 2020; 1:1-11; Joshi MN et al., Clin Endocrinol (Oxf)., 2016; 85(3):331-9; Prieux-Klotz C et al., Target Oncol., 2017; 12(3):301-308; Tajiri K et al., Jpn J Clin Oncol., 2018; 48(1):7-12). Existing data have shown that ICIs can induce off-target effects by 4 mechanisms, including direct binding to immune checkpoint molecules expressed on the surface of normal cells, and activating complement hypersensitivity; the presence of homologous antigens/epitopes in normal tissues and tumor cells; producing autoantibodies; increasing the level of pro-inflammatory cytokines, such as IL-6 and the like (Martins F et al., The Lancet Oncology, 20(1), e54-e64).

Currently, anti-IL-6 therapy, such as tocilizumab, a recombinant humanized anti-IL-6R monoclonal antibody, has been used to treat severe irAEs, severe or refractory arthritis, large vessel vasculitis, uveitis, myocarditis, pneumonia, myasthenia gravis and the like in the acute phase (Martins F et al., The Lancet Oncology, 20(1), e54-e64).

Binding of FcyRIa on macrophages to wild-type IgG1 or IgG4 antibodies can induce the macrophages to secrete IL-8 and IL-6 (Kinder M et al., mAbs., 2015), while inducing mutations in the Fc segments of antibodies and eliminating their binding to FcyRIa can effectively inhibit the secretion of IL-8, thereby improving the safety and efficacy of the antibodies.

### SUMMARY

The inventors used mammalian cell expression systems to express recombinant human CD73 and PD-1 as antigens to immunize mice, and obtained hybridoma cells by fusion of mouse spleen cells and myeloma cells. The inventors obtained the following hybridoma cell lines by screening a large number of the samples:
hybridoma cell line LT014 (also called CD73-19F3) deposited at China Center for Type Culture Collection (CCTCC) on June 19, 2018 with an accession number of CCTCC NO: C2018137; and
hybridoma cell line LT003 (also called PD-1-14C12) deposited at China Center for Type Culture Collection (CCTCC) on June 16, 2015 with an accession number of CCTCC NO: C2015105.

The inventors surprisingly found that:
the hybridoma cell line LT014 can secrete a specific monoclonal antibody (named as 19F3) specifically binding to human CD73, and the monoclonal antibody can effectively inhibit the enzyme activity reaction of CD73 in a non-substrate competition mode, reduce the production of adenosine, and promote the activity and the tumor inhibitory effect of T cells; and
the hybridoma cell line LT003 may secrete a specific monoclonal antibody (named as 14C12) specifically binding to PD-1, and the monoclonal antibody can effectively block the binding of PD-1 to PDL1.

Furthermore, the inventors creatively prepared humanized anti-CD73 antibodies (named as 19F3H2L2, 19F3H2L3, 19F3H2L3(hG1M) and 19F3H2L3(hG1TM), respectively) and humanized anti-PD-1 antibodies (named as 14C12H1L1 and 14C12H1L1(hG1TM)). Furthermore, the inventors creatively fused two types of humanized antibodies into new antibodies through protein recombination, and obtained humanized bifunctional antibodies (named as P1D7V01, P1D7V03, NTPDV1, NTPDV2, NTPDV3 and NTPDV4, respectively (also written as NTPDV1(hG1TM), NTPDV2(hG1TM), NTPDV3(hG1TM) and NTPDV4(hG1TM) herein and in the Chinese Patent Application NO. 202110270671.X)) capable of binding to CD73 and PD-1, inhibiting the activity of CD73 and blocking the binding of PD-1 to PDL1, and having the potential for use in preparing a medicament for preventing and treating of solid tumors and hematological tumors.

The present invention is detailed below.

One aspect of the present invention relates to an anti-CD73/anti-PD-1 bispecific antibody comprising:
a first protein functional region targeting PD-1, and
a second protein functional region targeting CD73.

In one embodiment of the present invention, in the bispecific antibody,
the first protein functional region comprises: HCDR1, HCDR2 and HCDR3 contained in a heavy chain variable region having an amino acid sequence set forth in SEQ ID NO: 44, wherein preferably the amino acid sequences of HCDR1, HCDR2 and HCDR3 are sequences set forth in SEQ ID NOs: 45-47, respectively, or sequences having at least 80%, 81%, 82%, 83%, 84%, 85%, 86%, 87%, 88%, 89% or 90%, preferably at least 91%, 92%, 93%, 94%, 95%, 96%, 97%, 98% or 99% sequence identity to the sequences set forth in SEQ ID NOs: 45-47, or amino acid sequences having one or more (preferably 1, 2 or 3) conservative amino acid mutations (preferably substitutions, insertions or deletions) compared with the sequences set forth in SEQ ID NOs: 45-47; and
LCDR1, LCDR2 and LCDR3 contained in a light chain variable region having an amino acid sequence set forth in SEQ ID NO: 49, wherein preferably the amino acid sequences of LCDR1, LCDR2 and LCDR3 are sequences set forth in SEQ ID NOs: 50-52, respectively, or sequences having at least 80%, 81%, 82%, 83%, 84%, 85%, 86%, 87%, 88%, 89% or 90%, preferably at least 91%, 92%, 93%, 94%, 95%, 96%, 97%, 98% or 99% sequence identity to the sequences set forth in SEQ ID NOs: 50-52, or amino acid sequences having one or more (preferably 1, 2 or 3) conservative amino acid mutations (preferably substitutions, insertions or deletions) compared with the sequences set forth in SEQ ID NOs: 50-52;
the second protein functional region comprises: HCDR1, HCDR2 and HCDR3 contained in a heavy chain variable region having an amino acid sequence set forth in SEQ ID NO: 2, wherein preferably the amino acid sequences of HCDR1, HCDR2 and HCDR3 are sequences set forth in SEQ ID NOs: 3-5, or sequences having at least 80%, 81%, 82%, 83%, 84%, 85%, 86%, 87%, 88%, 89% or 90%, preferably at least 91%, 92%, 93%, 94%, 95%, 96%, 97%, 98% or 99% sequence identity to the sequences set forth in SEQ ID NOs: 3-5, or amino acid sequences having one or more (preferably 1, 2 or 3) conservative amino acid mutations (preferably substitutions, insertions or deletions) compared with the sequences set forth in SEQ ID NOs: 3-5; and
LCDR1, LCDR2 and LCDR3 contained in a light chain variable region having an amino acid sequence set forth in SEQ ID NO: 7, wherein preferably the amino acid sequences of LCDR1, LCDR2 and LCDR3 are sequences set forth in SEQ ID NOs: 8-10, respectively, or sequences having at least 80%, 81%, 82%, 83%, 84%, 85%, 86%, 87%, 88%, 89% or 90%, preferably at least 91%, 92%, 93%, 94%, 95%, 96%, 97%, 98% or 99% sequence identity to the sequences set forth in SEQ ID NOs: 8-10, or amino acid sequences having one or more (preferably 1, 2 or 3) conservative amino acid mutations (preferably substitutions, insertions or deletions) compared with the sequences set forth in SEQ ID NOs: 8-10.

In one embodiment of the present invention, in the anti-CD73/anti-PD-1 bispecific antibody,
the first protein functional region comprises:
   a sequence having an amino acid sequence set forth in SEQ ID NO: 44 or SEQ ID NO: 62, or a sequence having at least 80%, 81%, 82%, 83%, 84%, 85%, 86%, 87%, 88%, 89% or 90%, preferably at least 91%, 92%, 93%, 94%, 95%, 96%, 97%, 98% or 99% sequence identity to the sequence set forth in SEQ ID NO: 44 or 62, or an amino acid sequence having one or more (preferably 1, 2 or 3) conservative amino acid mutations (preferably substitutions, insertions or deletions) compared with the sequences set forth in SEQ ID NO: 44 or 62; and
   a sequence having an amino acid sequence correspondingly set forth in SEQ ID NO: 49 or SEQ ID NO: 64, or a sequence having at least 80%, 81%, 82%, 83%, 84%, 85%, 86%, 87%, 88%, 89% or 90%, preferably at least 91%, 92%, 93%, 94%, 95%, 96%, 97%, 98% or 99% sequence identity to the sequence set forth in SEQ ID NO: 49 or 64, or an amino acid sequence having one or more (preferably 1, 2 or 3) conservative amino acid mutations (preferably substitutions, insertions or deletions) compared with the sequences set forth in SEQ ID NO: 49 or 64;
   and/or,
the second protein functional region comprises a sequence having an amino acid sequence set forth in SEQ ID NO: 2 or SEQ ID NO: 20, or a sequence having at least 80%, 81%, 82%, 83%, 84%, 85%, 86%, 87%, 88%, 89% or 90%, preferably at least 91%, 92%, 93%, 94%, 95%, 96%, 97%, 98% or 99% sequence identity to the sequence set forth in SEQ ID NO: 2 or 20, or an amino acid sequence having one or more (preferably 1, 2 or 3) conservative amino acid mutations (preferably substitutions, insertions or deletions) compared with the sequences set forth in SEQ ID NO: 2 or 20; and
a sequence having an amino acid sequence correspondingly set forth in SEQ ID NO: 7 or SEQ ID NO: 22, or a sequence having at least 80%, 81%, 82%, 83%, 84%, 85%, 86%, 87%, 88%, 89% or 90%, preferably at least 91%, 92%, 93%, 94%, 95%, 96%, 97%, 98% or 99% sequence identity to the sequence set forth in SEQ ID NO: 7 or 22, or an amino acid sequence having one or more (preferably 1, 2 or 3) conservative amino acid mutations (preferably substitutions, insertions or deletions) compared with the sequences set forth in SEQ ID NO: 7 or 22;
   or
the second protein functional region comprises a sequence having an amino acid sequence set forth in SEQ ID NO: 20, or a sequence having at least 80%, 81%, 82%, 83%, 84%, 85%, 86%, 87%, 88%, 89% or 90%, preferably at least 91%, 92%, 93%, 94%, 95%, 96%, 97%, 98% or 99% sequence identity to the sequence set forth in SEQ ID NO: 20, or an amino acid sequence having one or more (preferably 1, 2 or 3) conservative amino acid mutations (preferably substitutions, insertions or deletions) compared with the sequences set forth in SEQ ID NO: 20; and
a sequence having an amino acid sequence set forth in SEQ ID NO: 24, or a sequence having at least 80%, 81%, 82%, 83%, 84%, 85%, 86%, 87%, 88%, 89% or 90%, preferably at least 91%, 92%, 93%, 94%, 95%, 96%, 97%, 98% or 99% sequence identity to the sequence set forth in SEQ ID NO: 24, or an amino acid sequence having one or more (preferably 1, 2 or 3) conservative amino acid mutations (preferably substitutions, insertions or deletions) compared with the sequences set forth in SEQ ID NO: 24.

One aspect of the present invention relates to an anti-CD73/anti-PD-1 bispecific antibody comprising:
a first protein functional region targeting CD73, and
a second protein functional region targeting PD-1.

In one embodiment of the present invention, in the bispecific antibody,
the first protein functional region comprises: HCDR1, HCDR2 and HCDR3 contained in a heavy chain variable region having an amino acid sequence set forth in SEQ ID NO: 2, wherein preferably the amino acid sequences of HCDR1, HCDR2 and HCDR3 are sequences set forth in SEQ ID NOs: 3-5, respectively, or sequences having at least 80%, 81%, 82%, 83%, 84%, 85%, 86%, 87%, 88%, 89% or 90%, preferably at least 91%, 92%, 93%, 94%, 95%, 96%, 97%, 98% or 99% sequence identity to the sequences set forth in SEQ ID NOs: 3-5, or amino acid sequences having one or more (preferably 1, 2 or 3) conservative amino acid mutations (preferably substitutions, insertions or deletions) compared with the sequences set forth in SEQ ID NOs: 3-5; and
LCDR1, LCDR2 and LCDR3 contained in a light chain variable region having an amino acid sequence set forth in SEQ ID NO: 7, wherein preferably the amino acid sequences of LCDR1, LCDR2 and LCDR3 are sequences set forth in SEQ ID NOs: 8-10, respectively, or sequences having at least 80%, 81%, 82%, 83%, 84%, 85%, 86%, 87%, 88%, 89% or 90%, preferably at least 91%, 92%, 93%, 94%, 95%, 96%, 97%, 98% or 99% sequence identity to the sequences set forth in SEQ ID NOs: 8-10, or amino acid sequences having one or more (preferably 1, 2 or 3) conservative amino acid mutations (preferably substitutions, insertions or deletions) compared with the sequences set forth in SEQ ID NOs: 8-10;
the second protein functional region comprises: HCDR1, HCDR2 and HCDR3 contained in a heavy chain variable region having an amino acid sequence set forth in SEQ ID NO: 44, wherein preferably the amino acid sequences of HCDR1, HCDR2 and HCDR3 are sequences set forth in SEQ ID NOs: 45-47, respectively, or sequences having at least 80%, 81%, 82%, 83%, 84%, 85%, 86%, 87%, 88%, 89% or 90%, preferably at least 91%, 92%, 93%, 94%, 95%, 96%, 97%, 98% or 99% sequence identity to the sequences set forth in SEQ ID NOs: 45-47, or amino acid sequences having one or more (preferably 1, 2 or 3) conservative amino acid mutations (preferably substitutions, insertions or deletions) compared with the sequences set forth in SEQ ID NOs: 45-47; and
LCDR1, LCDR2 and LCDR3 contained in a light chain variable region having an amino acid sequence set forth in SEQ ID NO: 49, wherein preferably the amino acid sequences of LCDR1, LCDR2 and LCDR3 are sequences set forth in SEQ ID NOs: 50-52, respectively, or sequences having at least 80%, 81%, 82%, 83%, 84%, 85%, 86%, 87%, 88%, 89% or 90%, preferably at least 91%, 92%, 93%, 94%, 95%, 96%, 97%, 98% or 99% sequence identity to the sequences set forth in SEQ ID NOs: 50-52, or amino acid sequences having one or more (preferably 1, 2 or 3) conservative amino acid mutations (preferably substitutions, insertions or deletions) compared with the sequences set forth in SEQ ID NOs: 50-52.

In one embodiment of the present invention, in the anti-CD73/anti-PD-1 bispecific antibody,
the first protein functional region comprises:
   a sequence having an amino acid sequence set forth in SEQ ID NO: 2 or SEQ ID NO: 20, or a sequence having at least 80%, 81%, 82%, 83%, 84%, 85%, 86%, 87%, 88%, 89% or 90%, preferably at least 91%, 92%, 93%, 94%, 95%, 96%, 97%, 98% or 99% sequence identity to the sequence set forth in SEQ ID NO: 2 or 20, or an amino acid sequence having one or more (preferably 1, 2 or 3) conservative amino acid mutations (preferably substitutions, insertions or deletions) compared with the sequences set forth in SEQ ID NO: 2 or 20; and
   a sequence having an amino acid sequence correspondingly set forth in SEQ ID NO: 7, SEQ ID NO: 22 or SEQ ID NO: 24, or a sequence having at least 80%, 81%, 82%, 83%, 84%, 85%, 86%, 87%, 88%, 89% or 90%, preferably at least 91%, 92%, 93%, 94%, 95%, 96%, 97%, 98% or 99% sequence identity to the sequence set forth in SEQ ID NO: 7, SEQ ID NO: 22 or SEQ ID NO: 24, or an amino acid sequence having one or more (preferably 1, 2 or 3) conservative amino acid mutations (preferably substitutions, insertions or deletions) compared with the sequences set forth in SEQ ID NO: 7, 22 or 24; and/or,
the second protein functional region comprises a sequence having an amino acid sequence set forth in SEQ ID NO: 44 or SEQ ID NO: 62, or a sequence having at least 80%, 81%, 82%, 83%, 84%, 85%, 86%, 87%, 88%, 89% or 90%, preferably at least 91%, 92%, 93%, 94%, 95%, 96%, 97%, 98% or 99% sequence identity to the sequence set forth in SEQ ID NO: 44 or SEQ ID NO: 62, or an amino acid sequence having one or more (preferably 1, 2 or 3) conservative amino acid mutations (preferably substitutions, insertions or deletions) compared with the sequences set forth in SEQ ID NO: 44 or SEQ ID NO: 62; and
a sequence having an amino acid sequence correspondingly set forth in SEQ ID NO: 49 or 64, or a sequence having at least 80%, 81%, 82%, 83%, 84%, 85%, 86%, 87%, 88%, 89% or 90%, preferably at least 91%, 92%, 93%, 94%, 95%, 96%, 97%, 98% or 99% sequence identity to the sequence set forth in SEQ ID NO: 49 or 64, or an amino acid sequence having one or more (preferably 1, 2 or 3) conservative amino acid mutations (preferably substitutions, insertions or deletions) compared with the sequences set forth in SEQ ID NO: 49 or 64.

In one embodiment of the present invention, in the anti-CD73/anti-PD-1 bispecific antibody, the first protein functional region and the second protein functional region are linked directly or via a linker; preferably, the linker is (GGGGS)n, and n is a positive integer, e.g., 1, 2, 3, 4, 5 or 6.

In one embodiment of the present invention, the first protein functional region and the second protein functional region in the anti-CD73/anti-PD-1 bispecific antibody are independently an immunoglobulin or an antigen-binding fragment, such as a half-antibody, Fab, F(ab')₂ or a single chain fragment variable, preferably, the first protein functional region is an immunoglobulin and the second protein functional region is an antigen-binding fragment; or the first protein functional region is an antigen-binding fragment and the second protein functional region is an immunoglobulin.

In one embodiment of the present invention, the N terminus of the heavy chain variable region of the antigen-binding fragment is linked directly (or via a linker) to the C terminus of CH1 of the immunoglobulin, and the N terminus of the light chain variable region of the antigen-binding fragment is linked directly (or via a linker) to the C terminus of the light chain variable region CL of the immunoglobulin; or the N terminus of the heavy chain variable region of the antigen-binding fragment is linked directly (or via a linker) to the C terminus of the light chain variable region CL of the immunoglobulin, and the N terminus of the light chain variable region of the antigen-binding fragment is linked directly (or via a linker) to the C terminus of the heavy chain variable region CH1 of the immunoglobulin.

In one embodiment of the present invention, the C terminus of the heavy chain variable region of the antigen-binding fragment is linked directly (or via a linker) to the N terminus of the heavy chain of the immunoglobulin, and the C terminus of the light chain variable region of the antigen-binding fragment is linked directly (or via a linker) to the N terminus of the light chain of the immunoglobulin; or the C terminus of the heavy chain variable region of the antigen-binding fragment is linked directly (or via a linker) to the N terminus of the light chain of the immunoglobulin, and the C terminus of the light chain variable region of the antigen-binding fragment is linked directly (or via a linker) to the N terminus of the heavy chain of the immunoglobulin.

In one embodiment of the present invention, the antigen-binding fragment is a single chain fragment variable; preferably, the first protein functional region is an immunoglobulin and the second protein functional region is a single chain fragment variable; or the first protein functional region is a single chain fragment variable and the second protein functional region is an immunoglobulin.

In one embodiment of the present invention, the bispecific antibody is provided, wherein the numbers of the first protein functional region and the second protein functional region are each independently 1, 2 or more.

In one embodiment of the present invention, in the anti-CD73/anti-PD-1 bispecific antibody, the single chain fragment variable is a molecule formed by connecting an antibody heavy chain variable region (V_{H}) and an antibody light chain variable region (V_{L}) via a linker; preferably, the single chain fragment variable may have a general structure: NH₂-V_{L}-linker-V_{H}-COOH or NH₂-V_{H}-linker-V_{L}-COOH.

In one embodiment of the present invention, in the anti-CD73/anti-PD-1 bispecific antibody, when the single chain fragment variable is linked to the C terminus of the heavy chain of the immunoglobulin (C_{H}) (or the N terminus of the heavy chain, the C terminal of CH1 of the heavy chain variable region) by a linker, the antibody heavy chain variable region (V_{H}) of the single chain fragment variable may be firstly linked, or the antibody light chain variable region (V_{L}) of the single chain fragment variable may be firstly linked; preferably, the single chain fragment variable may have a general structure: C_{H}-linker-V_{H}-linker-V_{L}-COOH, or C_{H}-linker-V_{L}-linker-V_{H}-COOH, preferably,
the heavy chain variable region of the immunoglobulin comprises CDRs having amino acid sequences set forth in SEQ ID NOs: 3-5, and the light chain variable region of the immunoglobulin comprises CDRs having amino acid sequences set forth in SEQ ID NOs: 8-10;
the heavy chain variable region of the single chain fragment variable comprises CDRs having amino acid sequences set forth in SEQ ID NOs: 45-47, and the light chain variable region of the single chain fragment variable comprises CDRs having amino acid sequences set forth in SEQ ID NOs: 50-52,
preferably, when the single chain fragment variable (such as NH₂-V_{L}-linker-V_{H}-COOH or NH₂-V_{H}-linker-V_{L}-COOH)is linked to the C terminus of the heavy chain of the immunoglobulin via a linker, the antibody heavy chain variable region (V_{H}) of the single chain fragment variable comprising CDRs having amino acid sequences set forth in SEQ ID NOs: 45-47 may be firstly linked, or the antibody light chain variable region (V_{L}) of the single chain fragment variable comprising CDRs having amino acid sequences set forth in SEQ ID NOs: 50-52 may be firstly linked,
   or preferably,
the heavy chain variable region of the immunoglobulin comprises CDRs having amino acid sequences set forth in SEQ ID NOs: 45-47, and the light chain variable region of the immunoglobulin comprises CDRs having amino acid sequences set forth in SEQ ID NOs: 50-52; and/or,
the heavy chain variable region of the single chain fragment variable comprises CDRs having amino acid sequences set forth in SEQ ID NOs: 3-5, and the light chain variable region of the single chain fragment variable comprises CDRs having amino acid sequences set forth in SEQ ID NOs: 8-10,
wherein, when the single chain fragment variable (such as NH₂-V_{L}-linker-V_{H}-COOH or NH₂-V_{H}-linker-V_{L}-COOH)is linked to the C terminus of the heavy chain of the immunoglobulin via a linker, the antibody heavy chain variable region (V_{H}) of the single chain fragment variable comprising CDRs having amino acid sequences set forth in SEQ ID NOs: 3-5 may be firstly linked, or the antibody light chain variable region (V_{L}) of the single chain fragment variable comprising CDRs having amino acid sequences set forth in SEQ ID NOs: 8-10 may be firstly linked,
   preferably,
one immunoglobulin molecule is linked to two single chain fragment variable molecules, and more preferably, the two single chain fragment variable molecules are identical.

In one embodiment of the present invention, in the anti-CD73/anti-PD-1 bispecific antibody, the immunoglobulin is IgG, IgA, IgD, IgE or IgM, preferably IgG, e.g., IgG1, IgG2, IgG3 or IgG4.

In one embodiment of the present invention, in the anti-CD73/anti-PD-1 bispecific antibody, the single chain fragment variable is linked to the C terminus of the heavy chain of the immunoglobulin. Since an immunoglobulin consists of two heavy chains, two single chain fragment variable molecules are linked to one immunoglobulin molecule. Preferably, the two single chain fragment variable molecules are identical.

In one embodiment of the present invention, in the anti-CD73/anti-PD-1 bispecific antibody,
the heavy chain variable region of the immunoglobulin comprises CDRs having amino acid sequences set forth in SEQ ID NOs: 3-5, and the light chain variable region of the immunoglobulin comprises CDRs having amino acid sequences set forth in SEQ ID NOs: 8-10;
   and/or,
the heavy chain variable region of the single chain fragment variable comprises CDRs having amino acid sequences set forth in SEQ ID NOs: 45-47, and the light chain variable region of the single chain fragment variable comprises CDRs having amino acid sequences set forth in SEQ ID NOs: 50-52,
preferably, when the single chain fragment variable (such as NH₂-V_{L}-linker-V_{H}-COOH or NH₂-V_{H}-linker-V_{L}-COOH)is linked to the C terminus of the heavy chain of the immunoglobulin via a linker, the antibody heavy chain variable region (V_{H}) of the single chain fragment variable comprising CDRs having amino acid sequences set forth in SEQ ID NOs: 45-47 may be firstly linked, or the antibody light chain variable region (V_{L}) of the single chain fragment variable comprising CDRs having amino acid sequences set forth in SEQ ID NOs: 50-52 may be firstly linked.

In another embodiment of the present invention, in the anti-CD73/anti-PD-1 bispecific antibody,
the heavy chain variable region of the immunoglobulin comprises CDRs having amino acid sequences set forth in SEQ ID NOs: 45-47, and the light chain variable region of the immunoglobulin comprises CDRs having amino acid sequences set forth in SEQ ID NOs: 50-52; and/or,
the heavy chain variable region of the single chain fragment variable comprises CDRs having amino acid sequences set forth in SEQ ID NOs: 3-5, and the light chain variable region of the single chain fragment variable comprises CDRs having amino acid sequences set forth in SEQ ID NOs: 8-10,
wherein, when the single chain fragment variable (such as NH₂-V_{L}-linker-V_{H}-COOH or NH₂-V_{H}-linker-V_{L}-COOH)is linked to the C terminus of the heavy chain of the immunoglobulin via a linker, the antibody heavy chain variable region (V_{H}) of the single chain fragment variable comprising CDRs having amino acid sequences set forth in SEQ ID NOs: 3-5 may be firstly linked, or the antibody light chain variable region (V_{L}) of the single chain fragment variable comprising CDRs having amino acid sequences set forth in SEQ ID NOs: 8-10 may be firstly linked.

In one embodiment of the present invention, in the anti-CD73/anti-PD-1 bispecific antibody,
the heavy chain variable region of the immunoglobulin has an amino acid sequence selected from SEQ ID NO: 2 and SEQ ID NO: 20, and the light chain variable region of the immunoglobulin has an amino acid sequence correspondingly selected from SEQ ID NO: 7 and SEQ ID NO: 22; or the heavy chain variable region of the immunoglobulin has an amino acid sequence set forth in SEQ ID NO. 20, and the light chain variable region of the immunoglobulin has an amino acid sequence set forth in SEQ ID NO. 24;
   and/or,
the heavy chain variable region of the single chain fragment variable has an amino acid sequence selected from SEQ ID NO: 44 and SEQ ID NO: 62, and the light chain variable region of the single chain fragment variable has an amino acid sequence correspondingly selected from SEQ ID NO: 49 and SEQ ID NO: 64;
wherein, when the single chain fragment variable is linked to the C terminus of the heavy chain of the immunoglobulin via a linker, the antibody heavy chain variable region (V_{H}) of the single chain fragment variable may be firstly linked, or the antibody light chain variable region (V_{L}) of the single chain fragment variable may be firstly linked.

In another embodiment of the present invention, in the anti-CD73/anti-PD-1 bispecific antibody,
the heavy chain variable region of the immunoglobulin has an amino acid sequence selected from SEQ ID NO: 44 and SEQ ID NO: 62; the light chain variable region of the immunoglobulin has an amino acid sequence correspondingly selected from SEQ ID NO: 49 and SEQ ID NO: 64, or the heavy chain variable region of the single chain fragment variable has an amino acid sequence selected from SEQ ID NO: 2 and SEQ ID NO: 20, the light chain variable region of the single chain fragment variable has an amino acid sequence correspondingly selected from SEQ ID NO: 7 and SEQ ID NO: 22, or the heavy chain variable region of the single- chain antibody has an amino acid sequence set forth in SEQ ID NO: 20, and the light chain variable region of the single chain fragment variable has an amino acid sequence set forth in SEQ ID NO: 24.

Another aspect of the present invention relates to an isolated nucleic acid molecule comprising a nucleotide sequence capable of encoding a heavy chain variable region of a bispecific antibody, wherein,
the heavy chain variable region of the antibody comprises:
a CDR having an amino acid sequence of SEQ ID NOs: 3-5, a CDR having an amino acid sequence of SEQ ID NOs: 45-47, and a CDR having an amino acid sequence of SEQ ID NOs: 50-52;
and the heavy chain variable region of the bispecific antibody specifically binds to CD73 and PD-1 antigens as a part of the bispecific antibody, and the bispecific antibody further comprises a light chain variable region comprising:
   a CDR having an amino acid sequence of SEQ ID NOs: 8-10;
preferably, the CDRs of the light chain variable region are different from the CDRs of the heavy chain variable region.

In one embodiment of the present invention, in the bispecific antibody,
the immunoglobulin comprises a non-CDR region derived from a species other than murine, such as from a human antibody.

In one embodiment of the present invention, the constant regions of the immunoglobulin are humanized. For example, the heavy chain constant region is Ig gamma-1 chain C region, ACCESSION: P01857; and the light chain constant region is Ig kappa chain C region, ACCESSION: P01834.

In one embodiment of the present invention, the constant regions of the immunoglobulin are humanized. For example, the heavy chain constant region is Ig gamma-1 chain C region, ACCESSION: P01857; and the light chain constant region is Ig kappa chain C region, ACCESSION: P01834; wherein, according to the EU numbering system, the heavy chain constant region of the immunoglobulin comprises mutations at any 2 or 3 of positions 234, 235 and 237, and the affinity constant of the bispecific antibody for FcyRIa, FcyRIIIa and/or C1q is reduced after the mutations as compared to that before the mutations; preferably, the affinity constants are measured by a Fortebio Octet system.

In one or more embodiments of the present invention, for the bispecific antibody, according to the EU numbering system, the heavy chain constant region of the immunoglobulin has the following mutations at positions 234, 235 and/or 237:
L234A and L235A;
L234A and G237A;
L235A and G237A;
   or
L234A, L235A and G237A.

In the present invention, letters before the position number represent amino acids before mutation, and letters after the position number represent amino acids after mutation, unless otherwise specified.

In one or more embodiments of the present invention, for the bispecific antibody, according to the EU numbering system, the heavy chain constant region of the immunoglobulin has one or more mutations selected from:
N297A, D265A, D270A, P238D, L328E, E233D, H268D, P271G, A330R, C226S, C229S, E233P, P331S, S267E, L328F, A330L, M252Y, S254T, T256E, N297Q, P238S, P238A, A327Q, A327G, P329A, K322A, T394D, G236R, G236A, L328R, A330S, P331S, H268A, E318A and K320A.

In a specific embodiment, the anti-CD73/anti-PD-1 bispecific antibody has a structure shown as heavy chain-light chain-linker 1-scFv, and the scFv is selected from 14C12H1V-linker 2-14C12L1V, 14C12H1V-linker 1-14C12L1V, 14C12H1V-linker 2-14C12L1V and 14C12H1V-linker 1-14C12L1V, particularly selected from the group consisting of:
(1) NTPDV1, of which the heavy chain has an amino acid sequence set forth in SEQ ID NO: 85, the light chain has an amino acid sequence set forth in SEQ ID NO: 28, the linker 1 has an amino acid sequence set forth in SEQ ID NO: 79, 14C12H1V has an amino acid sequence set forth in SEQ ID NO: 66, the linker 2 has an amino acid sequence set forth in SEQ ID NO: 81, and 14C12L1V has an amino acid sequence set forth in SEQ ID NO: 68;
(2) NTPDV2, of which the heavy chain has an amino acid sequence set forth in SEQ ID NO: 85, the light chain has an amino acid sequence set forth in SEQ ID NO: 28, the linker 1 has an amino acid sequence set forth in SEQ ID NO: 79, 14C12H1V has an amino acid sequence set forth in SEQ ID NO: 66, the linker 1 has an amino acid sequence set forth in SEQ ID NO: 79, and 14C12L1V has an amino acid sequence set forth in SEQ ID NO: 68;
(3) NTPDV3, of which the heavy chain has an amino acid sequence set forth in SEQ ID NO: 85, the light chain has an amino acid sequence set forth in SEQ ID NO: 96, the linker 1 has an amino acid sequence set forth in SEQ ID NO: 79, 14C12H1V has an amino acid sequence set forth in SEQ ID NO: 66, the linker 2 has an amino acid sequence set forth in SEQ ID NO: 81, and 14C12L1V has an amino acid sequence set forth in SEQ ID NO: 68; and
(4) NTPDV4, of which the heavy chain has an amino acid sequence set forth in SEQ ID NO: 85, the light chain has an amino acid sequence set forth in SEQ ID NO: 96, the linker 1 has an amino acid sequence set forth in SEQ ID NO: 79, 14C12H1V has an amino acid sequence set forth in SEQ ID NO: 66, the linker 1 has an amino acid sequence set forth in SEQ ID NO: 79, and 14C12L1V has an amino acid sequence set forth in SEQ ID NO: 68. In one embodiment of the present invention, the bispecific antibody binds to CD73 protein and /or PD-1 protein with a K_{D} of less than about 10⁻⁵ M, e.g., less than about 10⁻⁶ M, 10⁻⁷ M, 10⁻⁸ M, 10⁻⁹ M, 10⁻¹⁰ M or less.

Yet another aspect of the present invention relates to a vector comprising the isolated nucleic acid molecule disclosed herein.

Yet another aspect of the present invention relates to a host cell comprising the isolated nucleic acid molecule or the vector disclosed herein.

Yet another aspect of the present invention relates to a method for preparing the bispecific antibody disclosed herein, comprising culturing the host cell disclosed herein in a suitable condition and isolating the bispecific antibody from the cell cultures.

Yet another aspect of the present invention relates to a conjugate comprising a bispecific antibody and a conjugated moiety, wherein the bispecific antibody is the bispecific antibody disclosed herein and the conjugated moiety is a detectable label; specifically, the conjugated moiety is a radioisotope, a fluorescent substance, a chemiluminescent substance, a colored substance or an enzyme.

Yet another aspect of the present invention relates to a kit comprising the bispecific antibody disclosed herein or comprising the conjugate disclosed herein; preferably, the kit further comprises a secondary antibody that specifically recognizes the bispecific antibody; optionally, the secondary antibody further comprises a detectable label, e.g., a radioisotope, a fluorescent substance, a chemiluminescent substance, a colored substance or an enzyme.

Yet another aspect of the present invention relates to use of the bispecific antibody disclosed herein in preparing a kit for detecting the presence or level of CD73 and/or PD-1 in a sample.

Yet another aspect of the present invention relates to a pharmaceutical composition comprising the bispecific antibody disclosed herein or the conjugate disclosed herein; optionally, the pharmaceutical composition further comprises a pharmaceutically acceptable carrier and/or excipient.

Yet another aspect of the present invention relates to use of the bispecific antibody disclosed herein or the conjugate disclosed herein in preventing and/or treating a tumor or anemia, or in diagnosing a tumor or anemia.

Yet another aspect of the present invention relates to use of the bispecific antibody disclosed herein or the conjugate disclosed herein in preparing a medicament for preventing and/or treating a tumor or anemia, or in preparing a medicament for diagnosing a tumor or anemia.

Yet another aspect of the present invention relates to use of the bispecific antibody disclosed herein or the conjugate disclosed herein in preparing:
a medicament for detecting the level of CD73 in a sample,
a medicament for inhibiting the enzyme activity reaction of CD73;
   and/or
a medicament for blocking the binding of PD-1 to PD-L1,
a medicament for down-regulating (e.g., down-regulating) the activity or level of PD-1,
a medicament for relieving the immunosuppression of PD-1 in an organism,
a medicament for elevating IL-2 expression in T lymphocytes, or
a medicament for elevating IFN-yexpression in T lymphocytes.

Yet another aspect of the present invention relates to an *in vivo* or *in vitro* method comprising administering to a cell or administering to a subject in need an effective amount of the bispecific antibody disclosed herein or the conjugate disclosed herein.

The anti-CD73/anti-PD-1 bispecific antibodies disclosed herein can inhibit the enzyme activity of CD73 a cell membrane surface, and can induce the secretion of IFNy and IL-2 to activate the immune response.

The variable regions of the light chain and the heavy chain determine the binding of the antigen; the variable region of each chain contains three hypervariable regions called complementarity determining regions (CDRs) (CDRs of the heavy chain (H) comprise HCDR1, HCDR2 and HCDR3, and CDRs of the light chain (L) comprise LCDR1, LCDR2 and LCDR3, which are named by Kabat et al., see Bethesda M.d., Sequences of Proteins of Immunological Interest, Fifth Edition, NIH Publication 1991; 1-3:91-3242. Preferably, CDRs may also be defined by the IMGT numbering system, see Ehrenmann F, Kaas Q, and Lefranc M P., IMGT/3Dstructure-DB and IMGT/DomainGapAlign: a database and a tool for immunoglobulins or antibodies, T cell receptors, MHC, IgSF and MhcSF[J]. Nucleic acids research 2009; 38(suppl_1): D301-D307.

The amino acid sequences of the CDRs of the monoclonal antibody in (1) to (11) below are analyzed by technical means well known to those skilled in the art, for example, according to the IMGT definition, and the results are as follows:
(1) 19F3
   The heavy chain variable region has an amino acid sequence set forth in SEQ ID NO: 2, and the light chain variable region has an amino acid sequence set forth in SEQ ID NO: 7. The 3 CDRs of the heavy chain variable region have the following amino acid sequences:
   HCDR1: GYSFTGYT (SEQ ID NO: 3),
   HCDR2: INPYNAGT (SEQ ID NO: 4), and
   HCDR3: ARSEYRYGGDYFDY (SEQ ID NO: 5);
   the 3 CDRs of the light chain variable region have the following amino acid sequences:
   LCDR1: QSLLNSSNQKNY (SEQ ID NO: 8),
   LCDR2: FAS (SEQ ID NO: 9), and
   LCDR3: QQHYDTPYT (SEQ ID NO: 10).
(2) 19F3H2L2
   The heavy chain variable region has an amino acid sequence set forth in SEQ ID NO: 20, and the light chain variable region has an amino acid sequence set forth in SEQ ID NO: 22.
   The 3 CDRs of the heavy chain variable region have the same amino acid sequences as 19F3.
   The 3 CDRs of the light chain variable region have the same amino acid sequences as 19F3.
(3) 19F3H2L3
   The heavy chain variable region has an amino acid sequence set forth in SEQ ID NO: 20, and the light chain variable region has an amino acid sequence set forth in SEQ ID NO: 24.
   The 3 CDRs of the heavy chain variable region have the same amino acid sequences as 19F3.
   The 3 CDRs of the light chain variable region have the same amino acid sequences as 19F3.
(4) 14C12
   The heavy chain variable region has an amino acid sequence set forth in SEQ ID NO: 44, and the light chain variable region has an amino acid sequence set forth in SEQ ID NO: 49.
   The 3 CDRs of the heavy chain variable region have the following amino acid sequences:
      HCDR1: GFAFSSYD (SEQ ID NO: 45)
      HCDR2: ISGGGRYT (SEQ ID NO: 46)
      HCDR3: ANRYGEAWFAY (SEQ ID NO: 47)
   the 3 CDRs of the light chain variable region have the following amino acid sequences: LCDR1: QDINTY (SEQ ID NO: 50)
      LCDR2: RAN (SEQ ID NO: 51)
      LCDR3: LQYDEFPLT (SEQ ID NO: 52)
(5) 14C12H1L1
   The heavy chain variable region has an amino acid sequence set forth in SEQ ID NO: 62, and the light chain variable region has an amino acid sequence set forth in SEQ ID NO: 64.
   The 3 CDRs of the heavy chain variable region have the same amino acid sequences as 14C12.
   The 3 CDRs of the light chain variable region have the same amino acid sequences as 14C12.
(6) The 9 CDRs of the heavy chain of NTPDV1, NTPDV2, NTPDV3 and NTPDV4 has the same amino acid sequences as those of the CDRs of 13F9 heavy chain, 14C12 heavy chain and 14C12 light chain regions, respectively, in the order from N terminus to C terminus. The sequences, in the order described above, are as follows:
   HCDR1: GYSFTGYT (SEQ ID NO: 3)
   HCDR2: INPYNAGT (SEQ ID NO: 4)
   HCDR3: ARSEYRYGGDYFDY (SEQ ID NO: 5)
   HCDR4: GFAFSSYD (SEQ ID NO: 45)
   HCDR5: ISGGGRYT (SEQ ID NO: 46)
   HCDR6: ANRYGEAWFAY (SEQ ID NO: 47)
   HCDR7: QDINTY (SEQ ID NO: 50)
   HCDR8: RAN (SEQ ID NO: 51)
   HCDR9: LQYDEFPLT (SEQ ID NO: 52)
   The 3 CDRs of the light chain has the same amino acid sequences as those of the three CDRs of 19F3 light chain, and the sequences are as follows:
   LCDR1: QSLLNSSNQKNY (SEQ ID NO: 8)
   LCDR2: FAS (SEQ ID NO: 9)
   LCDR3: QQHYDTPYT (SEQ ID NO: 10).

Yet another aspect of the present invention relates to hybridoma cell line LT014 deposited at China Center for Type Culture Collection (CCTCC) with a collection number of CCTCC NO: C2018137.

Yet another aspect of the present invention relates to hybridoma cell line LT003 deposited at China Center for Type Culture Collection (CCTCC) with a collection number of CCTCC NO: C2015105.

In the present invention, unless otherwise specified, the scientific and technical terms used herein have the meanings generally understood by those skilled in the art. In addition, the laboratory operations of cell culture, molecular genetics, nucleic acid chemistry and immunology used herein are the routine procedures widely used in the corresponding fields. Meanwhile, in order to better understand the present invention, the definitions and explanations of the relevant terms are provided below.

As used herein, the term EC₅₀ refers to the concentration for 50% of maximal effect, i.e., the concentration that can cause 50% of the maximal effect.

As used herein, the term "antibody" refers to an immunoglobulin molecule that generally consists of two pairs of polypeptide chains (each pair with one "light" (L) chain and one "heavy" (H) chain). Antibody light chains are classified as κ and λ light chains. Heavy chains are classified as µ, δ, γ, α, or ε. Isotypes of antibodies are defined as IgM, IgD, IgG, IgA, and IgE. In light chains and heavy chains, the variable region and constant region are linked by a "J" region of about 12 or more amino acids, and the heavy chain further comprises a "D" region of about 3 or more amino acids. Each heavy chain consists of a heavy chain variable region (VH) and a heavy chain constant region (CH). The heavy chain constant region consists of 3 domains (CH1, CH2, and CH3). Each light chain consists of a light chain variable region (VL) and a light chain constant region (CL). The light chain constant region consists of one domain CL. The constant region of the antibody can mediate the binding of immunoglobulins to host tissues or factors, including the binding of various cells of the immune system (e.g., effector cells) to the first component (C1q) of classical complement system. The VH and VL regions can be further subdivided into highly variable regions (called complementarity determining regions (CDRs)), between which conservative regions called framework regions (FRs) are distributed. Each VH and VL consists of 3 CDRs and 4 FRs arranged from amino terminus to carboxyl terminus in the following order: FR1, CDR1, FR2, CDR2, FR3, CDR3 and FR4. The variable regions (VH and VL) of each heavy chain/light chain pair form an antibody-binding site. The assignment of amino acids to the regions or domains is based on Bethesda M.d., Kabat Sequences of Proteins of Immunological Interest (National Institutes of Health, (1987 and 1991)), or Chothia & Lesk J. Mol. Biol., 1987; 196:901-917; Chothia et al., Nature, 1989; 342:878-883, or the definition of the IMGT numbering system, see the definition in Ehrenmann F, Kaas Q, Lefranc M P., IMGT/3Dstructure-DB and IMGT/DomainGapAlign: a database and a tool for immunoglobulins or antibodies, T cell receptors, MHC, IgSF and MhcSF[J]., Nucleic acids research, 2009; 38(suppl_1): D301-D307.

In particular, the heavy chain may also comprise more than 3 CDRs, such as 6, 9 or 12. For example, in the bispecific antibody disclosed herein, the heavy chain may be a heavy chain of IgG antibody with the C terminus linked to one ScFv, and in this case, the heavy chain comprises 9 CDRs.

The term "antibody" is not limited by any specific method for producing antibody. For example, the antibody includes a recombinant antibody, a monoclonal antibody and a polyclonal antibody. The antibody may be antibodies of different isotypes, such as IgG (e.g., subtype IgG1, IgG2, IgG3 or IgG4), IgA1, IgA2, IgD, IgE or IgM.

As used herein, the terms "mAb" and "monoclonal antibody" refer to an antibody or a fragment of an antibody that is derived from a group of highly homologous antibodies, i.e., from a group of identical antibody molecules, except for natural mutations that may occur spontaneously. The monoclonal antibody is highly specific for a single epitope on an antigen. The polyclonal antibody, relative to the monoclonal antibody, generally comprises at least 2 or more different antibodies which generally recognize different epitopes on an antigen. Monoclonal antibodies can generally be obtained using hybridoma technology first reported by Kohler et al (Köhler G, Milstein C. Continuous cultures of fused cells secreting antibody of predefined specificity [J]. Nature, 1975; 256(5517): 495), but can also be obtained using recombinant DNA technology (see, e.g., U.S. Patent 4,816,567).

As used herein, the term "humanized antibody" refers to an antibody or an antibody fragment obtained when all or a part of CDR regions of a human immunoglobulin (receptor antibody) are replaced by the CDR regions of a non-human antibody (donor antibody), wherein the donor antibody may be a non-human (e.g., mouse, rat or rabbit) antibody having expected specificity, affinity or reactivity. In addition, some amino acid residues in the framework regions (FRs) of the receptor antibody can also be replaced by the amino acid residues of corresponding non-human antibodies or by the amino acid residues of other antibodies to further improve or optimize the performance of the antibody. For more details on humanized antibodies, see, e.g., Jones et al., Nature, 1986; 321:522 525; Reichmann et al., Nature, 1988; 332:323 329; Presta, Curr. Op. Struct. Biol., 1992; 2:593-596; and Clark, Immunol. Today, 2000; 21: 397-402. In some cases, the antigen-binding fragments of the antibodies are diabodies, in which the V_{H} and V_{L} domains are expressed on a single polypeptide chain. However, the linker used is too short to allow the pairing of the two domains on the same chain. Thereby the domains are forced to pair with the complementary domains on the other chain and two antigen-binding sites are generated (see, e.g., Holliger P. et al., Proc. Natl. Acad. Sci. USA, 1993; 90:6444-6448 and Poljak R. J. et al., Structure, 1994; 2:1121-1123).

As used herein, the term "single chain fragment variable (ScFv)" refers to a molecule in which the antibody heavy chain variable region (V_{H}) and the antibody light chain variable region (V_{L}) are linked by a linker. The V_{L} and V_{H} domains are paired to form a monovalent molecule by a linker that enable them to produce a single polypeptide chain (see, e.g., Bird et al, Science, 1988; 242:423-426 and Huston et al, Proc. Natl. Acad. Sci. USA, 1988; 85:5879-5883). Such scFv molecules may have a general structure: NH₂-V_{L}-linker-V_{H}-COOH or NH₂-V_{H}-linker-V_{L}-COOH. An appropriate linker in the prior art consists of a repeating GGGGS amino acid sequence or a variant thereof. For example, a linker having the amino acid sequence (GGGGS)₄ may be used, but variants thereof may also be used (Holliger et al., Proc. Natl. Acad. Sci. USA, 1993; 90: 6444-6448). Other linkers that can be used in the present invention are described by Alfthan et al., Protein Eng., 1995; 8:725-73 1, Choi et al., Eur. J. Immunol., 2001; 31: 94-106, Hu et al., Cancer Res., 1996; 56:3055-3061, Kipriyanov et al., J. Mol. Biol., 1999; 293:41-56 and Roovers et al., Cancer Immunology, Immunotherapy, 2001, 50(1): 51-59.

As used herein, the term "isolated" refers to obtaining by artificial means from natural state. If a certain "isolated" substance or component appears in nature, it may be the case that change occurs in its natural environment, or that it is isolated from the natural environment, or both. For example, a certain non-isolated polynucleotide or polypeptide naturally occurs in a certain living animal, and the same polynucleotide or polypeptide with high purity isolated in such a natural state is referred to as an isolated polynucleotide or polypeptide. The term "isolated" does not exclude the existence of artificial or synthetic substances or other impurities that do not affect the activity of the substance. As used herein, the term "vector" refers to a nucleic acid vehicle into which a polynucleotide can be inserted. When a vector allows the expression of the protein encoded by the inserted polynucleotide, the vector is referred to as an expression vector. The vector can be introduced into a host cell by transformation, transduction or transfection, such that the genetic substance elements carried by the vector can be expressed in the host cell. Vectors are well known to those skilled in the art, including but not limited to: plasmids; phagemids; cosmids; artificial chromosomes, such as yeast artificial chromosome (YAC), bacterial artificial chromosome (BAC), or P1-derived artificial chromosome (PAC); phages such as lambda phages or M13 phages; and animal viruses. Animal viruses that can be used as vectors include, but are not limited to retroviruses (including lentiviruses), adenoviruses, adeno-associated viruses, herpes viruses (such as herpes simplex virus), poxviruses, baculoviruses, papillomaviruses, and papovaviruses (such as SV40). A vector may comprise a variety of elements that control expression, including, but not limited to promoter sequences, transcription initiation sequences, enhancer sequences, selection elements and reporter genes. In addition, the vector may further comprise a replication initiation site.

As used herein, the term "host cell" refers to cells to which vectors can be introduced, including, but not limited to, prokaryotic cells such as *E. coli* or *bacillus subtilis*, fungal cells such as yeast cells or *aspergillus*, insect cells such as S2 drosophila cells or Sf9, or animal cells such as fibroblasts, CHO cells, GS cells, COS cells, NSO cells, HeLa cells, BHK cells, HEK 293 cells, or human cells.

As used herein, the term "specifically bindi" refers to a non-random binding reaction between two molecules, such as a reaction between an antibody and an antigen it targets. In some embodiments, an antibody that specifically binds to an antigen (or an antibody that is specific for an antigen) means that the antibody binds to the antigen with an affinity (K_{D}) of less than about 10⁻⁵ M, such as less than about 10⁻⁶ M, 10⁻⁷ M, 10⁻⁸ M, 10⁻⁹ M or 10⁻¹⁰ M or less.

As used herein, the term "K_{D}" refers to a dissociation equilibrium constant for a specific antibody-antigen interaction, which is used to describe the binding affinity between the antibody and the antigen. A smaller dissociation equilibrium constant indicates a stronger antibody-antigen binding and a higher affinity between the antibody and the antigen. Typically, the antibody binds to the antigen (e.g., PD-1 protein) with a dissociation equilibrium constant (K_{D}) of less than about 10⁻⁵ M, such as less than about 10⁻⁶ M, 10⁻⁷ M, 10⁻⁸ M, 10⁻⁹ M or 10⁻¹⁰ M or less. K_{D} can be determined using methods known to those skilled in the art, for example, using a Fortebio system.

As used herein, the terms "monoclonal antibody" and "mAb" have the same meaning and can be used interchangeably; the terms "polyclonal antibody" and "pAb" have the same meaning and can be used interchangeably. Besides, herein, amino acids are generally represented by single-letter and three-letter abbreviations known in the art. For example, alanine can be represented by A or Ala.

As used herein, the term "pharmaceutically acceptable carrier and/or excipient" refers to a carrier and/or excipient that is pharmacologically and/or physiologically compatible with the subject and the active ingredient. Such carriers and/or excipients are well known in the art (see, e.g., Remington's Pharmaceutical Sciences, edited by Gennaro AR, 19th Ed., Pennsylvania: Mack Publishing Company, 1995), including, but not limited to: pH regulators, surfactants, adjuvants, and ionic strength enhancers. For example, the pH regulators include, but are not limited to, phosphate buffer; the surfactants include, but are not limited to, cationic, anionic, or non-ionic surfactants, such as Tween-80; the ionic strength enhancers include, but are not limited to, sodium chloride.

As used herein, the term "effective amount" refers to an amount sufficient to obtain or at least partially obtain desired effects. For example, a prophylactically effective amount against a disease (e.g., a tumor) refers to an amount sufficient to prevent, stop, or delay the onset of a disease (e.g., a tumor); a therapeutically effective amount refers to an amount sufficient to cure or at least partially stop a disease and complications thereof in patients suffering from the disease. It is undoubtedly within the ability of those skilled in the art to determine such an effective amount. For example, the amount effective for therapeutic purpose will depend on the severity of the disease to be treated, the overall state of the patient's own immune system, the general condition of the patient such as age, body weight and gender, the route of administration, and other treatments given concurrently, etc.

### Beneficial Effects

The monoclonal antibody of the present invention (such as 13F9H2L3) can well and specifically bind to CD73, and can effectively inhibit the enzyme activity reaction of CD73 in a non-substrate competition mode, reduce the production of adenosine, promote the activity of T cells and the tumor inhibitory effect.

The bispecific antibody disclosed herein, such as NTPDV1, NTPDV2, NTPDV3 and NTPDV4, can well and specifically bind to PD-1 and CD73, can effectively block the binding of PD-1 to PDL1, specifically relieve the immunosuppression of PD-1 in an organism, inhibit the catalytic activity of CD73, relieve the inhibition on immune cells by adenosine, activate T lymphocytes, and does not cause the release of cytokines IL-8 and IL-6, showing effectively increased safety and efficacy.

The bifunctional antibody disclosed herein has the potential for use in preparing an anti-tumor drug.

### BRIEF DESCRIPTION OF THE DRAWINGS

FIG. 1. Binding of P1D7V01, P1D7V02R, P1D7V03, P1D7V04R, 14C12H1L1 and nivolumab to PD-1-mFc determined by ELISA.
FIG. 2. Binding of P1D7V01, P1D7V02R, P1D7V03, P1D7V04R, 19F3 H2L3 and MEDI9447 to human NT5E-Biotin determined by ELISA.
FIG. 3. Activity of P1D7V01, P1D7V02R, P1D7V03, P1D7V04R, 14C12H1L1 and nivolumab competing with human PD-L1-mFc for binding to human PD-1-mFc-Biotin.
FIG. 4. Affinity constant of P1D7V01 for PD-1-mFc.
FIG. 5. Affinity constant of 14C12H1L1 for PD-1-mFc.
FIG. 6. Affinity constant of nivolumab for PD-1-mFc.
FIG. 7. Affinity constant of P1D7V01 for human NT5E(1-552)-his.
FIG. 8. Affinity constant of MEDI 9447 for human NTSE(1-552)-his.
FIG. 9. Binding activity of P1D7V01, P1D7V02R, P1D7V03, P1D7V04R and 14C12H1L1 to PD-1 on 293T-PD1 cell surface determined by FACS.
FIG. 10. Binding activity of P1D7V01, P1D7V03, MEDI9447 and 19F3H2L3 to CD73 on MDA-MB-231 cell surface determined by FACS.
FIG. 11. Inhibition of anti-CD73/anti-PD-1 bispecific antibodies on the enzyme activity of CD73 on MDA-MB-231 membrane surface.
FIG. 12. Inhibition of anti-CD73/anti-PD-1 bispecific antibodies on the enzyme activity of CD73 on U87-MG membrane surface.
FIG. 13. Biological activity of anti-CD73/anti-PD-1 bispecific antibodies for promoting IFN-y secretion in Raji-PDL1 mixed lymphocyte reaction system.
FIG. 14. Biological activity of anti-CD73/anti-PD-1 bispecific antibodies for promoting IL-2 secretion in Raji-PDL1 mixed lymphocyte reaction system.
FIG. 15. Biological activity of anti-CD73/anti-PD-1 bispecific antibodies for promoting IFN-y secretion in DC mixed lymphocyte reaction system.
FIG. 16. Biological activity of anti-CD73/anti-PD-1 bispecific antibodies for promoting IL-2 secretion in DC mixed lymphocyte reaction system.
FIG. 17. Affinity constant of 14C12H1L1(hG1TM) for PD-1-mFc.
FIG. 18. Affinity constant of nivolumab for PD-1-mFc.
FIG. 19. Affinity constant of NTPDV1 for PD-1-mFc.
FIG. 20. Affinity constant of NTPDV2 for PD-1-mFc.
FIG. 21. Affinity constant of NTPDV3 for PD-1-mFc.
FIG. 22. Affinity constant of NTPDV4 for PD-1-mFc.
FIG. 23. Affinity constant of 19F3H2L3(hG1M) for human NT5E(1-552)-his.
FIG. 24. Affinity constant of NTPDV1 for human NT5E(1-552)-his.
FIG. 25. Affinity constant of NTPDV2 for human NT5E(1-552)-his.
FIG. 26. Affinity constant of NTPDV3 for human NT5E(1-552)-his.
FIG. 27. Affinity constant of NTPDV4 for human NT5E(1-552)-his.
FIG. 28. Inhibition of the enzyme activity of CD73 on U87-MG cell membrane surface by anti-CD73/anti-PD-1 bispecific antibodies.
FIG. 29. Biological activity of anti-CD73/anti-PD-1 bispecific antibodies for promoting IFN-y and IL-2 secretion determined by mixed lymphocyte reaction (MLR).
FIG. 30. Effect of isotype control, 19F3H2L3(hG1M) and different doses of NTPDV2 on tumor volume in mice.
FIG. 31. Effect of isotype control, 19F3H2L3(hG1M) and different doses of NTPDV2 on body weight of mice.
FIG. 32. Effective elimination of PD-1/CD73 bispecific antibody-mediated IL-8 secretion in human macrophages by the amino acid mutations of Fc segments in a co-culture system of CHO-K1-PD1 cells and human macrophages.
FIG. 33. Effective elimination of PD-1/CD73 bispecific antibody-mediated IL-6 secretion in human macrophages by the amino acid mutations of Fc segments in a co-culture system of CHO-K1-PD1 cells and human macrophages.
FIG. 34. Effective elimination of PD-1/CD73 bispecific antibody-mediated IL-8 secretion in human macrophages by the amino acid mutations of Fc segments in a co-culture system of U87-MG cells and human macrophages.
FIG. 35. Effective elimination of PD-1/CD73 bispecific antibody-mediated IL-6 secretion in human macrophages by the amino acid mutations of Fc segments in a co-culture system of U87-MG cells and human macrophages.

### Collection information of biological materials:

The hybridoma cell line LT003 (also called PD-1-14C12), which was deposited at China Center for Type Culture Collection (CCTCC) on June 16, 2015 with a collection number of CCTCC NO: C2015105 and a collection address of Wuhan University, Wuhan, China, postal code: 430072.

The hybridoma cell line LT014 (also called CD73-19F3), which was deposited at China Center for Type Culture Collection (CCTCC) on June 21, 2018 with a collection number of CCTCC NO: C2018137 and a collection address of Wuhan University, Wuhan, China, postal code: 430072.

### DETAILED DESCRIPTION

The embodiments of the present invention will be described in detail below with reference to the examples. Those skilled in the art will understand that the following examples are only for illustrating the present invention, and should not be construed as limitations on the scope of the present invention. In the cases where the techniques or conditions are not specified, the examples were implemented according to the techniques or conditions described in the literature in the art (e.g., see, Molecular Cloning: A Laboratory Manual, authored by J. Sambrook et al., and translated by Peitang Huang et al., 3rd Edition, Science Press) or according to the product manual. Reagents or instruments used are commercially available conventional products if the manufacturers thereof are not specified. For example, MDA-MB-231 cells and U87-MG cells can be purchased from ATCC.

In the following examples of the present invention, BALB/c mice used were purchased from Guangdong Medical Laboratory Animal Center.

In the following examples of the present invention, the positive control antibody MEDI9447 (generic name: Oleclumab) used was produced by Zhongshan Akesobio Co. Ltd., the sequence of which is identical to the antibody SEQ ID NOs: 21-24 described in the Medmmune Limited's Patent Publication No. US20160129108A1.

In the following examples of the present invention, the marketed antibody nivolumab (trade name: Opdivo) for the same target was used, which was purchased from Bristol-Myers Squibb.

In the following examples of the present invention, the cell line 293T-PD1 used was constructed by Zhongshan Akesobio Co. Ltd. The cell line 293T-PD1 was prepared by viral infection of HEK293T cells using 3rd Generation Lentiviral Systems (see, e.g., A Third Generation Lentivirus Vector with a Conditional Packaging System. Dull T, Zufferey R, Kelly M, Mandel RJ, Nguyen M, Trono D, and Naldini L., J Virol., 1998. 72(11):8463-8471), wherein the lentivirus expression vector used was pCDH-CMV-PD-1FL-Puro (PD1, Genebank ID: NM_005018; vector pCDH-CMV-Puro, purchased from Youbio, Cat. No. VT1480).

In the following examples of the present invention, the cell line Raji-PDL1 used was constructed by Zhongshan Akesobio Co. Ltd. The cell line Raji-PDL1 was prepared by viral infection of Raji cells using 3rd Generation Lentiviral Systems (see, e.g., A Third Generation Lentivirus Vector with a Conditional Packaging System. Dull T, Zufferey R, Kelly M, Mandel RJ, Nguyen M, Trono D, and Naldini L., J Virol., 1998. 72(11):8463-8471), wherein the lentivirus expression vector used was plenti6.3-PDL1 (PDL1, Genebank ID: NP_054862.1; vector plenti6.3, purchased from Invitrogen, Cat. No. K5315-20).

In the following examples of the present invention, the cell line CHO-K1-PD1 used was constructed by Zhongshan Akesobio Co. Ltd. The cell line CHO-K1-PD1 was prepared by viral infection of CHO-K1 cells using 3rd Generation Lentiviral Systems (see, e.g., A Third Generation Lentivirus Vector with a Conditional Packaging System. Dull T, Zufferey R, Kelly M, Mandel RJ, Nguyen M, Trono D, and Naldini L., J Virol., 1998. 72(11):8463-8471), wherein the lentivirus expression vector used was pCDH-CMV-PD-1FL-Puro (PD1, Genebank ID: NM_005018; vector pCDH-CMV-Puro, purchased from Youbio, Cat. No. VT1480).

Nivolumab (trade name: Opdivo), an IgG4 subtype anti-PD-1 antibody carrying S228P mutation, was used as a control antibody in the examples and was purchased from Bristol-Myers Squibb.

In the following examples of the present invention, the isotype control antibodiy used, i.e., hIgG1, was an antibody targeting human anti-hen egg lysozyme (HEL), and the variable region sequence of the antibody is from the study reported by Acierno et al., entitled "Affinity maturation increases the stability and plasticity of the Fv domain of anti-protein antibodies" (Acierno et al., J Mol biol., 2007; 374(1): 130-46). In the constant region fragment of hIgG1, the heavy chain constant region is Ig gamma-1 chain C region, ACCESSION: P01857, and the light chain constant region is Ig kappa chain C region, ACCESSION: P01834; the hIgG1 was prepared in the laboratory of Zhongshan Akesobio Co. Ltd.

### Example 1: Preparation of anti-CD73 antibody 19F3

### 1. Preparation of hybridoma cell line LT014

The antigen used to prepare the anti-CD73 antibody was human NT5E-his (for NT5E, Genbank ID: NP_002517.1, position: 1-552). Spleen cells of immunized mice were fused with myeloma cells of the mice to prepare hybridoma cells. With human NT5E-Biotin (for NT5E, Genbank ID: NP_002517.1, position: 1-552) taken as an antigen, the hybridoma cells were screened by indirect ELISA to obtain hybridoma cells capable of secreting antibodies that can specifically bind to CD73. The hybridoma cells obtained by ELISA screening were subjected to limiting dilution to obtain a stable hybridoma cell line. The above hybridoma cell line was named as hybridoma cell line LT014, and the monoclonal antibody secreted therefrom was named 19F3.

The hybridoma cell line LT014 (also called CD73-19F3) was deposited at China Center for Type Culture Collection (CCTCC) on June 21, 2018 with a collection number of CCTCC NO: C2018137 and a collection address of Wuhan University, Wuhan, China, postal code: 430072.

### 2. Preparation of anti-CD73 antibody 19F3

The cell line LT014 prepared above was cultured with a chemical defined medium (CD medium, containing 1% Penicillin-Streptomycin) in a 5% CO₂, 37 °C incubator. After 7 days, the supernatants were collected and purified by high-speed centrifugation, vacuum filtration through a microfiltration membrane and a HiTrap protein A HP column to obtain an antibody 19F3.

### Example 2: Sequence analysis of anti-CD73 antibody 19F3

mRNA was extracted from the cell line LT014 prepared in Example 1 according to the method described in the manual of RNAprep pure Cell/Bacteria Kit (Tiangen, Cat. No. DP430).

cDNA was synthesized according to the manual of Invitrogen SuperScript^{®} III First-Strand Synthesis System for RT-PCR and amplified by PCR.

The PCR-amplified products were directly subjected to TA cloning according to the manual of the pEASY-T1 Cloning Kit (Transgen CT 101).

The TA-cloned products were directly sequenced, and the sequencing results are as follows:
The nucleotide sequence (363 bp) of 19F3 heavy chain variable region is set forth in SEQ ID NO: 1, and the encoded amino acid sequence (121 aa) is set forth in SEQ ID NO: 2.

According to the IMGT numbering system, the heavy chain CDR1 has a sequence set forth in SEQ ID NO: 3, the heavy chain CDR2 has a sequence set forth in SEQ ID NO: 4,
and the heavy chain CDR3 has a sequence set forth in SEQ ID NO: 5.

The nucleotide sequence (339 bp) of 19F3 light chain variable region is set forth in SEQ ID NO: 6, and the encoded amino acid sequence (113 aa) is set forth in SEQ ID NO: 7. According to the IMGT numbering system, the light chain CDR1 has a sequence set forth in SEQ ID NO: 8, the light chain CDR2 has a sequence set forth in SEQ ID NO: 9, and the light chain CDR3 has a sequence set forth in SEQ ID NO: 10.

The amino acid sequences of 4 framework regions (FR-H1 to FR-H4) of the 19F3 heavy chain are set forth in SEQ ID NO: 11 to SEQ ID NO: 14, respectively; the amino acid sequences of 4 framework regions (FR-H1 to FR-H4) of the 19F3 light chain are set forth in SEQ ID NO: 15 to SEQ ID NO: 18, respectively.

### Example 3: Design, preparation and detection of humanized anti-human CD73 antibodies

### 1. Design of light and heavy chain sequences of humanized antibodies 19F3H2L3 and 19F3H2L2

Based on the three-dimensional crystal structure of human CD73 protein (Hage T, Reinemer P, Sebald W., Crystals of a 1:1 complex between human interleukin-4 and the extracellular domain of its receptor alpha chain., Eur J Biochem., 1998; 258(2):831-6) and the sequence of murine antibody 19F3 obtained in Example 2, the variable region sequences of antibodies 19F3H1L1, 19F3H2L3 and 19F3H2L3 were obtained by computer modeling and mutation design. The corresponding heavy chain variable region sequences were 19F3H1 and 19F3H2, respectively (with amino acid sequences set forth in SEQ ID NO: 93 and SEQ ID NO: 97, respectively), and the light chain variable region sequences were 19F3L1, 19F3L2 and 19F3L3, respectively (with amino acid sequences set forth in SEQ ID NO: 95, SEQ ID NO: 98 and SEQ ID NO: 99, respectively). The antibody constant region sequences are from NCBI database: the heavy chain constant region is Ig gamma-1 chain C region, ACCESSION: P01857; the light chain constant region is Ig kappa chain C region, ACCESSION: P01834. 19F3H2L3 is also known as 19F3H2L3(hG1WT) in the Chinese Patent Application No. 202110270671.X, wherein the light and heavy chain variable regions of 19F3H1L1, 19F3H2L2 and 19F3H2L3 can further be noted as 19F3H1V (or 19F3H1_{V}), 19F3H2V (or 19F3H2_{V}), 19F3L1V (or 19F3L1_{V}), 19F3L2V (or 19F3L2_{V}) and 19F3L3V (or 19F3L3_{V}).
(1) Heavy chain variable region and light chain variable region sequences of humanized monoclonal antibody 19F3H1L1 are as follows:
   The nucleotide sequence (363 bp) of the heavy chain variable region is set forth in SEQ ID NO: 92, and the encoded amino acid sequence (121 aa) is set forth in SEQ ID NO: 93.
   The nucleotide sequence (339 bp) of the light chain variable region is set forth in SEQ ID NO: 94, and the encoded amino acid sequence (113 aa) is set forth in SEQ ID NO: 95.
(2) Heavy chain variable region and light chain variable region sequences of humanized monoclonal antibody 19F3H2L2 are as follows:
   The nucleotide sequence (363 bp) of the heavy chain variable region 19F3H2 is set forth in SEQ ID NO: 19, and the encoded amino acid sequence (121 aa) is set forth in SEQ ID NO: 20.
   The nucleotide sequence (339 bp) of the light chain variable region 19F3L3 is set forth in SEQ ID NO: 21, and the encoded amino acid sequence (113 aa) is set forth in SEQ ID NO: 22.
(3) Heavy chain variable region and light chain variable region sequences of humanized monoclonal antibody 19F3H2L3 are as follows:
   The nucleotide sequence (363 bp) of the heavy chain variable region 19F3H2 is set forth in SEQ ID NO: 19, and the encoded amino acid sequence (121 aa) is set forth in SEQ ID NO: 20.
   The nucleotide sequence (339 bp) of the light chain variable region 19F3L3 is set forth in SEQ ID NO: 23, and the encoded amino acid sequence (113 aa) is set forth in SEQ ID NO: 24.

### 2. Preparation of humanized antibodies 19F3H1L1, 19F3H2L2 and 19F3H2L3

Heavy chain constant regions used Ig gamma -1 chain C region, ACCESSION: P01857; the light chain constant regions used Ig kappa chain C region, ACCESSION: P01834. The heavy chain cDNA and light chain cDNA of 19F3H1L1, 19F3H2L2 and 19F3H2L3 were separately cloned into pUC57simple (provided by GenScript) vectors to obtain pUC57simple-19F3H1, pUC57simple-19F3L1, pUC57simple-19F3H2, pUC57simple-19F3L2 and pUC57simple-19F3L3. Referring to the standard techniques described in Molecular Cloning: A Laboratory Manual (Second Edition), the heavy and light chain full-length genes synthesized by EcoRI&HindIII digestion were subcloned into an expression vector pcDNA3.1 through digestion by an restriction enzyme (EcoRI&HindIII) to obtain expression plasmids pcDNA3.1-19F3H1, pcDNA3.1-19F3L1, pcDNA3.1-19F3H2, pcDNA3.1-19F3L2, and pcDNA3.1-19F3L3, and the heavy/light chain genes of the recombinant expression plasmids were further subjected to sequencing analysis. Then the designed gene combinations comprising the corresponding light and heavy chain recombinant plasmids (pcDNA3.1-19F3H1/pcDNA3.1-19F3L1, pcDNA3.1-19F3H2/pcDNA3.1-19F3L2, and pcDNA3.1-19F3H2/pcDNA3.1-19F3L3) were separately co-transfected into 293F cells, and the culture solutions were collected and purified. After the sequences were verified, endotoxin-free expression plasmids were prepared, and were transiently transfected into HEK293 cells for antibody expression. The culture solutions were collected after 7 days, and subjected to affinity purification on a Protein A column to obtain humanized antibodies.

### 3. Design of light and heavy chain sequences of humanized antibodies 19F3H2L3(hG1M) and 19F3H2L3(hG1TM)

On the basis of 19F3H2L3 obtained in of Example 3 1, 19F3H2L3(hG1M) was obtained by introducing a leucine-to-alanine point mutation at position 234 (L234A) and a leucine-to-alanine point mutation at position 235 (L235A) of the heavy chain. The nucleotide and amino acid sequences of the heavy chain of 19F3H2L3(hG1M) are set forth in SEQ ID NO: 25 and SEQ ID NO: 26, respectively; the light chain constant region of 19F3H2L3(hG1M) is an Ig kappa chain C region, ACCESSION: P01834, and the nucleotide and amino acid sequences of the light chain of 19F3H2L3(hG 1M) are set forth in SEQ ID NO: 27 and SEQ ID NO: 28, respectively.

On the basis of 19F3H2L3 obtained in step 1 of Example 3, 19F3H2L3(hG1TM) was obtained by introducing a leucine-to-alanine point mutation at position 234 (L234A), a leucine-to-alanine point mutation at position 235 (L235A), and a glycine-to-alanine point mutation at position 237 (G237A) in the heavy chain. The nucleotide and amino acid sequences of the heavy chain are set forth in SEQ ID NO: 29 and SEQ ID NO: 30, respectively; and the light chain is identical to 19F3H2L3(hG1M).

The amino acid sequences of 4 framework regions (FR-H1 to FR-H4) of 19F3H2 are set forth in SEQ ID NO: 31 to SEQ ID NO: 34, respectively;
the amino acid sequences of 4 framework regions (FR-H1 to FR-H4) of the 19F3L2 light chain are set forth in SEQ ID NO: 35 to SEQ ID NO: 38, respectively; and the amino acid sequences of 4 framework regions (FR-H1 to FR-H4) of the 19F3L3 light chain are set forth in SEQ ID NO: 39 to SEQ ID NO: 42, respectively.

### 4. Preparation of humanized antibody 19F3H2L3(hG1M)

The heavy chain cDNA and light chain cDNA of 19F3H2L3(hG1M) were separately cloned into vector pUC57simple (provided by Genscript) to obtain pUC57simple-19F3H2(hG1M) and pUC57simple-19F3L3, respectively. Referring to the standard techniques described in Molecular Cloning: A Laboratory Manual (Second Edition), the heavy and light chain full-length genes synthesized by EcoRI&HindIII digestion were subcloned into an expression vector pcDNA3.1 through digestion by an restriction enzyme (EcoRI&HindIII) to obtain expression plasmids pcDNA3.1-19F3H2(hG1M) and pcDNA3.1-19F3L3, and the heavy/light chain genes of the recombinant expression plasmids were further subjected to sequencing analysis. Then the designed gene combination comprising the corresponding light and heavy chain recombinant plasmids pcDNA3.1-19F3H2(hG1M)/pcDNA3.1-19F3L3 was co-transfected into 293F cells, and the culture solutions were collected and purified. After the sequences were verified, endotoxin-free expression plasmids were prepared, and were transiently transfected into HEK293 cells for antibody expression. The culture solutions were collected after 7 days, and subjected to affinity purification on a Protein A column to obtain a humanized antibody 19F3H2L3(hG1M).

### Example 4: Preparation of anti-PD-1 antibody 14C12

### 1. Preparation of the hybridoma cell line LT003

Using PD-1-mFc fusion protein (PD-1 GenBank: NM-005018, mFc SEQ ID NO: 89) as an antigen, spleen cells of an immunized BALB/c mice (purchased from Guangdong Medical Laboratory Animal Center) and mouse myeloma cells were fused into hybridoma cells, and the established methods (e.g., Stewart, S.J., "Monoclonal Antibody Production", in Basic Methods in Antibody Production and Characterization, Eds. G.C. Howard and D.R. Bethell, Boca Raton: CRC Press, 2000) were referred to.

The plate was coated with PD-1-hFc (PD-1, Genbank ID: NM_005018, hFc is a human IgG Fc purification tag, specifically Ig gamma-1 chain C region, Genbank ID: P01857, positions 114-330) for indirect ELISA. By screening, hybridoma cells secreting new antibodies specifically binding to PD-1 were obtained.

Hybridoma cell lines capable of secreting a monoclonal antibody that competes with the ligand PD-L1-hFc (PD-L1 Genbank ID: NP_054862.1) for binding to PD-1 were screened by competitive ELISA, and a stable hybridoma cell line was obtained by limiting dilution. The LT003 stable cell line (PD-1-14C12) was obtained by limiting dilution, and the secreted monoclonal antibody was named as 14C12.

The hybridoma cell line LT003 (also called PD-1-14C12) was deposited at China Center for Type Culture Collection (CCTCC) on June 16, 2015 with a collection number of CCTCC NO: C2015105 and a collection address of Wuhan University, Wuhan, China, postal code: 430072.

### 2. Preparation of anti-PD-1 antibody 14C12

The LT003 cells prepared above were cultured in an IMDM medium containing 10% low IgG fetal bovine serum (IMDM medium containing 1% Penicillin-Streptomycin, cultured in a 5% CO₂, 37 °C cell incubator). After 7 days, the cell culture supernatant was collected and purified to obtain antibody 14C12.

### Example 5: Sequence analysis of anti-PD-1 antibody 14C12

mRNA was extracted from the hybridoma cell line LT003 prepared in Example 1 according to the method described in the manual of RNAprep pure Cell/Bacteria Kit (Tiangen, Cat. No. DP430).

cDNA was synthesized according to the manual of Invitrogen SuperScript^{®} III First-Strand Synthesis System for RT-PCR and amplified by PCR.

The PCR-amplified products were directly subjected to TA cloning according to the manual of the pEASY-T1 Cloning Kit (Transgen CT 101).

The TA-cloned products were directly sequenced, and the sequencing results are as follows:
The nucleotide sequence (354 bp) of the heavy chain variable region is set forth in SEQ ID NO: 43, and the encoded amino acid sequence (118 aa) is set forth in SEQ ID NO: 44. According to the IMGT numbering system, the heavy chain CDR1 has a sequence set forth in SEQ ID NO: 45, the heavy chain CDR2 has a sequence set forth in SEQ ID NO: 46, and the heavy chain CDR3 has a sequence set forth in SEQ ID NO: 47.

The nucleotide sequence (321 bp) of the light chain variable region is set forth in SEQ ID NO: 48, and the encoded amino acid sequence (107 aa) is set forth in SEQ ID NO: 49. According to the IMGT numbering system, the light chain CDR1 has a sequence set forth in SEQ ID NO: 50, the light chain CDR2 has a sequence set forth in SEQ ID NO: 51, and the light chain CDR3 has a sequence set forth in SEQ ID NO: 52.

The amino acid sequences of 4 framework regions (FR-H1 to FR-H4) of the 14C12 heavy chain are set forth in SEQ ID NO: 53 to SEQ ID NO: 56, respectively; the amino acid sequences of 4 framework regions (FR-H1 to FR-H4) of the 14C12 light chain are set forth in SEQ ID NO: 57 to SEQ ID NO: 60, respectively.

### Example 6: Design and preparation of humanized anti-PD-1 antibodies 14C12H1L1 and 14C12H1L1(hGlTM)

### 1. Design of humanized anti-PD-1 antibody 14C12H1L1

The light and heavy chain sequences of the humanized antibody 14C12H1L1 were designed according to the three-dimensional crystal structure of PD-1 protein (Shinohara T, et al., Structure and chromosomal localization of the human PD-1 gene (PDCD1). Genomics 1995, 23 (3): 704-6) and the sequence of antibody 14C12 obtained in Example 5 by computer simulation of antibody model and designing mutations according to the model to obtain the variable region sequences of antibody 14C12H1L1.

The designed variable region sequences are as follows.

The nucleotide sequence (354 bp) of the heavy chain variable region 14C12H1 of the humanized monoclonal antibody 14C12H1L1 is set forth in SEQ ID NO: 61, and the encoded amino acid sequence (118 aa) is set forth in SEQ ID NO: 62.

The nucleotide sequence (321 bp) of the light chain variable region 14C12L1 of the humanized monoclonal antibody 14C12H1L1 is set forth in SEQ ID NO: 63, and the encoded amino acid sequence (107 aa) is set forth in SEQ ID NO: 64.

The constant region of the antibody 14C12H1L1 is from NCBI database (the heavy chain constant region is Ig gamma-1 chain C region, ACCESSION: P01857; the light chain constant region is Ig kappa chain C region, ACCESSION: P01834). The nucleotide sequence and amino acid sequence of the 14C12H1L1 heavy chain are set forth in SEQ ID NOs: 65 and 66, respectively, and the nucleotide sequence and amino acid sequence of the 14C12H1L1 light chain are set forth in SEQ ID NOs: 67 and 68, respectively. 14C12H1L1 is also known as 14C12H1L1(hGlWT) herein and in Chinese Patent Application No. 202110270671.X, wherein the heavy chain variable region and the light chain variable region of 14C12H1L1 are also known as 14C12H1V (or 14C12H1_{V}) and 14C12L1_{V} (or 14C12L1_{V}) herein and in Chinese Patent Application No. 202110270671.X.

### 2. Design of light and heavy chain sequences of humanized antibody 14C12H1L1(hG1TM)

On the basis of 14C12H1L1 obtained in step 1 of Example 6, 14C12H1L1(hG1TM) was obtained by introducing a leucine-to-alanine point mutation at position 234 (L234A), a leucine-to-alanine point mutation at position 235 (L235A), and a glycine-to-alanine point mutation at position 237 (G237A) in the heavy chain. The nucleotide and amino acid sequences of the heavy chain of 14C12H1L1(hG1TM) are set forth in SEQ ID NO: 69 and SEQ ID NO: 70, respectively; and the light chain is identical to 14C12H1L1.

The amino acid sequences of 4 framework regions (FR-H1 to FR-H4) of 14C12H1 are set forth in SEQ ID NO: 71 to SEQ ID NO: 74, respectively;
the amino acid sequences of 4 framework regions (FR-H1 to FR-H4) of the 14C12L1 light chain are set forth in SEQ ID NO: 75 to SEQ ID NO: 78, respectively.

### 3. Preparation of humanized antibodies 14C12H1L1 and 14C12H1L1(hG1TM)

The heavy chain cDNA and light chain cDNA of 14C12H1L1(hG1TM) and 14C12H1L1 were separately cloned into pUC57simple (provided by GenScript) vectors to obtain pUC57simple-14C12H1, pUC57simple-14C12L1 and pUC57simple-14C12H1(hG1TM). Referring to the standard techniques described in Molecular Cloning: A Laboratory Manual (Second Edition), the heavy and light chain full-length genes synthesized by EcoRI&HindIII digestion were subcloned into an expression vector pcDNA3.1 through digestion by an restriction enzyme (EcoRI&HindIII) to obtain expression plasmids pcDNA3.1-14C12H1, pcDNA3.1-14C12L1 and pcDNA3.1-14C12H1(hG1TM), and the heavy/light chain genes of the recombinant expression plasmids were further subjected to sequencing analysis. Then the designed gene combinations comprising the corresponding light and heavy chain recombinant plasmids (pcDNA3.1-14C12H1(hG1TM)/pcDNA3.1-14C12L1, and pcDNA3.1-14C12H1/pcDNA3.1-14C12L1) were separately co-transfected into 293F cells, and the culture solutions were collected and purified. After the sequences were verified, endotoxin-free expression plasmids were prepared, and were transiently transfected into HEK293 cells for antibody expression. The culture solutions were collected after 7 days, and subjected to affinity purification on a Protein A column to obtain humanized antibodies.

### Example 7: Sequence design and expression of anti-PD-1/CD73 bifunctional antibodies

### 1. Sequence design

The structure of the bifunctional antibody described herein is in the Morrison form (IgG-scFv), i.e., C-termini of two heavy chains of an IgG antibody are each linked to a scFv fragment of another antibody, and the main composition design of the heavy and light chains is as shown in Table 1 below. Table 1 Table 1. Composition design of heavy and light chains of P1D7V01, P1D7V02R, P1D7V03, P1D7V04R, P1D7V07 and P1D7V08.

| Bispecific antibody No. | Heavy chain | | | Light chain |
|---|---|---|---|---|
| | IgG moiety | Linker | scFv moiety | |
| P1D7V01 | 14C12H1 | Linker 1 | 19F3H2_{V}-Linker1-19F3L3_{V} | 14C12L1 |
| P1D7V02R | 14C12H1 | Linker 1 | 19F3L3_{V}-Linker1-19F3H2_{V} | 14C12L1 |
| P1D7V03 | 14C12H1 | Linker2 | 19F3H2_{V}-Linker1-19F3L3_{V} | 14C12L1 |
| P1D7V04R | 14C12H1 | Linker2 | 19F3L3_{V}-Linker1-19F3H2_{V} | 14C12L1 |
| P1D7V07 | 19F3H2 | Linker 1 | 14C12H1_{V}-Linker1-14C12L1_{V} | 19F3L3 |
| P1D7V08 | 19F3H2 | Linker2 | 14C12H1_{V}-Linker1-14C12L1_{V} | 19F3L3 |

In the Table 1 above:
(1) Those with "V" label at lower right corner refer to the variable region of corresponding heavy chain or the variable region of corresponding light chain. For those without "V" label, the corresponding heavy or light chain is the full length comprising the constant region. The corresponding sequences described in the above examples are referred to for the amino acid sequences of these variable regions or the full length and the nucleotide sequences encoding them.
(2) The amino acid sequence of linker 1 is (GGGGS)₄ (the nucleotide sequence is SEQ ID NO: 80, and the amino acid sequence is SEQ ID NO: 79), and the amino acid sequence of linker 2 is (GGGGS)₃ (the nucleotide sequence is SEQ ID NO: 82, and the amino acid sequence is SEQ ID NO: 81).

### 2. Expression and purification of antibodies

The heavy chain cDNA sequence and the light chain cDNA sequence of P1D7V01 were each cloned into vector pUC57simple (provided by Genscript) to obtain plasmids pUC57simple-VP101H and pUC57simple-VP101L, respectively.

Plasmids pUC57simple-VP101H and pUC57simple-VP101L were enzyme-digested (HindIII&EcoRI), and heavy and light chains isolated by electrophoresis were subcloned into vector pcDNA3.1, and recombinant plasmids were extracted to co-transfect 293F cells. After 7 days of cell culture, the culture medium was separated by centrifugation at high speed, and the supernatant was concentrated and loaded onto a HiTrap MabSelect SuRe column. The protein was eluted in one step with an elution buffer. The target sample was isolated and the buffer was exchanged into PBS.

The purified antibodies P1D7V02R, P1D7V03, P1D7V04R, P1D7V07 and P1D7V08 were obtained according to the above expression and purification methods for P1D7V01.

### Example 8: Assay for binding activity of anti-CD73/anti-PD-1 bispecific antibodies to antigens by ELISA

### 1. Binding activity of P1D7V01, P1D7V02R, P1D7V03 and P1D7V04R to antigen PD-1-mFc determined by ELISA The method is specifically as follows.

A microplate was coated with PD-1-mFc at 0.5 µg/mL and incubated at 4 °C overnight. Then the microplate coated with antigens was washed once with PBST, and then blocked with a PBS solution containing 1% BSA as blocking solution at 37 °C for 2 h. After blocking, the microplate was washed 3 times with PBST. The antibodies serially diluted with PBST solution (the dilution gradients for the antibody are shown in Table 2) were added. The microplate containing the test antibodies was incubated at 37 °C for 30 min, and then washed 3 times with PBST. After washing, HRP-labeled goat anti-human IgG (H+L) (Jackson, Cat. No. 109-035-088) secondary antibody working solution diluted in a ratio of 1:5000 was added, and then the microplate was incubated at 37 °C for 30 min. After incubation, the plate was washed 4 times with PBST, TMB (Neogen, 308177) was added in the dark for chromogenesis for 5 min, and then a stop solution was added to terminate chromogenic reaction. The microplate was put into a microplate reader immediately, and the OD value of each well in the microplate was read at 450 nm. The data were analyzed and processed by SoftMax Pro 6.2.1.

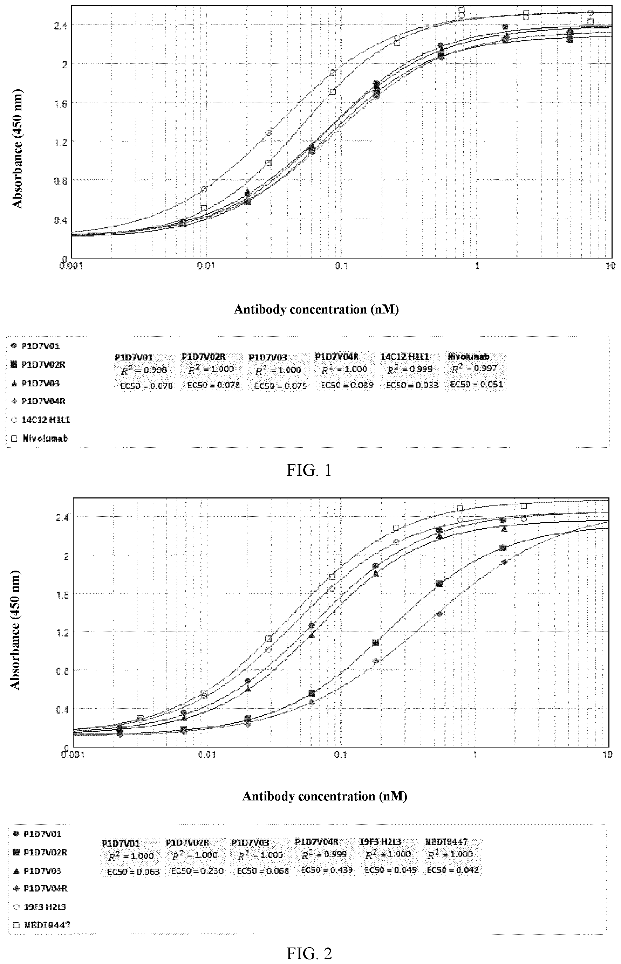

The results are shown in Table 2 and FIG. 1. It can be seen from the figure that P1D7V01, P1D7V02R, P1D7V03 and P1D7V04R can effectively bind to the antigen PD-1-mFc in a dose-dependent manner. The absorbance intensity of each dose is shown in Table 2. By quantitative analysis of the absorbance of the bound antibodies, the binding efficiency EC₅₀ values of the antibodies P1D7V01, P1D7V02R, P1D7V03, P1D7V04R, 14C12H1L1 and nivolumab (as a control) obtained by curve fitting were 0.078 nM, 0.078 nM, 0.075 nM, 0.089 nM, 0.033 nM and 0.051 nM, respectively.

The above experimental results showed that in the same experimental condition, the binding activities of P1D7V01, P1D7V02R, P1D7V03 and P1D7V04R to PD-1-mFc are comparable to that of the reference drugs 14C12H1L1 and nivolumab for the same target, suggesting that P1D7V01, P1D7V02R, P1D7V03, P1D7V04R have the activity of effectively binding to PD-1-mFc.

**Table 2. Binding of P1D7V01, P1D7V02R, P1D7V03, P1D7V04R, 14C12H1L1 and nivolumab to PD-1-mFc determined by ELISA**

| Antibody dilution concentration (µg/mL) | Antigen coating PD-1-mFc 0.5 µg/mL | | | | | | | | | | | |
|---|---|---|---|---|---|---|---|---|---|---|---|---|
| | P1D7V01 | | P1D7V02R | | P1D7V03 | | P1D7V04R | | 14C12H1L1 | | Nivolumab | |
| 1 | 2.345 | 2.300 | 2.169 | 2.323 | 2.406 | 2.292 | 2.225 | 2.393 | 2.441 | 2.609 | 2.386 | 2.473 |
| 1:3 | 2.461 | 2.295 | 2.114 | 2.361 | 2.288 | 2.303 | 2.199 | 2.302 | 2.414 | 2.548 | 2.441 | 2.600 |
| 1:9 | 2.279 | 2.085 | 2.063 | 2.094 | 2.135 | 2.178 | 2.075 | 2.043 | 2.461 | 2.525 | 2.519 | 2.572 |
| 1:27 | 1.838 | 1.765 | 1.701 | 1.696 | 1.777 | 1.764 | 1.659 | 1.659 | 2.236 | 2.295 | 2.196 | 2.219 |
| 1:81 | 1.153 | 1.097 | 1.089 | 1.120 | 1.149 | 1.154 | 1.062 | 1.131 | 1.858 | 1.954 | 1.711 | 1.712 |
| 1:243 | 0.629 | 0.684 | 0.570 | 0.582 | 0.675 | 0.686 | 0.584 | 0.592 | 1.251 | 1.318 | 0.980 | 0.965 |
| 1:729 | 0.361 | 0.364 | 0.334 | 0.349 | 0.360 | 0.370 | 0.340 | 0.341 | 0.683 | 0.709 | 0.521 | 0.498 |
| 0 | 0.201 | 0.198 | 0.187 | 0.210 | 0.197 | 0.197 | 0.213 | 0.257 | 0.208 | 0.199 | 0.196 | 0.190 |
| Secondary antibody | Goat anti-human IgG (H+L), HRP (1:5000) | | | | | | | | | | | |
| EC50(nM) | 0.078 | 0.078 | 0.075 | 0.089 | 0.033 | 0.051 | | | | | | |

### 2. Binding activity of P1D7V01, P1D7V02R, P1D7V03 and P1D7V04R to antigen human NT5E-biotin determined by ELISA

A microplate was coated with streptavidin at 2 µg/mL and then incubated at 4 °C overnight. After incubation, the microplate coated with streptavidin was washed once with PBST, and blocked with a PBS solution containing 1% BSA as a microplate blocking solution at 37 °C for 2 h. After blocking, the microplate was washed 3 times with PBST. Then, 0.5 µg/mL antigen human NT5E-Biotin was added and incubated at 37 °C for 30 min. Then, the plate was washed 3 times with PBST. The antibodies serially diluted with PBST solution (the dilution gradients for the antibody are shown in Table 3) were added to wells of the microplate. The microplate containing the test antibodies was incubated at 37 °C for 30 min, and then washed 3 times with PBST. After washing, HRP-labeled goat anti-human IgG (H+L) (Jackson, Cat. No. 109-035-088) secondary antibody working solution diluted in a ratio of 1:5000 was added, and the microplate was incubated at 37 °C for 30 min. After incubation, the plate was washed 4 times with PBST, TMB (Neogen, 308177) was added in the dark for chromogenesis for 5 min, and then a stop solution was added to terminate chromogenic reaction. The microplate was put into a microplate reader immediately, and the OD value of each well in the microplate was read at 450 nm. The data were analyzed and processed by SoftMax Pro 6.2.1.

The results are shown in Table 3 and FIG. 2. It can be seen from the figure that P1D7V01, P1D7V02R, P1D7V03 and P1D7V04R can effectively bind to the antigen human NT5E-biotin in a dose-dependent manner. The absorbance intensity of each dose is shown in Table 3. By quantitative analysis of the absorbance of the bound antibodies, the binding efficiency EC₅₀ values of the antibodies P1D7V01, P1D7V02R, P1D7V03, P1D7V04R, 19F3H2L3 and MEDI9447 (as a control antibody) obtained by curve fitting were 0.063 nM, 0.230 nM, 0.068 nM, 0.439 nM, 0.045 nM and 0.042 nM, respectively. The above experimental results showed that in the same experimental condition, the binding activities of bispecific antibodies P1D7V01, P1D7V02R, P1D7V03 and P1D7V04R to human NT5E-biotin are comparable to that of the reference drugs 19F3H2L3 and MEDI9447 for the same target, suggesting that P1D7V01, P1D7V02R, P1D7V03 and P1D7V04R have the activity of effectively binding to human NT5E-biotin.

**Table 3. Binding of P1D7V01, P1D7V02R, P1D7V03, P1D7V04R, 19F3H2L3 and MEDI9447 to human NT5E-biotin determined by ELISA**

| Antibody dilution concentration (µg/mL) | Antigen coating: SA (2 µg/mL) | | | | | | | | | | | |
|---|---|---|---|---|---|---|---|---|---|---|---|---|
| | Human NT5E-biotin (0.5 µg/mL) | | | | | | | | | | | |
| | P1D7V01 | | P1D7V02R | | P1D7V03 | | P1D7V04R | | 19F3 H2L3 | | MEDI9447 | |
| 0.333 | 2.465 | 2.254 | 2.125 | 2.024 | 2.245 | 2.311 | 1.992 | 1.864 | 2.345 | 2.413 | 2.460 | 2.570 |
| 1:3 | 2.310 | 2.207 | 1.718 | 1.680 | 2.202 | 2.194 | 1.385 | 1.397 | 2.366 | 2.376 | 2.434 | 2.528 |
| 1:9 | 1.899 | 1.856 | 1.090 | 1.086 | 1.834 | 1.782 | 0.886 | 0.898 | 2.164 | 2.120 | 2.219 | 2.345 |
| 1:27 | 1.276 | 1.239 | 0.556 | 0.554 | 1.199 | 1.132 | 0.464 | 0.465 | 1.681 | 1.631 | 1.729 | 1.810 |
| 1:81 | 0.704 | 0.654 | 0.289 | 0.279 | 0.610 | 0.601 | 0.235 | 0.230 | 1.048 | 0.978 | 1.091 | 1.166 |
| 1:243 | 0.363 | 0.333 | 0.183 | 0.178 | 0.308 | 0.302 | 0.155 | 0.156 | 0.548 | 0.504 | 0.547 | 0.587 |
| 1:729 | 0.199 | 0.197 | 0.138 | 0.141 | 0.183 | 0.184 | 0.130 | 0.122 | 0.283 | 0.278 | 0.288 | 0.305 |
| 0 | 0.135 | 0.119 | 0.115 | 0.117 | 0.116 | 0.119 | 0.115 | 0.111 | 0.120 | 0.126 | 0.121 | 0.121 |

| Secondary antibody | Goat anti-human IgG (H+L), HRP (1:5000) | | | | | | | | | | | |
|---|---|---|---|---|---|---|---|---|---|---|---|---|
| EC50(nM) | 0.063 | | 0.230 | | 0.068 | | 0.439 | | 0.045 | | 0.042 | |

### Example 9: Activity of anti-CD73/anti-PD-1 bispecific antibodies completing with human PD-L1-mFc for binding to human PD-1-mFc-biotin determined by competitive ELISA

A microplate was coated with human PD-L1-mFc (PD-L1 Genbank ID: NP_054862.1, mFc SEQ ID NO: 143) at 2 µg/mL and incubated at 4 °C overnight. After incubation, the microplate was blocked with a PBS solution containing 1% BSA at 37 °C for 2 h. After blocking, the plate was washed three times and dried. The antibody was serially diluted to 7 concentrations in a gradient ratio of 1:3 on a dilution plate with 10 µg/mL as the starting concentration, and a blank control was set. Then an equal volume of 0.3 µg/mL human PD-1-mFc-biotin solution was added, and the system was mixed well and incubated at room temperature for 20 min. Then the mixture after reaction was added to the coated microplate, and the microplate was incubated for at 37 °C for 30 min. After incubation, the plate was washed three times with PBST and dried. SA-HRP (KPL, 14-30-00) working solution was added, and the plate was incubated at 37 °C for 30 min. After incubation, the plate was washed four times and patted dry. Then TMB (Neogen, 308177) was added in the dark for chromogenesis for 5 min, and a stop solution was added to terminate chromogenic reaction. Then the microplate was put into a microplate reader immediately, and the OD value of each well in the microplate was read at 450 nm. The data were analyzed and processed by SoftMax Pro 6.2.1.

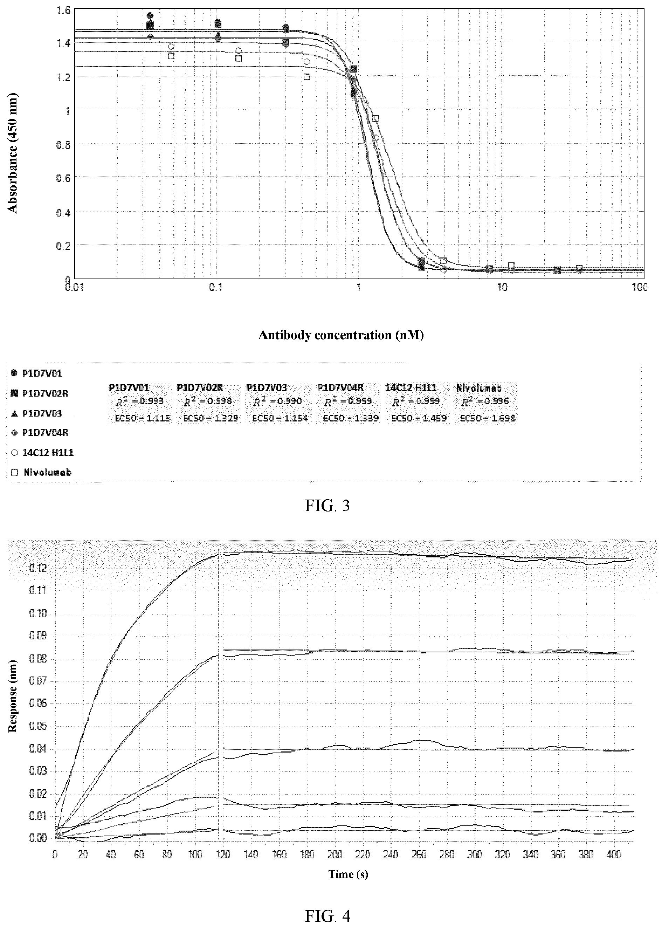

The results are shown in FIG. 3. The OD values for all the dosages are shown in Table 4. By quantitative analysis of the absorbance intensity of the bound antibody, the curve simulation was performed to give the binding efficiency EC₅₀ of the antibody (Table 4). The results showed that P1D7V01, P1D7V02R, P1D7V03, P1D7V04R, 14C12H1L1 and nivolumab (as a control) can effectively block the binding of the antigen human PD-1-mFc-biotin to its receptor human PD-L1-mFc in a dose-dependent manner. The EC₅₀ values of P1D7V01, P1D7V02R, P1D7V03, P1D7V04R, 14C12H1L1 and nivolumab for blocking the binding of human PD-1-mFc-biotin to its ligand human PD-L1-mFc were 1.115 nM, 1.329 nM, 1.154 nM, 1.339 nM, 1.459 nM and 1.698 nM, respectively.

**Table 4. Activity of P1D7V01, P1D7V02R, P1D7V03, P1D7V04R, 14C12H1L1 and nivolumab competing with human PD-L1-mFc for binding to human PD-1-mFc-biotin**

| Antibody dilution (µg/mL) | Antigen coating: human PD-L1-mFc 2 µg/mL | | | | | | | | | | | |
|---|---|---|---|---|---|---|---|---|---|---|---|---|
| | P1D7V01 | | P1D7V02R | | P1D7V03 | | P1D7V04R | | 14C12 H1L1 | | Nivolumab | |
| 5 | 0.049 | 0.047 | 0.047 | 0.048 | 0.048 | 0.048 | 0.048 | 0.051 | 0.046 | 0.047 | 0.057 | 0.057 |
| 1:3 | 0.050 | 0.050 | 0.051 | 0.051 | 0.052 | 0.050 | 0.052 | 0.054 | 0.048 | 0.050 | 0.075 | 0.076 |
| 1:9 | 0.070 | 0.065 | 0.097 | 0.104 | 0.067 | 0.068 | 0.105 | 0.107 | 0.053 | 0.055 | 0.107 | 0.100 |
| 1:27 | 1.056 | 1.109 | 1.253 | 1.216 | 1.109 | 1.121 | 1.187 | 1.158 | 0.817 | 0.849 | 1.014 | 0.875 |
| 1:81 | 1.460 | 1.505 | 1.414 | 1.372 | 1.453 | 1.491 | 1.424 | 1.337 | 1.263 | 1.299 | 1.235 | 1.152 |
| 1:243 | 1.474 | 1.551 | 1.503 | 1.505 | 1.449 | 1.447 | 1.442 | 1.382 | 1.316 | 1.383 | 1.387 | 1.207 |
| 1:729 | 1.499 | 1.604 | 1.501 | 1.493 | 1.569 | 1.452 | 1.436 | 1.424 | 1.336 | 1.403 | 1.347 | 1.284 |
| 0 | 1.399 | 1.283 | 1.467 | 1.456 | 1.288 | 1.252 | 1.343 | 1.342 | 1.302 | 1.391 | 1.265 | 1.156 |

| Human PD-1-mFc-biotin: 0.3 µg/mL | | | | | | | | | | | | |
|---|---|---|---|---|---|---|---|---|---|---|---|---|
| Secondary antibody | SA-HRP(1:4000) | | | | | | | | | | | |
| EC50(nM) | 1.115 | | 1.329 | | 1.154 | | 1.339 | | 1.459 | | 1.698 | |

### Example 10: Kinetic parameters for binding of anti-CD73/anti-PD-1 bispecific antibodies to antigen human PD-1-mFc determined by Fortebio system

The sample dilution buffer was PBST, 0.1% BSA, pH 7.4. The antibody was immobilized on an AHC sensor at a concentration of 5 µg/mL with an immobilization height of about 0.4 nm. The sensor was equilibrated in a buffer for 60 s, and the binding of the immobilized antibody on the sensor to the antigen PD-1-mFc at concentrations of 0.6-50 nM (three-fold dilution) was determined in 120 s. The protein was dissociated in the buffer for 180 s. The detection temperature was 37 °C, the detection frequency was 0.3 Hz, and the sample plate shaking rate was 1000 rpm. The data were analyzed by 1: 1 model fitting to obtain affinity constants.

The determination results of the affinity constants of the humanized antibodies P1D7V01, 14C12H1L1 and nivolumab (as a control antibody) for human PD-1-mFc are shown in Table 5, and the detection results are shown in FIG. 4, FIG. 5 and FIG. 6. The affinity constants of the humanized antibodies P1D7V01, 14C12H1L1 and nivolumab for human PD-1-mFc were 1.76E-10 M, 1.64E-10 M and 2.32E-10 M, respectively. The above experimental results show that the binding ability of P1D7V01 is comparable to that of 14C12H1L1 and nivolumab, suggesting that the humanized antibody P1D7V01 has stronger binding ability to human PD-1-mFc.

**Table 5. Determination of affinity constants of P1D7V01, 14C12H1L1 and nivolumab for PD-1-mFc**

| **Test antibodies** | **KD (M)** | **Kon (1/Ms)** | **SE (kon)** | **Kdis (1/s)** | **S E (kdis)** | **Rmax (nm)** |
|---|---|---|---|---|---|---|
| P1D7V01 | 1.76E-10 | 4.19E+05 | 1.52E+04 | 7.37E-05 | 3.12E-05 | 0.13-0.17 |
| 14C12H1L1 | 1.64E-10 | 4.55E+05 | 1.61E+04 | 7.47E-05 | 2.98E-05 | 0.24-0.28 |
| Nivolumab | 2.32E-10 | 5.85E+05 | 2.03E+04 | 1.36E-04 | 3.47E-05 | 0.02-0.14 |

| | | | | | | |
|---|---|---|---|---|---|---|
| K_{D} is the affinity constant; K_{D} = kdis/kon | | | | | | |

### Example 11: Kinetic parameters for binding of anti-CD73/anti-PD-1 bispecific antibodies to antigen human NT5E(1-552)-his determined by Fortebio system

The sample dilution buffer was PBST, pH 7.4. The antibody was immobilized on a Protein A sensor at a concentration of 5 µg/mL with the immobilization time of about 15 s. The sensor was equilibrated in a buffer for 120 s, and the binding of the immobilized antibody on the sensor to the antigen human NT5E(1-552)-his at concentrations of 3.125-200 nM (two-fold dilution) was determined for 120 s. The protein was dissociated in the buffer for 600 s. The sensor was refreshed with 10 mM Gly solution, pH 1.5. The detection temperature was 37 °C, the detection frequency was 0.6 Hz, and the sample plate shaking rate was 1000 rpm. The data were analyzed by 1:1 model fitting to obtain affinity constants.

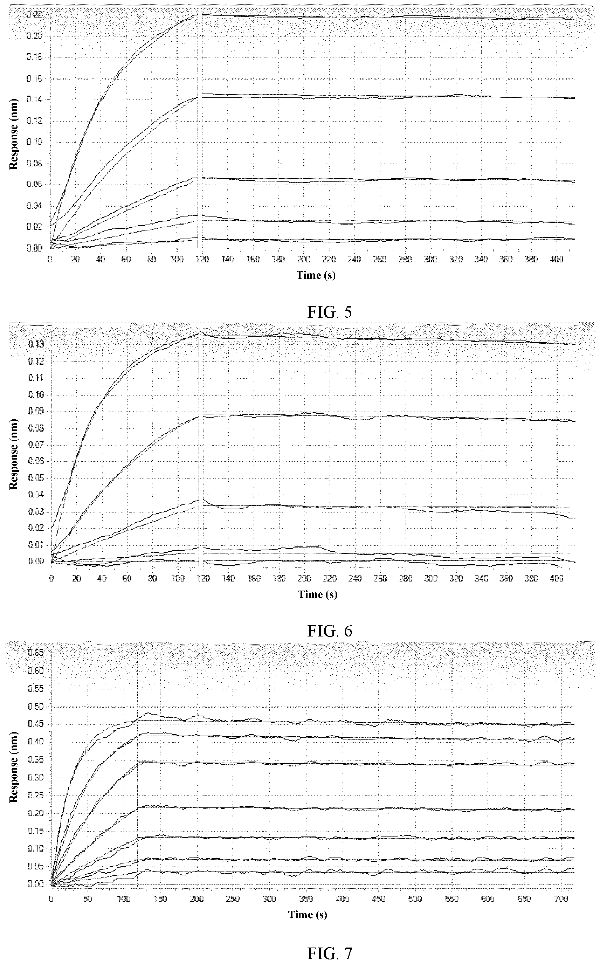

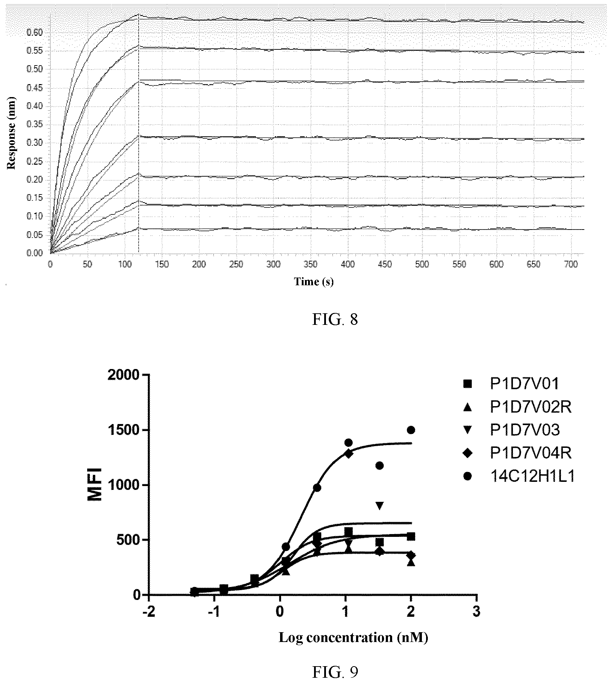

The determination results of the affinity constants of the humanized antibodies P1D7V01 and MEDI9447 (as a control antibody) for human NT5E(1-552)-his are shown in Table 6, and the detection results are shown in FIG. 7 and FIG. 8. The affinity constants of the humanized antibodies P1D7V01 and MEDI9447 for human NT5E(1-552)-his are 2.29E-10 M and 1.04E-10 M respectively.

The above experimental results showed that the binding ability of P1D7V01 is comparable to that of MEDI9447, suggesting that the humanized antibody P1D7V01 has stronger binding ability to human NTSE(1-552)-his.

**Table 6. Affinity constants of P1D7V01 and MEDI9447 for NTSE(1-552)-his**

| **Test antibodies** | **KD (M)** | **Kon (1/Ms)** | **S E (kon)** | **Kdis (1/s)** | **S E (kdis)** | **Rmax (nm)** |
|---|---|---|---|---|---|---|
| P1D7V01 | 2.29E-10 | 1.81E+05 | 2.30E+03 | 4.13E-05 | 4.54E-06 | 0.47-0.57 |
| MEDI 9447 | 1.04E-10 | 2.34E+05 | 3.20E+03 | 2.44E-05 | 5.02E-06 | 0.59-0.82 |

| | | | | | | |
|---|---|---|---|---|---|---|
| K_{D} is the affinity constant; K_{D} = kdis/kon | | | | | | |

### Example 12: Binding activity of anti-CD73/anti-PD-1 bispecific antibodies determined by FACS

### 1. Binding activity of anti-CD73/anti-PD-1 bispecific antibody to PD-1 on 293T-PD1 membrane surface determined by FACS

293T-PD1 cells in logarithmic growth phase were collected and transferred to a 1.5 mL centrifuge tube at 3 × 10⁵ cells/tube. 500 µL of PBSA was added, and the mixture was centrifuged at 5600 rpm for 5 min to remove the supernatant. 100 µL of antibodies diluted by PBSA (at the final concentrations of 100 nM, 33.33 nM, 11.11 nM, 3.7 nM, 1.23 nM, 0.41 nM, 0.14 nM and 0.05 nM, respectively) were added, respectively. The system was mixed gently and uniformly, and then was incubated on ice for 1 h. Then 500 µL of PBSA was added, and the mixture was centrifuged at 5600 rpm for 5 min to remove the supernatant. The 500-fold diluted FITC labeled goat anti-human IgG secondary antibody (Jackson, Cat. No. 109-095-098) was added to resuspend and mix well, and the mixture was incubated on ice in the dark for 0.5 h. 500 µL of PBSA was added, and the mixture was centrifuged at 5600 rpm for 5 min to remove the supernatant. At last, 200 µL of PBSA was added to resuspend cell precipitates, and the mixture was transferred to a flow tube for FACSCalibur detection.

The experimental results are shown in Table 7 and FIG. 9, and P1D7V01, P1D7V02R, P1D7V03, and P1D7V04R can specifically bind to PD-1 on the 293T-PD1 membrane surface in a dose-dependent manner, and compared with PD1 single-target control antibody 14C12H1L1, it is stronger than that of 14C12H1L1.

Under the same experimental conditions, EC₅₀ values of P1D7V01, P1D7V02R, P1D7V03 and P1D7V04R binding to 293T-PD1 were 1.000 nM, 1.075 nM, 1.377 nM and 1.57 nM, respectively, and the EC₅₀ value of 14C12H1L1 binding to 293T-PD1 was 2.111 nM.

The above experimental results showed that in the same experimental condition, P1D7V01, P1D7V02R, P1D7V03, P1D7V04R and 293T-PD1 all have binding activity superior to that of the PD1 single-target control antibody 14C12H1L1, suggesting that P1D7V01, P1D7V02R, P1D7V03 and P1D7V04R have the activity of effectively binding to PD-1 on the 293T-PD1 membrane surface.

**Table 7. Binding activity of P1D7V01, P1D7V02R, P1D7V03, P1D7V04R and 14C12H1L1 to PD-1 on 293T-PD1 cell surface determined by FACS.**

| **Concentration (nM)** | **0.05** | **0.14** | **0.41** | **1.23** | **3.70** | **11.11** | **33.33** | **100.00** | **EC50** |
|---|---|---|---|---|---|---|---|---|---|
| **14C12H1L1** | 35.69 | 57.70 | 129.42 | 437.62 | 974.97 | 1384.91 | 1174.75 | 1500.20 | **2.111** |
| **P1D7V01** | 26.65 | 58.65 | 150.57 | 307.19 | 530.77 | 577.76 | 479.34 | 531.15 | **1.000** |
| **P1D7V02R** | 23.44 | 42.98 | 108.44 | 217.56 | 404.33 | 426.06 | 405.29 | 301.40 | **1.075** |
| **P1D7V03** | 24.05 | 49.21 | 118.03 | 289.08 | 384.62 | 459.40 | 808.38 | 343.48 | **1.377** |
| **P1D7V04R** | 24.79 | 52.43 | 112.56 | 288.79 | 466.72 | 1284.73 | 400.33 | 360.79 | **1.57.0** |

### 2. Binding activity of anti-CD73/anti-PD-1 bispecific antibody to CD73 on MDA-MB-231 membrane surface determined by FACS

MDA-MB-231 cells in logarithmic phase were digested with conventional trypsin and transferred to a 1.5 mL centrifuge tube at 3 × 10⁵ cells/tube. 500 µL of PBSA was added, and the mixture was centrifuged at 5600 rpm for 5 min to remove the supernatant. 100 µL of antibodies diluted by PBSA (at the final concentrations of 100 nM, 33.33 nM, 11.11 nM, 3.7 nM, 1.23 nM, 0.41 nM, 0.14 nM and 0.05 nM, respectively) were added, respectively. The system was mixed gently and uniformly, and then was incubated on ice for 1 h. Then 500 µL of PBSA was added, and the mixture was centrifuged at 5600 rpm for 5 min to remove the supernatant. The 500-fold diluted FITC labeled goat anti-human IgG secondary antibody (Jackson, Cat. No. 109-095-098) was added to resuspend and mix well, and the mixture was incubated on ice in the dark for 0.5 h. 500 µL of PBSA was added, and the mixture was centrifuged at 5600 rpm for 5 min to remove the supernatant. At last, 200 µL of PBSA was added to resuspend cell precipitates, and the mixture was transferred to a flow tube for FACSCalibur detection.

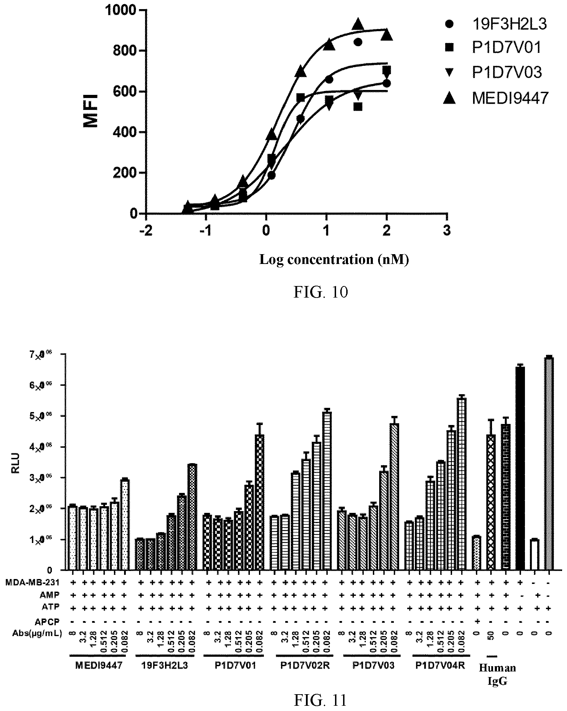

The experimental results are as shown in Table 8 and FIG. 10. The binding activities of P1D7V01 and P1D7V03 to CD73 on the MDA-MB-231 membrane surface were superior to that of 19F3H2L3, wherein P1D7V01 was superior to the reference drug MEDI9447 for the same target. In the same experimental condition, the EC₅₀ values of P1D7V01 and P1D7V03 binding to CD73 on the MDA-MB-231 membrane surface were 1.384 nM and 2.009 nM, respectively, and the EC₅₀ values of MEDI9447 and 19F3H2L3 binding to CD73 on MDA-MB-231 membrane surface were 1.589 nM and 2.773 nM, respectively. The above experimental results showed that P1D7V01, P1D7V03, 19F3H2L3 and the reference drug MEDI9447 for the same target can specifically bind to CD73 on the MDA-MB-231 membrane surface in a dose-dependent manner. The binding activities of P1D7V01 and P1D7V03 were superior to that of 19F3H2L3, wherein P1D7V01 was superior to the reference drug MEDI9447 for the same target. It was suggested that P1D7V01 and P1D7V03 have the activity of effectively binding to CD73 on the MDA-MB-231 membrane surface.

**Table. 8. Binding activity of P1D7V01, P1D7V03, MEDI9447 and 19F3H2L3 to CD73 on the MDA-MB-231 cell surface determined by FACS**

| **Concentration (nM)** | **0.05** | **0.14** | **0.41** | **1.23** | **3.70** | **11.11** | **33.33** | **100.00** | **EC50** |
|---|---|---|---|---|---|---|---|---|---|
| **MEDI9447** | 32.59 | 64.38 | 157.75 | 388.51 | 697.80 | 829.29 | 930.26 | 878.04 | 1.589 |
| **19F3H2L3** | 25.95 | 45.13 | 102.56 | 189.07 | 466.87 | 658.72 | 843.09 | 639.57 | 2.773 |
| **P1D7V01** | 23.37 | 38.02 | 76.84 | 271.72 | 571.56 | 560.81 | 525.91 | 705.50 | 1.384 |
| **P1D7V03** | 16.96 | 44.05 | 106.39 | 233.32 | 457.61 | 525.37 | 576.19 | 677.66 | 2.009 |

### Example 13: Detection of inhibition of anti-CD73/anti-PD-1 bispecific antibody on enzyme activity of CD73 on cell membrane surface

### 1. Detection of inhibition of anti-CD73/anti-PD-1 bispecific antibody on enzyme activity of CD73 on MDA-MB-231 membrane surface

The experimental procedures were as follows. MDA-MB-231 cells in logarithmic phase in good condition were taken, resuspended in a serum-free RPMI-1640 culture solution, and then counted. The MDA-MB-231 cells were seeded into a 96-well plate at 2 × 10⁴ cells/100 µL/well. The antibody was diluted with the serum-free RPMI-1640 culture solution (serial 2.5-fold dilution). The antibody was added to the 96-well plate at 50 µL/well, and the plate was incubated at 37 °C for 1 h. After 1 h, 50 µL of 1200 µM RPMI-1640-diluted AMP (TCL, Cat. No. A0157) was added to each well. After 3 h, 25 µL of cell culture supernatant was taken and transferred to a new 96-well plate, and 25 µL of 100 µM ATP (TCL, Cat. No. A0158) was added to each well. 50 µL of CTG (CellTiterGlo, promega, Cat. No. G8641) chromogenic solution was added to each well for chromogenesis, and relative fluorescence intensity RLU was read by a multi-label microplate tester (PerkinElmer 2140-0020).

The experimental results are as shown in FIG. 11. The AMP amounts of P1D7V01, P1D7V02R, P1D7V03 and P1D7V04R are comparable to that of the positive control MEDI9447 in concentration-dependent manners.

The above experimental results showed that the added AMP can be converted into adenosine A without antibodies by the enzyme activity of CD73 on the MDA-MB-231 cell surface, and that after the addition of the antibody, the enzyme-catalyzed activity was decreased due to the binding of antibodiesP1D7V01, P1D7V02R, P1D7V03 and P1D7V04R to CD73, so that the AMP was not converted into adenosine A. It was suggested that the antibodies effectively inhibit the enzyme activity reaction thereof in a non-substrate competition mode and reduce the production of adenosine.

### 2. Detection of inhibition of anti-CD73/anti-PD-1 bispecific antibody on enzyme activity of CD73 on U87-MG membrane surface

U87-MG cells in logarithmic phase in good condition were taken, resuspended in a serum-free RPMI-1640 culture solution, and then counted. The U87-MG cells were seeded into a 96-well plate at 2 × 10⁴ cells/100 µL/well. The antibody was diluted with the serum-free RPMI-1640 culture solution (serial 2.5-fold dilution). The antibody was added to the 96-well plate at 50 µL/well, and the plate was incubated at 37 °C for 1 h. After 1 h, 50 µL of 1200 µM RPMI-1640-diluted AMP was added to each well. After 3 h, 25 µL of cell culture supernatant was taken and transferred to a new 96-well plate, and 25 µL of 100 µM ATP was added to each well. 50 µL of CTG (CellTiterGlo) chromogenic solution was added to each well for chromogenesis, and relative fluorescence intensity RLU were read by a multi-label microplate tester (PerkinElmer 2140-0020).

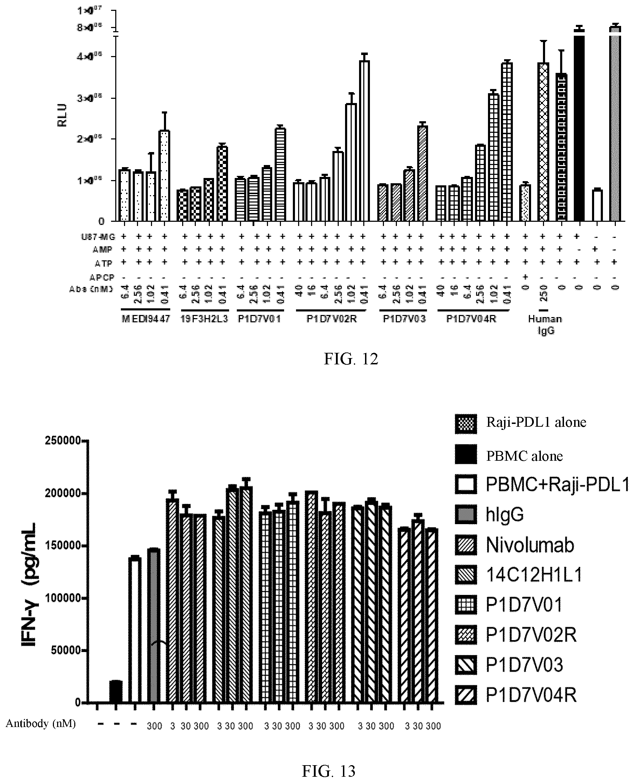

The experimental results are as shown in FIG. 12. The AMP amounts of P1D7V01, P1D7V02R, P1D7V03 and P1D7V04R are comparable to that of the positive control MEDI9447 in concentration-dependent manners.

The above experimental results showed that the added AMP can be converted into adenosine A without antibodies by the enzyme activity of CD73 on the U87-MG cell surface, and that after the addition of the antibody, the enzyme-catalyzed function was decreased due to the binding of antibodiesP1D7V01, P1D7V02R, P1D7V03 and P1D7V04R to CD73, so that the AMP was not converted into adenosine A. It was suggested that the antibodies effectively inhibit the enzyme activity reaction thereof in a non-substrate competition mode and reduce the production of adenosine.

### Example 14: Biological activity of anti-CD73/anti-PD-1 bispecific antibodies for promoting IFN-γ and IL-2 secretion determined by mixed lymphocyte reaction (MLR)

### 1. Biological activity of anti-CD73/anti-PD-1 bispecific antibodies for promoting IFN-y secretion in Raji-PDL1 mixed lymphocyte reaction system

Raji-PDL1 cells were normally subcultured. PBMCs were thawed, incubated with 10 mL of a 1640 complete medium, and stimulated with 0.5 µg/mL SEB (Dianotech, Cat. No. S010201) for two days. Raji-PDL1 cells were treated with 25 µg/mL MMC (Sigma, Cat. No. M4287) and placed in a 37 °C incubator for 1 h. PBMCs (peripheral blood mononuclear cells) stimulated with SEB for 2 days and Raji-PDL1 cells treated with MMC for 1 h were collected, washed twice with PBS, resuspended in the complete medium, and counted. The cells were separately added to a U-shaped 96-well plate at 100,000 cells/well. The antibodies were added according to the study design and cultured in an incubator for 3 days. After 3 days, the cell culture supernatant was collected and determined for IFN-y by ELISA.

As shown in FIG. 13, the mixed culture of human PBMCs and Raji-PDL1 cells significantly promoted the secretion of IFN-y from PBMCs, and the addition of antibodies to the mixed culture system significantly induced secretion of IFN-y in PBMCs. In terms of the level of promoting IFN-y secretion activity, the antibodies P1D7V01, P1D7V02R, P1D7V03 and P1D7V04R have the activity comparable to that of the parent PD-1 single-target antibody 14C12H1L1.

### 2. Biological activity of anti-CD73/anti-PD-1 bispecific antibodies for promoting IL-2 secretion in Raji-PDL1 mixed lymphocyte reaction system

Raji-PDL1 cells were normally subcultured. PBMCs were thawed, incubated with 10 mL of a 1640 complete medium, and stimulated with SEB (0.5 µg/mL) for two days. Raji-PDL1 cells were treated with 25 µg/mL MMC and placed in a 37 °C incubator for 1 h. PBMCs (peripheral blood mononuclear cells) stimulated with SEB for 2 days and Raji-PDL1 cells treated with MMC for 1 h were collected, washed twice with PBS, resuspended in the complete medium, and counted. The cells were separately added to a U-shaped 96-well plate at 100,000 cells/well. The antibodies were added according to the study design and cultured for 3 days. The cell culture supernatant was collected and determined for IL-2 by ELISA.

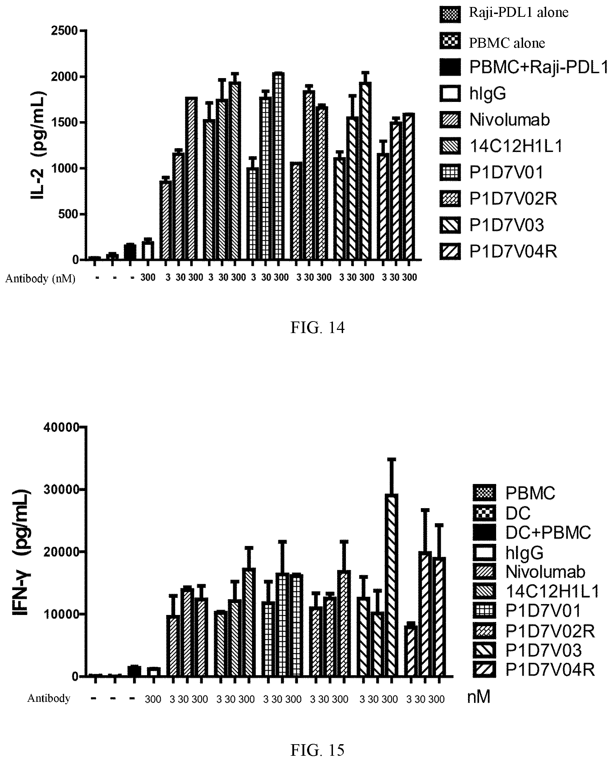

As shown in FIG. 14, the mixed culture of human PBMCs and Raji-PDL1 cells had certain promoting effect on the secretion of IL-2 in PBMCs, and the simultaneous addition of antibodies to the mixed culture system significantly induced IL-2 secretion in PBMCs in a significant dose-dependent manner. In terms of the level of promoting IL-2 secretion activity, the bifunctional antibodies P1D7V01, P1D7V02R, P1D7V03 and P1D7V04R have the activity at low concentrations slightly lower than that of the parent PD-1 single-target antibody 14C12H1L1, and the activity at medium-high concentrations comparable to that of the parent PD-1 single-target antibody 14C12H1L1. Compared with the PD1 target positive control drug nivolumab, P1D7V01, P1D7V02R, P1D7V03 and P1D7V04R have better IL-2 secretion promotion potential at three different antibody concentration levels.

### 3. Bioactivity detection of anti-CD73/anti-PD-1 bispecific antibody promoting DC mixed lymph reaction system to secrete IFN-y

PBMCs in normal human peripheral blood were isolated, resuspended in a complete medium, and seed into a culture dish. The culture dish was placed in an incubator overnight for culture. The suspended PBMCs were collected and removed. The adherent cells at the bottom of the dish were washed with PBS buffer and then subjected to DC maturation induction. 10 mL of RPMI1640 complete medium containing GM-CSF and IL-4 was added to each dish with GM-CSF and IL-4 each at a concentration of 1000 U/mL. The dishes were cultured in a 37 °C, 5% carbon dioxide incubator for three days. Then half of the medium was exchanged, 1000 U/mL GM-CSF and IL-4 each was added, and the dishes were put into a 37 °C, 5% carbon dioxide incubator for continuous culture for three days. After three days, half amount of the medium was changed again, 1000 U/mL GM-CSF and IL-4 each, and 100 U/mL TNF-α were added, and the dishes were cultured for another two days. PBMCs from other donors were freshly isolated and seeded into a 96-well plate at 100,000 cells/well after cell counting. DCs induced to mature were collected and washed once with the complete medium. The cells were counted and seed into the 96-well plate containing PBMC at 10,000 cells/well. The antibodies were added according to the study design. The system was mixed well and cultured in a 37 °C, 5% carbon dioxide incubator for 5 days for co-culture. After 5 days, the cell culture supernatant was collected and quantitatively determined for IFN-y by ELISA.

The results are as shown in FIG. 15. Compared with the culture of DCs or PBMCs alone, the mixed culture of DCs and PBMCs significantly promoted the secretion of IFN-γ; and compared with an isotype control, IFN-y secretion level was further significantly improved by the addition of the anti-CD73/anti-PD-1 bispecific antibody based on the mixed culture of DCs and PBMCs.

Biological activity of anti-CD73/anti-PD-1 bispecific antibodies for promoting IL-2 secretion in DC mixed lymphocyte reaction system

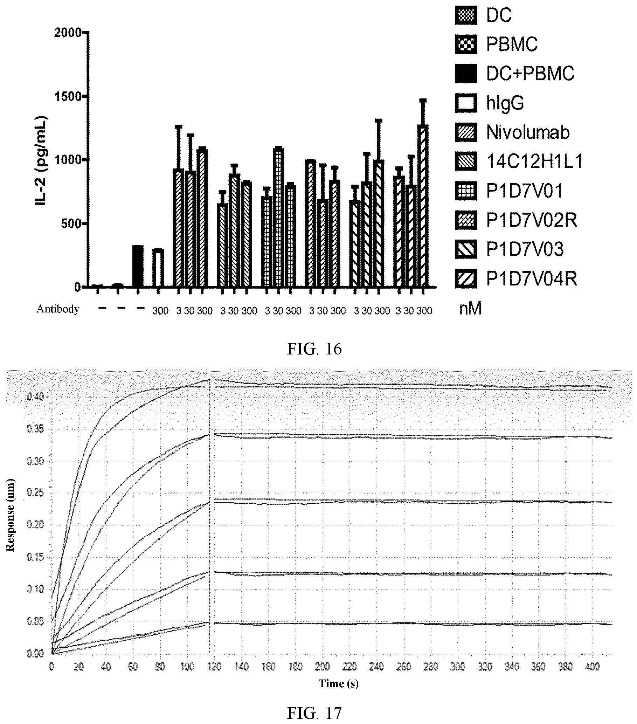

PBMCs in normal human peripheral blood were isolated, resuspended in a complete medium, and seed into a culture dish. The culture dish was placed in an incubator overnight for culture. The suspended PBMCs were collected and removed. The adherent cells at the bottom of the dish were washed with PBS buffer and then subjected to DC maturation induction. 10 mL of RPMI1640 complete medium containing GM-CSF and IL-4 was added to each dish with GM-CSF and IL-4 each at a concentration of 2000 U/mL. The dishes were cultured in a 37 °C, 5% carbon dioxide incubator for three days. Then half amount of the medium was changed, 50 ng/mL IFN-y and 100 ng/mL LPS were added, and the dishes were cultured for another two days. PBMCs from other donors were thawed and seeded into a 96-well plate at 100,000 cells/well after cell counting. DCs induced to mature were collected and washed once with the complete medium. The cells were counted and seed into the 96-well plate containing PBMCs at 10,000 cells/well. The antibodies were added according to the study design. The system was mixed well and cultured in a 37 °C, 5% carbon dioxide incubator for 5 days for co-culture. After 5 days, the cell culture supernatant was collected and quantitatively determined for IL-2 by ELISA. The results are as shown in FIG. 16. Compared with the culture of DCs or PBMCs alone, the mixed culture of DCs and PBMCs significantly promoted the secretion of IL-2; and compared with an isotype control, IL-2 secretion level was further significantly improved by the addition of the anti-CD73/anti-PD-1 bispecific antibody based on the mixed culture of DCs and PBMCs.

### Example 15: Preparation of anti-PD-1/CD73 bispecific antibodies NTPDV1, NTPDV2, NTPDV3 and NTPDV4

The structural patterns of the bispecific antibodies NTPDV1, NTPDV2, NTPDV3 and NTPDV4 are in the Morrison format (IgG-scFv), i.e., C termini of two heavy chains of one IgG antibody are separately linked to the scFv fragment of another antibody via linkers. The components for the heavy and light chain design are shown in Table 9 below. NTPDV1, NTPDV2, NTPDV3 and NTPDV4 are known as NTPDV1(hG1TM), NTPDV2(hG1TM), NTPDV3(hG1TM) and NTPDV4(hG1TM) herein and in Chinese Patent Application No. 202110270671.X, because the constant regions of the immunoglobulin moieties thereof have introduced amino acid mutations to eliminate their binding activity to FcyR.

**Table 9. Sequence design of NTPDV1, NTPDV2, NTPDV3 and NTPDV4**

| | Heavy chain | | | Light chain |
|---|---|---|---|---|
| | Immunoglobulin moiety | Linker | scFv moiety | |
| NTPDV1 | 19F3H2 (hG1TM) | Linker 1 | 14C12H1_{V}-Linker 2-14C12L1_{V} | 19F3L3 |
| NTPDV2 | | | 14C12H1_{V}-Linker 1-14C12L1_{V} | |
| NTPDV3 | | | 14C12H1_{V}-Linker 2-14C12L1_{V} | 19F3L2 |
| NTPDV4 | | | 14C12H1_{V}-Linker1-14C12L1_{V} | |

In the Table 9 above:
Those with "V" label at lower right corner refer to the variable region of corresponding heavy chain or the variable region of corresponding light chain. For those without "V" label, the corresponding heavy or light chain is the full length comprising the constant region. The corresponding sequences described in the above examples are referred to for the amino acid sequences of these variable regions or the full length and the nucleotide sequences encoding them.

The amino acid sequence of linker1 consists of 4 repeats of (GGGGS), i.e., (GGGGS)₄ or (G₄S)₄ (nucleotide sequence SEQ ID NO: 80, and amino acid sequence SEQ ID NO: 79). The amino acid sequence of linker2 consists of 3 repeats of (GGGGS), i.e., (GGGGS)₄ or (G₄S)₃ (nucleotide sequence SEQ ID NO: 82, and amino acid sequence SEQ ID NO: 81). The 3 CDR sequences of the light chains 19F3L2 and 19F3L3 of the immunoglobulin moiety in NTPDV1, NTPDV2, NTPDV3 and NTPDV4 are identical to the light chain CDR sequences of 19F3.

The 3 CDR sequences of 19F3H2(hG1TM) of the immunoglobulin moiety in NTPDV1, NTPDV2, NTPDV3 and NTPDV4 are identical to the heavy chain CDR sequences of 19F3.

The CDR sequences of 14C12H1V-Linker2-14C12L1V and 14C12H1V-Linker1-14C12L1V of the scFv moiety in NTPDV1, NTPDV2, NTPDV3 and NTPDV4 are identical to the heavy chain CDRs and light chain CDRs of 14C12.

The amino acid sequences of the heavy chains of NTPDV2 and NTPDV4 are identical and are marked as NTPDH2/4 (SEQ ID NO: 83), and the nucleotide sequence of the heavy chains of NTPDV2 or NTPDV4 is SEQ ID NO: 84.

The amino acid sequences of the heavy chains of NTPDV1 and NTPDV3 are identical and are marked as NTPDH1/3 (SEQ ID NO: 85), and the nucleotide sequence of the heavy chains of NTPDV1 or NTPDV3 is SEQ ID NO: 86.

The 3 CDR sequences of 19F3L3 and 19F3L2 of the immunoglobulin moiety in NTPDV1, NTPDV2, NTPDV3 and NTPDV4 are identical to the three CDRs of the light chain of antibody 19F3.

The amino acid sequence of the light chain 19F3L3 of the immunoglobulin moiety in NTPDV1 or NTPDV2 is identical to the light chain sequence (SEQ ID NO: 28) of antibody 19F3H2L3(G1M), and the nucleotide sequence of the light chain 19F3L3 of the immunoglobulin moiety in NTPDV1 or NTPDV2 is SEQ ID NO: 27. The amino acid sequence of the light chain 19F3L2 of the immunoglobulin moiety in NTPDV3 or NTPDV4 is SEQ ID NO: 96, and the nucleotide sequence of the light chain 19F3L2 of the immunoglobulin moiety in NTPDV3 or NTPDV4 is SEQ ID NO: 100.
(1) NTPDV1, of which the heavy chain has an amino acid sequence set forth in SEQ ID NO: 85, the light chain has an amino acid sequence set forth in SEQ ID NO: 28, the linker 1 has an amino acid sequence set forth in SEQ ID NO: 79, 14C12H1V has an amino acid sequence set forth in SEQ ID NO: 66, the linker 2 has an amino acid sequence set forth in SEQ ID NO: 81, and 14C12L1V has an amino acid sequence set forth in SEQ ID NO: 68;
(2) NTPDV2, of which the heavy chain has an amino acid sequence set forth in SEQ ID NO: 85, the light chain has an amino acid sequence set forth in SEQ ID NO: 28, the linker 1 has an amino acid sequence set forth in SEQ ID NO: 79, 14C12H1V has an amino acid sequence set forth in SEQ ID NO: 66, the linker 1 has an amino acid sequence set forth in SEQ ID NO: 79, and 14C12L1V has an amino acid sequence set forth in SEQ ID NO: 68;
(3) NTPDV3, of which the heavy chain has an amino acid sequence set forth in SEQ ID NO: 85, the light chain has an amino acid sequence set forth in SEQ ID NO: 96, the linker 1 has an amino acid sequence set forth in SEQ ID NO: 79, 14C12H1V has an amino acid sequence set forth in SEQ ID NO: 66, the linker 2 has an amino acid sequence set forth in SEQ ID NO: 81, and 14C12L1V has an amino acid sequence set forth in SEQ ID NO: 68; and
(4) NTPDV4, of which the heavy chain has an amino acid sequence set forth in SEQ ID NO: 85, the light chain has an amino acid sequence set forth in SEQ ID NO: 96, the linker 1 has an amino acid sequence set forth in SEQ ID NO: 79, 14C12H1V has an amino acid sequence set forth in SEQ ID NO: 66, the linker 1 has an amino acid sequence set forth in SEQ ID NO: 79, and 14C12L1V has an amino acid sequence set forth in SEQ ID NO: 68.

### Expression and purification of antibodies

The heavy chain cDNA sequences of NTPDV1 and NTPDV3, the heavy chain cDNA sequences of NTPDV2 and NTPDV4, and the light chain cDNA sequences thereof were separately cloned into vector pUC57simple (provided by Genscript) to obtain plasmids pUC57simple-NTPDH2/4, pUC57simple-NTPDH1/3, pUC57simple-19F3L3 and pUC57simple-19F3L2, respectively.

Plasmid pUC57simple-NTPDH2/4 and plasmid pUC57simple-19F3L3, plasmid pUC57simple-NTPDH2/4 and plasmid pUC57simple-19F3L2, plasmid pUC57simple-NTPDH1/3 and plasmid pUC57simple-19F3L3, and plasmid pUC57simple-NTPDH1/3 and plasmid pUC57simple-19F3L2 were digested (HindIII&EcoRI). The recovered heavy and light chains were separately subcloned into vector pcDNA3.1 to obtain plasmids pcDNA3.1-NTPDH2/4 and pcDNA3.1-19F3L3, plasmids pcDNA3.1-NTPDH2/4 and pcDNA3.1-19F3L2, plasmids pcDNA3.1-NTPDH1/3 and pcDNA3.1-19F3L3, and plasmids pcDNA3.1-NTPDH1/3 and pcDNA3.1-19F3L2. The recombinant plasmids were extracted and co-transfected into 293F cells. After 7 days of cell culture, the culture medium was separated by centrifugation at high speed, and the supernatant was concentrated and loaded onto a HiTrap MabSelect SuRe column. The protein was eluted in one step with an elution buffer. The target sample was isolated and the buffer was exchanged into PBS.

The purified antibodies NTPDV1, NTPDV2, NTPDV3 and NTPDV4 were obtained according to the expression and purification methods mentioned in the above preparation examples.

### Example 16: Assay for binding activity of anti-CD73/anti-PD-1 bispecific antibodies to antigens by ELISA

### 1. Binding activity of NTPDV1, NTPDV2, NTPDV3 and NTPDV4 to antigen PD-1-mFc determined by ELISA

A microplate was coated with PD-1-mFc (0.5 µg/mL) and incubated at 4 °C overnight. Then the microplate coated with antigens was washed once with PBST, and then blocked with a PBS solution containing 1% BSA as blocking solution at 37 °C for 2 h. After blocking, the microplate was washed 3 times with PBST. The antibodies serially diluted with PBST solution (the dilution gradients for the antibody are shown in Table 2) were added. The microplate containing the test antibodies was incubated at 37 °C for 30 min, and then washed 3 times with PBST. After washing, HRP-labeled goat anti-human IgG Fc (Jackson, Cat. No. 109-035-098) secondary antibody working solution diluted in a ratio of 1:5000 was added, and then the microplate was incubated at 37 °C for 30 min. After incubation, the plate was washed 4 times with PBST, TMB (Neogen, 308177) was added in the dark for chromogenesis for 8 min, and then a stop solution was added to terminate chromogenic reaction. The microplate was put into a microplate reader immediately, and the OD value of each well in the microplate was read at 450 nm. The data were analyzed and processed by SoftMax Pro 6.2.1.

The results are shown in Table 10. It can be seen that NTPDV1, NTPDV2, NTPDV3 and NTPDV4 can effectively bind to the antigen PD-1-mFc in a dose-dependent manner. The absorbance intensity of each dose is shown in Table 10. By quantitative analysis of the absorbance of the bound antibodies, the binding efficiency EC₅₀ values of the antibodies NTPDV1, NTPDV2, NTPDV3, NTPDV4 and 14C12H1L1(hG1TM) (as a control) obtained by curve fitting were 0.101 nM, 0.119 nM, 0.110 nM, 0.123 nM and 0.031 nM, respectively.

The above experimental results showed that in the same experimental condition, the binding activities of NTPDV1, NTPDV2, NTPDV3 and NTPDV4 to PD-1-mFc are comparable to that of the reference drugs 14C12H1L1(hG1TM) for the same target, suggesting that NTPDV1, NTPDV2, NTPDV3 and NTPDV4 have the activity of effectively binding to PD-1-mFc.

**Table 10. Binding of NTPDV1, NTPDV2, NTPDV3, NTPDV4 and 14C12H1L1(hG1TM) to PD-1-mFc determined by ELISA**

| Antibody dilution concentration (nM) | Antigen coating PD-1-mFc 0.5 µg/mL | | | | | | | | | |
|---|---|---|---|---|---|---|---|---|---|---|
| | NTPDV1 | | NTPDV2 | | NTPDV3 | | NTPDV4 | | 14C12H1L1 (hG1TM) | |
| 5 | 2.222 | 2.231 | 2.249 | 2.249 | 2.204 | 2.171 | 2.244 | 2.289 | 2.399 | 2.524 |
| 1:3 | 2.206 | 2.195 | 2.160 | 2.223 | 2.211 | 2.167 | 2.222 | 2.209 | 2.361 | 2.441 |
| 1:9 | 2.039 | 2.027 | 1.922 | 2.013 | 2.010 | 1.971 | 1.920 | 1.944 | 2.273 | 2.401 |
| 1:27 | 1.514 | 1.530 | 1.425 | 1.446 | 1.489 | 1.414 | 1.417 | 1.464 | 2.071 | 2.091 |
| 1:81 | 0.880 | 0.844 | 0.804 | 0.802 | 0.829 | 0.807 | 0.794 | 0.804 | 1.644 | 1.691 |
| 1:243 | 0.400 | 0.396 | 0.363 | 0.369 | 0.386 | 0.381 | 0.373 | 0.379 | 1.006 | 1.059 |
| 1:729 | 0.183 | 0.184 | 0.176 | 0.174 | 0.175 | 0.174 | 0.170 | 0.178 | 0.503 | 0.531 |
| 0 | 0.053 | 0.056 | 0.055 | 0.056 | 0.056 | 0.056 | 0.054 | 0.059 | 0.057 | 0.059 |

| Secondary antibody | HRP-labeled goat anti-human IgG Fc (1:5000) | | | | | | | | | |
|---|---|---|---|---|---|---|---|---|---|---|
| EC50 (nM) | 0.101 | | 0.119 | | 0.110 | | 0.123 | | 0.031 | |

### 2. Binding activity of NTPDV1, NTPDV2, NTPDV3 and NTPDV4 to antigen human NT5E-biotin determined ELISA

A microplate was coated with streptavidin SA (2 µg/mL) and then incubated at 4 °C overnight. After incubation, the microplate coated with streptavidin was washed once with PBST, and blocked with a PBS solution containing 1% BSA as a microplate blocking solution at 37 °C for 2 h. After blocking, the microplate was washed 3 times with PBST. Then, 0.5 µg/mL antigen human NT5E-Biotin was added and incubated at 37 °C for 30 min. Then, the plate was washed 3 times with PBST. The antibodies serially diluted with PBST solution (the dilution gradients for the antibody are shown in Table 11) were added to wells of the microplate. The microplate containing the test antibodies was incubated at 37 °C for 30 min, and then washed 3 times with PBST. After washing, HRP-labeled goat anti-human IgG Fc (Jackson, Cat. No. 109-035-098) secondary antibody working solution diluted in a ratio of 1:5000 was added, and the microplate was incubated at 37 °C for 30 min. After incubation, the plate was washed 4 times with PBST, TMB (Neogen, 308177) was added in the dark for chromogenesis for 7 min, and then a stop solution was added to terminate chromogenic reaction. The microplate was put into a microplate reader immediately, and the OD value of each well in the microplate was read at 450 nm. The data were analyzed and processed by SoftMax Pro 6.2.1.

The results are shown in Table 11. It can be seen that NTPDV1, NTPDV2, NTPDV3, NTPDV4 and 19F3H2L3(hG1M) can effectively bind to the antigen human NT5E-biotin in a dose-dependent manner (the absorbance intensity of each dose is shown in Table 11). By quantitative analysis of the absorbance of the bound antibodies, the binding efficiency EC₅₀ values of the antibodies NTPDV1, NTPDV2, NTPDV3, NTPDV4 and 19F3H2L3(hG1M) (as a control antibody) obtained by curve fitting were 0.079 nM, 0.082 nM, 0.084 nM, 0.077 nM and 0.029 nM, respectively.

The above experimental results showed that in the same experimental condition, the binding activities of bispecific antibodies NTPDV1, NTPDV2, NTPDV3 and NTPDV4 to human NT5E-biotin are comparable to that of the reference drug 19F3H2L3(hG1M) for the same target, suggesting that NTPDV1, NTPDV2, NTPDV3 and NTPDV4 have the activity of effectively binding to human NT5E-biotin.

**Table 11. Binding of NTPDV1, NTPDV2, NTPDV3, NTPDV4 and 19F3H2L3(hG1M) to human NT5E-biotin determined by ELISA**

| Antibody dilution concentration (nM) | Antigen coating SA: 2 µg/mL | | | | | | | | | |
|---|---|---|---|---|---|---|---|---|---|---|
| | Human NT5E-biotin (0.5 µg/mL) | | | | | | | | | |
| | NTPDV1 | | NTPDV2 | | NTPDV3 | | NTPDV4 | | 19F3H2L3 (hG1M) | |
| 5 | 2.286 | 2.300 | 2.322 | 2.333 | 2.364 | 2.286 | 2.298 | 2.291 | 2.371 | 2.383 |
| 1:3 | 2.412 | 2.347 | 2.450 | 2.409 | 2.381 | 2.412 | 2.427 | 2.421 | 2.420 | 2.506 |
| 1:9 | 2.227 | 2.177 | 2.226 | 2.238 | 2.230 | 2.081 | 2.186 | 2.222 | 2.322 | 2.345 |
| 1:27 | 1.795 | 1.729 | 1.744 | 1.746 | 1.768 | 1.698 | 1.734 | 1.759 | 2.125 | 2.162 |
| 1:81 | 1.071 | 1.070 | 1.103 | 1.058 | 1.068 | 1.025 | 1.095 | 1.125 | 1.649 | 1.752 |
| 1:243 | 0.528 | 0.527 | 0.540 | 0.551 | 0.552 | 0.534 | 0.552 | 0.611 | 1.067 | 1.143 |
| 1:729 | 0.273 | 0.274 | 0.271 | 0.275 | 0.279 | 0.267 | 0.275 | 0.309 | 0.542 | 0.601 |
| 0 | 0.092 | 0.092 | 0.090 | 0.087 | 0.092 | 0.090 | 0.092 | 0.095 | 0.112 | 0.206 |

| Secondary antibody | HRP-labeled goat anti-human IgG Fc (1:5000) | | | | | | | | | |
|---|---|---|---|---|---|---|---|---|---|---|
| EC50 (nM) | 0.079 | | 0.082 | | 0.084 | | 0.077 | | 0.029 | |

### Example 17: Activity of anti-CD73/anti-PD-1 bispecific antibodies completing with human PD-L1-mFc for binding to human PD-1-mFc-biotin determined by competitive ELISA

A microplate was coated with human PD-L1-mFc (PD-L1 Genbank ID: NP_054862.1, mFc SEQ ID NO: 89) at 2 µg/mL and incubated at 4 °C overnight. After incubation, the microplate was blocked with a PBS solution containing 1% BSA at 37 °C for 2 h. After blocking, the plate was washed once and dried. The antibody was serially diluted to 7 concentrations in a gradient ratio of 1:3 on a dilution plate with 10 µg/mL as the starting concentration, and a blank control was set. Then an equal volume of 0.3 µg/mL human PD-1-mFc-biotin solution was added, and the system was mixed well and incubated at room temperature for 10 min. Then the mixture after reaction was added to the coated microplate, and the microplate was incubated for at 37 °C for 30 min. After incubation, the plate was washed three times with PBST and dried. SA-HRP (KPL, 14-30-00) working solution was added, and the plate was incubated at 37 °C for 30 min. After incubation, the plate was washed four times and patted dry. Then TMB (Neogen, 308177) was added in the dark for chromogenesis for 5 min, and a stop solution was added to terminate chromogenic reaction. Then the microplate was put into a microplate reader immediately, and the OD value of each well in the microplate was read at 450 nm. The data were analyzed and processed by SoftMax Pro 6.2.1.

The OD values for all the dosages are shown in Table 12. By quantitative analysis of the absorbance intensity of the bound antibody, the curve simulation was performed to give the binding efficiency EC₅₀ of the antibody (Table 12).

The results showed that NTPDV1, NTPDV2, NTPDV3, NTPDV4 and 14C12H1L1(hG1TM) (as a control) can effectively block the binding of the antigen human PD-1-mFc-biotin to its receptor human PD-L1-mFc in a dose-dependent manner. The EC₅₀ values of NTPDV1, NTPDV2, NTPDV3, NTPDV4 and 14C12H1L1(hG1TM) for blocking the binding of human PD-1-mFc-biotin to its ligand human PD-L1-mFc were 2.249 nM, 2.253 nM, 2.332 nM, 2.398 nM, 2.216 nM and 2.231 nM, respectively.

**Table 12. Activity of NTPDV1, NTPDV2, NTPDV3, NTPDV4 and 14C12H1L1(hG1TM) competing with human PD-L1-mFc for binding to human PD-1-mFc-biotin**

| Antibody dilution (nM) | Antigen coating: human PD-L1-mFc 2 µg/mL | | | | | | | | | |
|---|---|---|---|---|---|---|---|---|---|---|
| | NTPDV1 | | NTPDV2 | | NTPDV3 | | NTPDV4 | | 14C12H1L1 (hG1TM) | |
| 50 | 0.086 | 0.082 | 0.084 | 0.081 | 0.077 | 0.087 | 0.076 | 0.076 | 0.081 | 0.077 |
| 1:3 | 0.083 | 0.083 | 0.083 | 0.084 | 0.083 | 0.085 | 0.084 | 0.090 | 0.087 | 0.082 |
| 1:9 | 0.202 | 0.195 | 0.204 | 0.204 | 0.195 | 0.183 | 0.189 | 0.214 | 0.120 | 0.119 |
| 1:27 | 0.604 | 0.616 | 0.564 | 0.593 | 0.628 | 0.598 | 0.521 | 0.584 | 0.576 | 0.634 |
| 1:81 | 0.837 | 0.821 | 0.790 | 0.809 | 0.816 | 0.884 | 0.782 | 0.768 | 0.797 | 0.871 |
| 1:243 | 0.946 | 0.938 | 0.822 | 0.929 | 0.895 | 0.851 | 0.926 | 0.891 | 0.880 | 0.934 |
| 1:729 | 0.956 | 0.938 | 0.884 | 0.878 | 0.928 | 0.923 | 0.854 | 0.902 | 0.901 | 0.964 |
| 0 | 1.012 | 0.952 | 0.891 | 0.966 | 0.918 | 0.983 | 0.919 | 0.966 | 0.904 | 0.954 |

| Human PD-1-mFc-biotin: 0.3 µg/mL | | | | | | | | | | |
|---|---|---|---|---|---|---|---|---|---|---|
| Secondary antibody SA-HRP (1:4000) | | | | | | | | | | |
| EC50 (nM) | 2.253 | | 2.332 | | 2.398 | | 2.216 | | 2.231 | |

### Example 18: Kinetic parameters for binding of anti-CD73/anti-PD-1 bispecific antibodies to antigen human PD-1-mFc determined by Fortebio system

The sample dilution buffer was PBS (0.02% Tween-20, 0.1% BSA, pH 7.4). PD1-mFc was immobilized on the AMC sensor at a concentration of 5 µg/mL with an immobilization height of about 0.1 nM (time 60 s). The sensor was equilibrated in a buffer for 60 s, and the binding of the immobilized PD1-mFc on the sensor to the antibodies at concentrations of 0.62-50 nM (three-fold dilution) was determined for 120 s. The protein was dissociated in the buffer for 300 s. The detection temperature was 30 °C, the detection frequency was 0.3 Hz, and the sample plate shaking rate was 1000 rpm. The data were analyzed by 1: 1 model fitting to obtain affinity constants.

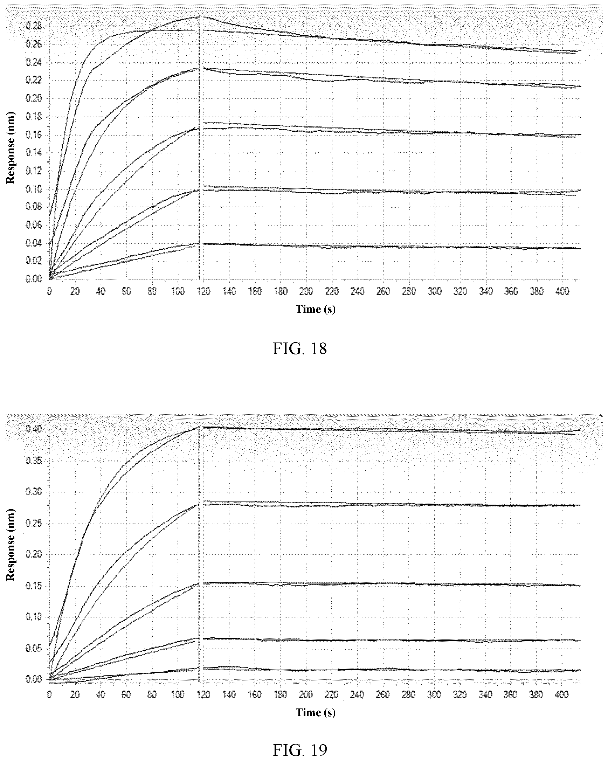

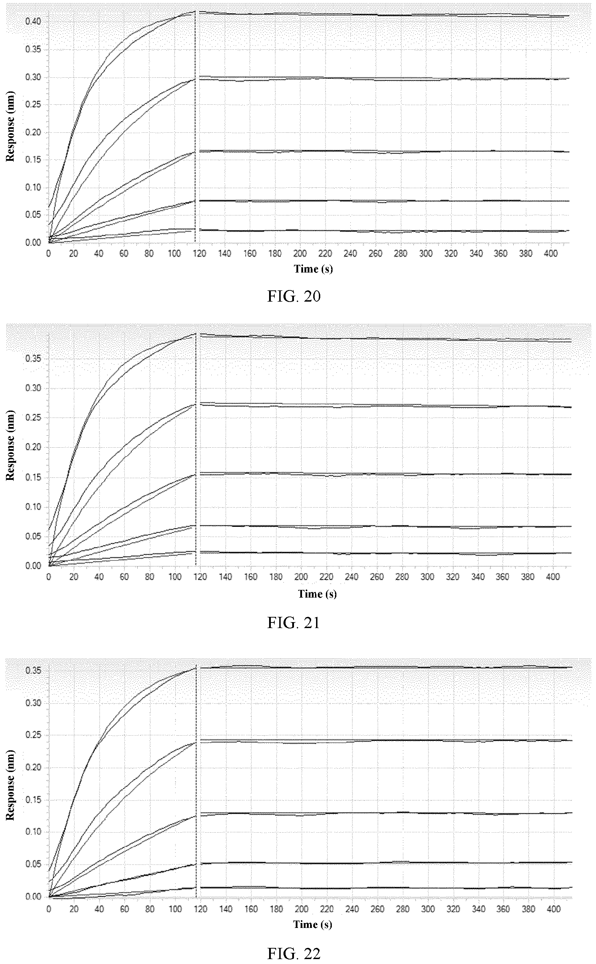

The determination results of the affinity constants of humanized antibodies NTPDV1, NTPDV2, NTPDV3, NTPDV4 and nivolumab (as control antibody) for human PD-1-mFc are shown in Table 13, and the detection results are shown in FIGs. 19, 20, 21, 22 and 18. The affinity constants of the humanized antibodies NTPDV1, NTPDV2, NTPDV3, NTPDV4 and nivolumab for human PD-1-mFc were 1.40E-10 M, 7.39E-11 M, 1.25E-10 M, 1.13E-11 M and 2.26E-10 M, respectively. The above experimental results showed that the binding ability of NTPDV1, NTPDV3 and nivolumab is comparable, and the binding ability of NTPDV2 and NTPDV4 is superior to that of nivolumab, suggesting that the humanized antibodies NTPDV1, NTPDV2, NTPDV3 and NTPDV4 have stronger binding ability to human PD-1-mFc.

**Table 13. Affinity constants of 14C12H1L1(hG1TM), NTPDV1, NTPDV2, NTPDV3, NTPDV4 and nivolumab for PD-1-mFc**

| **Test antibodies** | **KD (M)** | **Kon (1/Ms)** | **S E (kon)** | **Kdis (1/s)** | **S E (kdis)** | **Rmax (nm)** |
|---|---|---|---|---|---|---|
| 14C12H1L1(hG1TM) | 4.45E-11 | 1.18E+06 | 5.74E+04 | 5.23E-05 | 4.47E-05 | 0.38-0.57 |
| Nivolumab | 2.26E-10 | 1.52E+06 | 7.79E+04 | 3.43E-04 | 4.66E-05 | 0.25-0.37 |
| NTPDV1 | 1.40E-10 | 6.09E+05 | 2.01E+04 | 8.55E-05 | 3.18E-05 | 0.38-0.52 |
| NTPDV2 | 7.39E-11 | 6.83E+05 | 2.47E+04 | 5.05E-05 | 3.50E-05 | 0.40-0.55 |
| NTPDV3 | 1.25E-10 | 6.68E+05 | 2.52E+04 | 8.35E-05 | 3.44E-05 | 0.38-0.50 |
| NTPDV4 | 1.13E-11 | 5.35E+05 | 1.59E+04 | 6.03E-06 | 2.77E-05 | 0.35-0.47 |

| | | | | | | |
|---|---|---|---|---|---|---|
| K_{D} is the affinity constant; K_{D} = kdis/kon | | | | | | |

### Example 19: Kinetic parameters for binding of anti-CD73/anti-PD-1 bispecific antibodies to antigen human NT5E (1-552)-his determined by Fortebio system

The sample dilution buffer was PBS (0.02% Tween-20, 0.1% BSA, pH 7.4). HNT5E(1-552)-his was immobilized on the HIS1K sensor at a concentration of 5 µg/mL with an immobilization height of about 0.4 nM (time 50 s). The sensor was equilibrated in a buffer for 60 s, and the binding of the immobilized HNT5E(1-552)-his on the sensor to the antibodies at concentrations of 0.31-25 nM (three-fold dilution) was determined for 100 s. The protein was dissociated in the buffer for 180 s. The detection temperature was 30 °C, the detection frequency was 0.3 Hz, and the sample plate shaking rate was 1000 rpm. The data were analyzed by 1: 1 model fitting to obtain affinity constants.

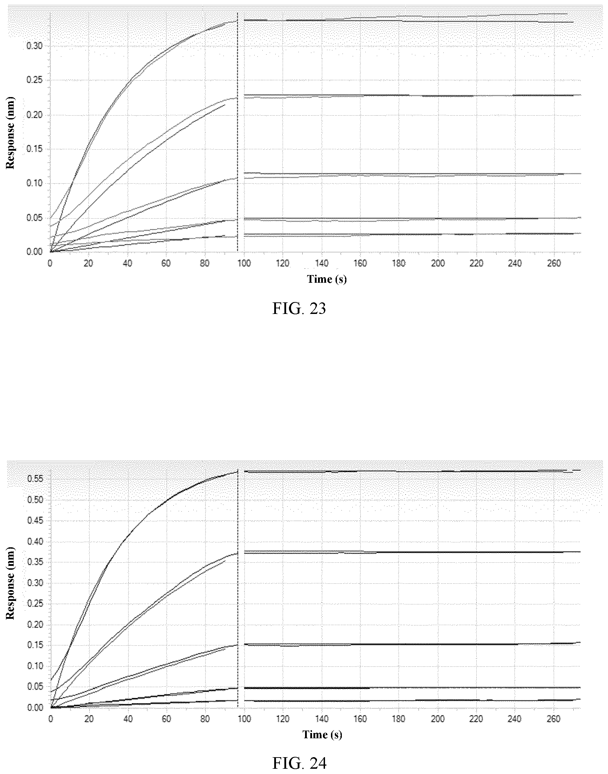

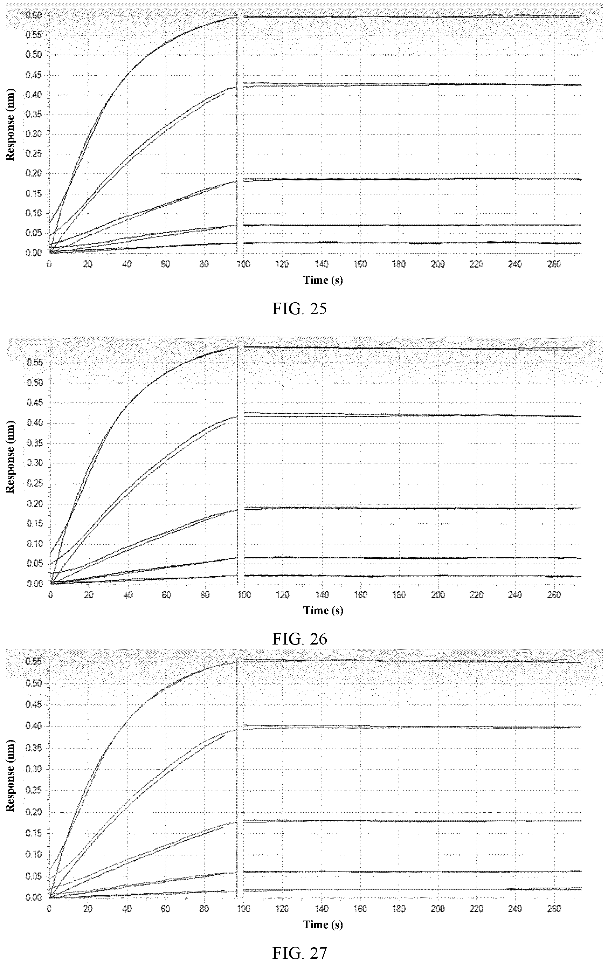

The determination results of the affinity constants of the humanized antibodies 19F3H2L3(hG1M) (as a control antibody), NTPDV1, NTPDV2, NTPDV3 and NTPDV4 for human NT5E(1-552)-his are shown in Table 14, and the detection results are shown in FIGs. 24-27. The affinity constants of the humanized antibodies NTPDV1, NTPDV2, NTPDV3 and NTPDV4 for human NT5E(1-552)-his were 3.29E-11 M, 2.88E-11 M, 7.92E-11 M and 5.77E-11 M, respectively.

The above experimental results showed that the binding ability of 19F3H2L3(hG1M), NTPDV1, NTPDV2, NTPDV3 and NTPDV4 is comparable, suggesting that the humanized antibodies NTPDV1, NTPDV2, NTPDV3 and NTPDV4 has stronger binding ability to human NTSE(1-552)-his.

**Table 14. Affinity constants of 19F3H2L3(hG1M), NTPDV1, NTPDV2, NTPDV3 and NTPDV4 for human NTSE(1-552)-his**

| **Test antibodies** | **KD (M)** | **kon (1/Ms)** | **S E (kon)** | **kdis (1/s)** | **S E (kdis)** | **Rmax (nm)** |
|---|---|---|---|---|---|---|
| **19F3H2L3 (HG1M)** | 4.05E-11 | 1.16E+06 | 6.50E+04 | 4.68E-05 | 8.96E-05 | 0.36-0.77 |
| **NTPDV1** | 3.29E-11 | 1.16E+06 | 3.27E+04 | 3.80E-05 | 4.63E-05 | 0.49-0.61 |
| **NTPDV2** | 2.88E-11 | 1.27E+06 | 3.84E+04 | 3.66E-05 | 4.88E-05 | 0.63-0.71 |
| **NTPDV3** | 7.92E-11 | 1.28E+06 | 4.08E+04 | 1.02E-04 | 5.29E-05 | 0.55-0.65 |
| **NTPDV4** | 5.77E-11 | 1.23E+06 | 3.82E+04 | 7.09E-05 | 4.95E-05 | 0.52-0.63 |

| | | | | | | |
|---|---|---|---|---|---|---|
| K_{D} is the affinity constant; K_{D} = kdis/kon | | | | | | |

### Example 20: Detection of inhibition of anti-CD73/anti-PD-1 bispecific antibody on enzyme activity of CD73 on U87-MG cell membrane surface

U87-MG cells in logarithmic phase in good condition were taken, resuspended in a serum-free RPMI-1640 culture solution, and then counted. The U87-MG cells were seeded into a 96-well plate at 2.5 × 10⁴ cells/60 µL/well. The antibody was diluted according to the study design with the serum-free RPMI-1640 culture solution. The antibody was added to the 96-well plate at 60 µL/well, and the plate was incubated at 37 °C for 1 h. After 1 h, 60 µL of 600 µM RPMI-1640-diluted AMP was added to each well. After 3 h, 100 µL of cell culture supernatant was taken and transferred to a new 96-well plate, 40 µL of CTG (CellTiterGlo) chromogenic solution was added to each well, and the plate was placed in the dark at room temperature for 5 min. After 5 min, 10 µL of 300 µM ATP was added to each well for chromogenesis. Relative fluorescence intensity RLU were read by a multi-label microplate tester (PerkinElmer 2140-0020).

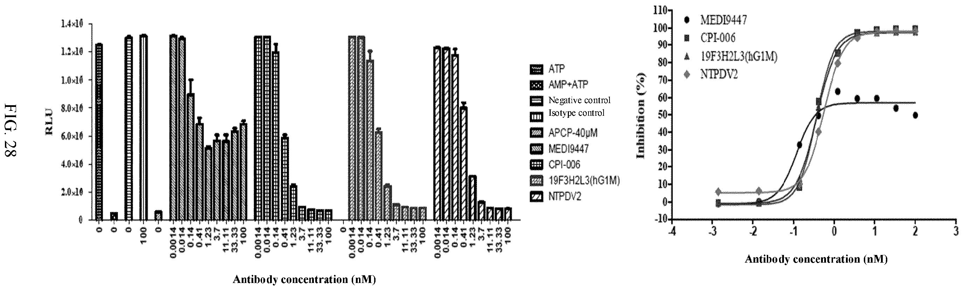

The experimental results are as shown in Table 15 and FIG. 28, the AMP amount of NTPDV2 is comparable to that of the positive control MEDI9447.

The above experimental results showed that the added AMP can be converted into adenosine A without antibodies by the enzyme activity of CD73 on the U87-MG cell surface, and that after the addition of the antibody, the enzyme-catalyzed function was decreased due to the binding of antibody NTPDV2 to CD73, so that the AMP was not converted into adenosine A. It was suggested that the antibodies effectively inhibit the enzyme activity reaction thereof in a non-substrate competition mode and reduce the production of adenosine.

**Table 15. Detection of inhibition of anti-CD73/anti-PD-1 bispecific antibody on enzyme activity of CD73 on U87-MG cell membrane surface**

| **Name of antibody** | **MEDI9447** | **19F3H2L3(hG1M)** | **NTPDV2** |
|---|---|---|---|
| **IC50 (nM)** | 0.1189 | 0.3647 | 0.5551 |

### Example 21: Biological activity of anti-CD73/anti-PD-1 bispecific antibodies for promoting IFN-γ and IL-2 secretion determined by mixed lymphocyte reaction (MLR)

### 1. Biological activity of anti-CD73/anti-PD-1 bispecific antibodies for promoting IFN-y secretion in Raji-PDL1 mixed lymphocyte reaction system

Raji-PDL1 cells were normally subcultured. PBMCs were thawed, incubated with 10 mL of a 1640 complete medium, and stimulated with 0.5 µg/mL SEB (Dianotech, Cat. No. S010201) for two days. Raji-PDL1 cells were treated with 25 µg/mL MMC (mitomycin C, Stressmarq, catalog: SIH-246, Cat. No. SM286474) and placed in a 37 °C incubator for 1 h. PBMCs (peripheral blood mononuclear cells) stimulated with SEB for 2 days and Raji-PDL1 cells treated with MMC for 1 h were collected, washed twice with PBS, resuspended in the complete medium, and counted. The cells were separately added to a U-shaped 96-well plate at 1 × 10⁵ cells/well. The antibodies were added according to the study design and cultured in an incubator for 3 days. After 3 days, the cell culture supernatant was collected and determined for IFN-y by ELISA.

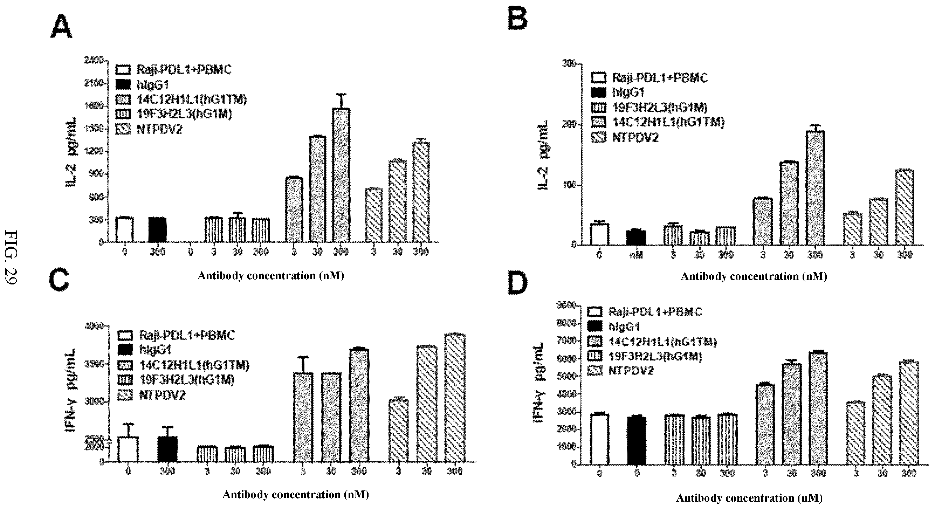

As shown in FIG. 29, the mixed culture of human PBMCs and Raji-PDL1 cells significantly promoted the secretion of IFN-y from PBMCs, and the addition of antibodies to the mixed culture system significantly induced secretion of IFN-y in PBMCs. In terms of the level of promoting IFN-y secretion activity, the antibody NTPDV2 has the activity comparable to that of the parent PD-1 single-target antibody 14C12H1L1.

### 2. Biological activity of anti-CD73/anti-PD-1 bispecific antibodies for promoting IL-2 secretion in Raji-PDL1 mixed lymphocyte reaction system

Raji-PDL1 cells were normally subcultured. PBMCs were thawed, incubated with 10 mL of a 1640 complete medium, and stimulated with SEB (0.5 µg/mL) for two days. Raji-PDL1 cells were treated with 25 µg/mL MMC and placed in a 37 °C incubator for 1 h. PBMCs (peripheral blood mononuclear cells) stimulated with SEB for 2 days and Raji-PDL1 cells treated with MMC for 1 h were collected, washed twice with PBS, resuspended in the complete medium, and counted. The cells were separately added to a U-shaped 96-well plate at 1 × 10⁵ cells/well. The antibodies were added according to the study design and cultured for 3 days. The cell culture supernatant was collected and determined for IL-2 by ELISA.

As shown in FIG. 29, the mixed culture of human PBMCs and Raji-PDL1 cells had certain promoting effect on the secretion of IL-2 from PBMCs, and the simultaneous addition of antibodies to the mixed culture system significantly induced IL-2 secretion in PBMCs in a significant dose-dependent manner. In terms of the level of promoting IL-2 secretion activity, the bifunctional antibody NTPDV2 has the activity at low a concentration comparable to that of the parent PD-1 single-target antibody 14C12H1L1, and the activity at a medium-high concentration slightly lower than that of the parent PD-1 single-target antibody 14C12H1L1.

### Example 22: Experiment of inhibition of tumor growth in vivo by anti-CD73/anti-PD-1 bispecific antibody

In order to determine the anti-tumor activity *in vivo* of the anti-CD73/anti-PD-1 bispecific antibody, firstly, MC38-hPDL1/hCD73 cells (from GemPharmatech Co., Ltd.) were subcutaneously inoculated into female C57BL6-hPD1hPDL1hCD73 triple-transgenic mice aged 5-7 weeks (from GemPharmatech Co., Ltd.). The modeling and specific administration methods are shown in Table 16. After the administration, the length and width of each group of tumors were measured, and the tumor volume was calculated.

**Table 16. Study design**

| Grouping | n | Tumor xenograft | Condition of administration |
|---|---|---|---|
| Isotype control | 8 | MC38-hPDL1/hCD73, 1 million cells/mouse, s.c. | Isotype control antibody hIgG, 10 mg/kg |
| | | | Twice weekly, for three weeks |
| 19F3H2L3 (hG1M) | 8 | | 19F3H2L3(hG1M)m 10 mg/kg |
| | | | Twice weekly, for three weeks |
| NTPDV2 | 8 | | NTPDV2, 13.3 mg/kg |
| | | | Twice weekly, for three weeks |
| NTPDV2 | 8 | | NTPDV2, 1.33 mg/kg |
| | | | Twice weekly, for three weeks |

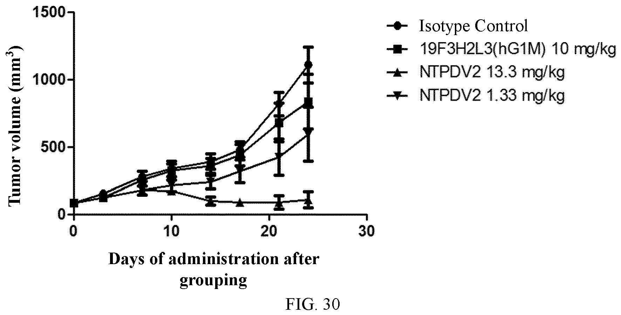

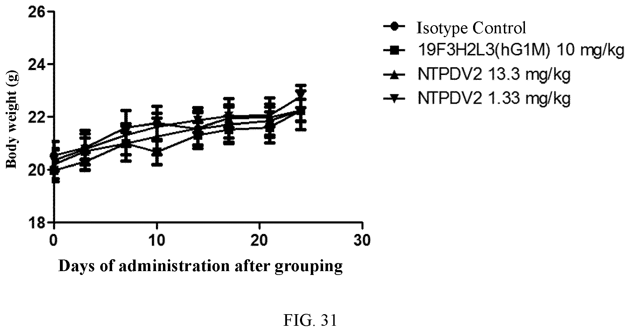

The experimental results are as shown in FIG. 30 and FIG. 31. The results showed that compared with an isotype control antibodies hIgG and 19F3H2L3(hG1M), NTPDV2 at different doses can effectively inhibit the growth of mouse tumors, and NTPDV2 at high dose is superior to NTPDV2 at low-dose in inhibiting tumors. In addition, NTPDV2 had no effect on the body weight of the tumor-bearing mouse.

### Experimental Example 23: Effective elimination of bispecific immune checkpoint inhibitor PD-1/CD73 bispecific antibody-mediated IL-8 and IL-6 secretions in human macrophages by the amino acid mutations of Fc segments

HPMMs were prepared by induction of PBMCs. PBMCs used in this study were isolated and prepared in Zhongshan Akesobio Co. Ltd., with informed consent of the donor. Ficoll-Paque PLUS lymphocyte isolation solution (GE, Cat. No. 17-1440-03); RPMI 1640 (Gibco, Cat. No. 22400-105); CHO-K1-PD1 cells (constructed by Zhongshan Akesobio Co. Ltd.); U87-MG cells (cells from ATCC, purchased from Beijing Zhongyuan Ltd.); FBS (Fetal Bovine Serum, Excell bio, Cat. No. FSP 500); human IFN-y protein (Sinobio, Cat. No. 11725-HNAS-100); LPS (lipopolysaccharide) (Sigma, Cat. No. L4391); a 96-well cell culture plate (Corning).

Healthy human PBMCs were isolated according to the instructions of insolation solution Ficoll-PaqueTM Plus reagent and resuspended in a 1640 medium containing 2% FBS. The plate was incubated in a 5% CO₂ cell incubator at 37 °C. After 2 h, the supernatant was removed, and the adherent cells were washed twice with PBS and 1640 complete medium (containing 10% FBS) and 100 ng/mL human M-CSF were added for 7 days of induction. On day 3 and day 5, the medium was exchanged, and M-CSF was added to induce HPMM. On day 7, after induction of HPMM, the cells were collected, adjusted to the concentration of 0.1 million cells/mL with the complete medium, and filled into a 96-well plate. The recombinant human IFN-y (50 ng/mL) was added, and incubated in an incubator for 24 h. After 24 h, CHO-K1-PD1 cells expressing human PD-1 or U87-MG cells constitutively expressing human CD73 in logarithmic phase were collected. The cells were resuspended and then adjusted to the concentration of 0.3 million cells/mL with the complete medium. The antibody was diluted with the complete medium to working concentrations of 25 nM, 2.5 nM and 0.25 nM. An isotype control antibody and a blank control were designed simultaneously. The supernatant in the 96-well plate was removed, the CHO-K1-PD1 or U87-MG cell suspension and antibodies were added (with a final volume of 200 µL). The system was mixed well and incubated in an incubator for 24 h. The mixture was centrifuged at 500× g for 5 min, the supernatant was collected, and the secretion amounts of IL-8 and IL-6 were determined with a Dakewe kit. LPS was used as a positive control and was adjusted to a concentration of 100 ng/mL with the complete medium in the experiment.

In this example, the co-culture of CHO-K1-PD1 and U87-MG cells as target cells with HPMM induced the activation of HPMM, and after the activated HPMM was linked to the target cells by antibody Fab, the Fc fragment of the antibody interacted with FcyR on HPMM, causing the secretion of cytokine by HPMM.

### 3. Experimental results

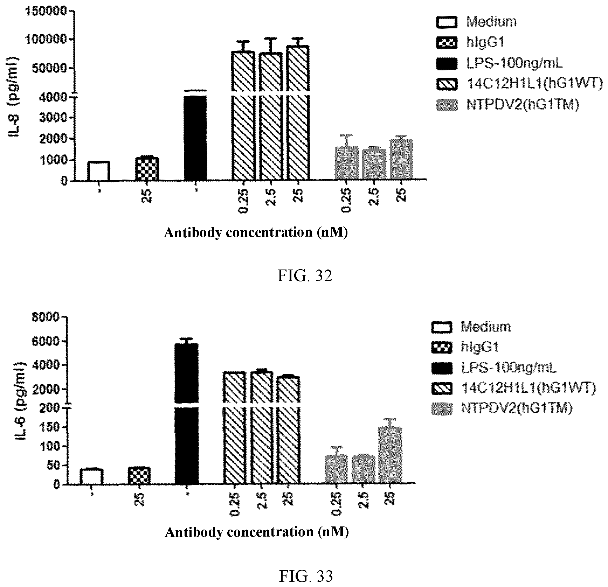

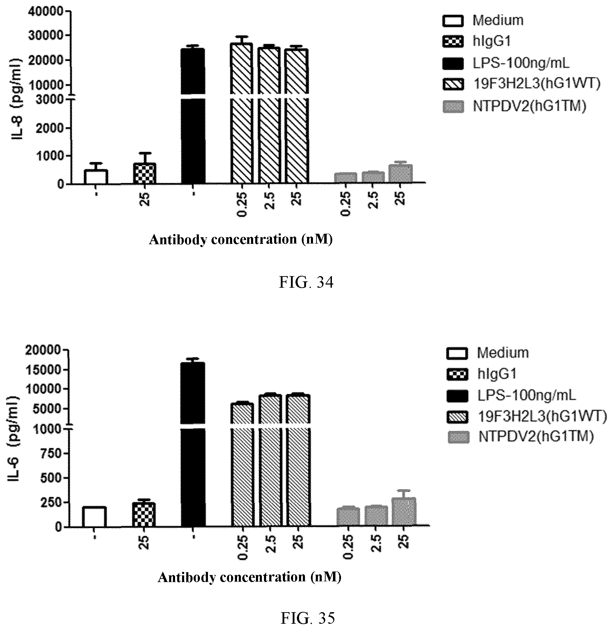

The results are shown in FIGs. 32-35.

The results showed that compared with the wild-type IgG1 subtype PD-1 antibody or CD73 antibody, the anti-PD-1/CD73 bispecific antibody carrying the Fc fragment mutation is able to effectively eliminate the secretion of IL-6 and/or IL-8 in immune cells.

The preferred embodiments of the present invention have been described above in detail, but the present invention is not limited to the embodiments. Those skilled in the art can make various equivalent modifications or replacements without violating the spirit of the present invention. These equivalent modifications or replacements are included in the scope defined by the claims of the present application.

### SEQUENCE LISTING

**Nucleotide sequence of 19F3 heavy chain variable region:** 
GAGGTGCAGCTGCAGCAGTCCGGACCAGAGCTGGTGAAGCCTGGCGCCTCCATGCGGAT
GTCTTGTAAGGCCTCTGGCTACAGCTTCACCGGCTATACAATGAACTGGGTGAAGCAGT
CTCACGGCAAGAATCTGGAGTGGATCGGCCTGATCAACCCTTACAATGCCGGCACCAG
CTATAACCAGAAGTTTAAGGGCAAGGCCACCCTGACAGTGGACAAGAGCTCCTCTACCGC
CTACATGGAGCTGCTGTCCCTGACATCTGAGGATAGCGCCGTGTACTATTGCGCCCGGTC
CGAGTACAGATATGGCGGCGACTACTTTGATTATTGGGGCCAGGGCACCACACTGACA
GTGAGCTCC
(SEQ ID NO: 1, with CDR sequences underlined)
**Amino acid sequence of 19F3 heavy chain variable region:** 
EVQLQQSGPELVKPGASMRMSCKASGYSFTGYTMNWVKQSHGKNLEWIGLINPYNAGTSY
NQKFKGKATLTVDKSSSTAYMELLSLTSEDSAVYYCARSEYRYGGDYFDYWGQGTTLTVSS
(SEQ ID NO: 2, with CDR sequences underlined)
HCDR1 of **19F3**: GYSFTGYT
   (SEQ ID NO: 3)
HCDR2 of **19F3**: INPYNAGT
   (SEQ ID NO: 4)
HCDR3 of 19F3: ARSEYRYGGDYFDY
   (SEQ ID NO: 5)
**Nucleotide sequence of 19F3 light chain variable region:** 
GACATCGTGATGACCCAGTCTCCAAGCTCCCTGGCAATGAGCGTGGGACAGAAGGTGAC
AATGTCTTGTAAGTCTAGCCAGAGCCTGCTGAACTCCTCTAATCAGAAGAACTACCTG
GCCTGGTATCAGCAGAAGCCAGGCCAGTCTCCCAAGCTGCTGGTGTACTTTGCCAGCAC
CAGGGAGTCCGGAGTGCCTGACAGATTCATCGGCTCCGGCTCTGGCACAGACTTCACCCT
GACAATCAGCTCCGTGCAGGCAGAGGACCTGGCAGATTATTTCTGCCAGCAGCACTACG
ACACCCCTTATACATTTGGCGGCGGCACCAAGCTGGAGATCAAG
(SEQ ID NO: 6, with CDR sequences underlined)
**Amino acid sequence of 19F3 light chain variable region:** 
DIVMTQSPSSLAMSVGQKVTMSCKSSQSLLNSSNQKNYLAWYQQKPGQSPKLLVYFASTRE
SGVPDRFIGSGSGTDFTLTISSVQAEDLADYFCQQHYDTPYTFGGGTKLEIK
(SEQ ID NO: 7, with CDR sequences underlined)
LCDR1 amino acid sequence of **19F3**: QSLLNSSNQKNY
   (SEQ ID NO: 8)
LCDR2 amino acid sequence of **19F3**: FAS
   (SEQ ID NO: 9)
LCDR3 amino acid sequence of **19F3**: QQHYDTPYT
   (SEQ ID NO: 10)
**Amino acid sequences of 19F3 heavy chain framework regions:**
   FR-H1: EVQLQQSGPELVKPGASMRMSCKAS (SEQ ID NO: 11)
   FR-H2: MNWVKQSHGKNLEWIGL (SEQ ID NO: 12)
   FR-H3: SYNQKFKGKATLTVDKSSSTAYMELLSLTSEDSAVYYC (SEQ ID NO: 13)
   FR-H4: WGQGTTLTVSS (SEQ ID NO: 14)
**Amino acid sequences of 19F3 light chain framework regions:**
   FR-L1: DIVMTQSPSSLAMSVGQKVTMSCKSS (SEQ ID NO: 15)
   FR-L2: LAWYQQKPGQSPKLLVY (SEQ ID NO: 16)
   FR-L3: TRESGVPDRFIGSGSGTDFTLTISSVQAEDLADYFC (SEQ ID NO: 17)
   FR-L4: FGGGTKLEIK (SEQ ID NO: 18)
**Nucleotide sequence of 19F3H2:** (SEQ ID NO: 19, with CDR sequences underlined) 
CAGGTGCAGCTGGTGCAGTCTGGAGCAGAGGTGGTGAAGCCAGGAGCCTCTGTGAAGGT
GAGCTGTAAGGCCAGCGGCTACTCCTTCACCGGCTATACAATGAACTGGGTGAGGCAG
GCACCAGGACAGAATCTGGAGTGGATCGGCCTGATCAACCCTTACAATGCCGGCACCT
CTTATAACCAGAAGTTTCAGGGCAAGGTGACCCTGACAGTGGACAAGTCCACCTCTACAG
CCTACATGGAGCTGAGCTCCCTGCGGAGCGAGGATACAGCCGTGTACTATTGCGCCCGG
TCCGAGTACAGATATGGCGGCGACTACTTTGATTATTGGGGCCAGGGCACCACACTGA
CCGTGTCTAGC
**Amino acid sequence of 19F3H2:** (SEQ ID NO: 20, with CDR sequences underlined) 
QVQLVQSGAEVVKPGASVKVSCKASGYSFTGYTMNWVRQAPGQNLEWIGLINPYNAGTSY
NQKFQGKVTLTVDKSTSTAYMELSSLRSEDTAVYYCARSEYRYGGDYFDYWGQGTTLTVSS
**Nucleotide sequence of 19F3L2:** (SEQ ID NO: 21, with CDR sequences underlined) 
GACATCGTGATGACCCAGTCCCCAAGCTCCCTGGCCGTGTCTGTGGGAGAGCGGGTGACA
ATCTCCTGTAAGTCTAGCCAGTCTCTGCTGAACTCCTCTAATCAGAAGAACTACCTGGC
CTGGTATCAGCAGAAGCCCGGCCAGGCCCCTAAGCTGCTGATCTACTTCGCCTCTACCAG
GGAGAGCGGAGTGCCAGACAGATTCTCTGGCAGCGGCTCCGGCACAGACTTCACCCTGAC
AATCAGCTCCGTGCAGGCAGAGGACGTGGCAGATTACTATTGCCAGCAGCACTACGATA
CCCCCTATACATTTGGCGGCGGCACCAAGCTGGAGATCAAG
**Amino acid sequence of 19F3L2:** (SEQ ID NO: 22, with CDR sequences underlined) 
DIVMTQSPSSLAVSVGERVTISCKSSQSLLNSSNQKNYLAWYQQKPGQAPKLLIYFASTRESG
VPDRFSGSGSGTDFTLTISSVQAEDVADYYCQQHYDTPYTFGGGTKLEIK
**Nucleotide sequence of 19F3L3:** (SEQ ID NO: 23, with CDR sequences underlined) 
GACATCGTGATGACCCAGTCCCCAAGCTCCCTGGCCGTGTCTGTGGGAGAGCGGGTGACA
ATCTCCTGTAAGTCTAGCCAGTCTCTGCTGAACTCCTCTAATCAGAAGAACTACCTGGC
CTGGTATCAGCAGAAGCCCGGCCAGGCCCCTAAGCTGCTGATCTACTTCGCCTCTACCAG
GGAGAGCGGAGTGCCAGACAGATTCTCTGGCAGCGGCTCCGGCACAGACTTCACCCTGAC
AATCAGCTCCCTGCAGGCAGAGGACGTGGCCGTGTACTATTGCCAGCAGCACTACGATA
CCCCCTATACATTTGGCGGCGGCACCAAGCTGGAGATCAAG
**Amino acid sequence of 19F3L3:** (SEQ ID NO: 24, with CDR sequences underlined) 
DIVMTQSPSSLAVSVGERVTISCKSSQSLLNSSNQKNYLAWYQQKPGQAPKLLIYFASTRESG
VPDRFSGSGSGTDFTLTISSLQAEDVAVYYCQQHYDTPYTFGGGTKLEIK
Nucleotide sequence of **19F3H2L3(G1M)** heavy chain (SEQ ID NO: 25, with non-variable region sequences underlined) 
CAGGTGCAGCTGGTGCAGTCTGGAGCAGAGGTGGTGAAGCCAGGAGCCTCTGTGAAGGT
GAGCTGTAAGGCCAGCGGCTACTCCTTCACCGGCTATACAATGAACTGGGTGAGGCAGGC
ACCAGGACAGAATCTGGAGTGGATCGGCCTGATCAACCCTTACAATGCCGGCACCTCTTA
TAACCAGAAGTTTCAGGGCAAGGTGACCCTGACAGTGGACAAGTCCACCTCTACAGCCTA
CATGGAGCTGAGCTCCCTGCGGAGCGAGGATACAGCCGTGTACTATTGCGCCCGGTCCGA
GTACAGATATGGCGGCGACTACTTTGATTATTGGGGCCAGGGCACCACACTGACCGTGTC
TAGCGCCTCCACAAAG ggG ccC agc gtg ttt cct ctc gcc ccc tcc tcc aaa a
gcaccagcggaggaaccgctgctctcggatgtctggtgaaggactacttccctgaacccgtcaccgtg
agctggaatagcggcgctctgacaagcggagtccatacattccctgctgtgctgcaaagcagcggact
ctattccctgtccagcgtcgtcacagtgcccagcagcagcctgggcacccagacctacatctgtaacg
tcaaccacaagccctccaacaccaaggtggacaagaaagtggagcccaaatcctgcgacaagacacac
 
acctgtcccccctgtcctgctcccgaaGCTGCTggaggccctagcgtcttcctctttcctcccaaac
ccaaggacaccctcatgatcagcagaacccctgaagtcacctgtgtcgtcgtggatgtcagccatgag
gaccccgaggtgaaattcaactggtatgtcgatggcgtcgaggtgcacaacgccaaaaccaagcccag
ggaggaacagtacaactccacctacagggtggtgtccgtgctgacagtcctccaccaggactggctga
acggcaaggagtacaagtgcaaggtgtccaacaaggctctccctgcccccattgagaagaccatcagc
aaggccaaaggccaacccagggagccccaggtctatacactgcctccctccagggacgaactcaccaa
gaaccaggtgtccctgacctgcctggtcaagggcttttatcccagcgacatcgccgtcgagtgggagt
ccaacggacagcccgagaataactacaagaccacccctcctgtcctcgactccgacggctccttcttc
ctgtacagcaagctgaccgtggacaaaagcaggtggcagcagggaaacgtgttctcctgcagcgtgat
gcacgaagccctccacaaccactacacccagaaaagcctgtccctgagccccggcaaatga
Amino acid sequence of **19F3H2L3(G1M)** heavy chain (SEQ ID NO: 26, with non-variable region sequences underlined) 
QVQLVQSGAEVVKPGASVKVSCKASGYSFTGYTMNWVRQAPGQNLEWIGLINPYNAGTSYNQKFQGKV
TLTVDKSTSTAYMELSSLRSEDTAVYYCARSEYRYGGDYFDYWGQGTTLTVSSASTKGPSVFPLAPSS
KSTSGGTAALGCLVKDYFPEPVTVSWNSGALTSGVHTFPAVLQSSGLYSLSSVVTVPSSSLGTQTYIC
NVNHKPSNTKVDKKVEPKSCDKTHTCPPCPAPEAAGGPSVFLFPPKPKDTLMISRTPEVTCVVVDVSH
EDPEVKFNWYVDGVEVHNAKTKPREEQYNSTYRVVSVLTVLHQDWLNGKEYKCKVSNKALPAPIEKTI
SKAKGQPREPQVYTLPPSRDELTKNQVSLTCLVKGFYPSDIAVEWESNGQPENNYKTTPPVLDSDGSF
FLYSKLTVDKSRWQQGNVFSCSVMHEALHNHYTQKSLSLSPGK
Nucleotide sequence of **19F3H2L3(G1M)** light chain (SEQ ID NO: 27, with non-variable region sequences underlined) 
GACATCGTGATGACCCAGTCCCCAAGCTCCCTGGCCGTGTCTGTGGGAGAGCGGGTGACA
ATCTCCTGTAAGTCTAGCCAGTCTCTGCTGAACTCCTCTAATCAGAAGAACTACCTGGCCT
GGTATCAGCAGAAGCCCGGCCAGGCCCCTAAGCTGCTGATCTACTTCGCCTCTACCAGGG
AGAGCGGAGTGCCAGACAGATTCTCTGGCAGCGGCTCCGGCACAGACTTCACCCTGACAA
TCAGCTCCCTGCAGGCAGAGGACGTGGCCGTGTACTATTGCCAGCAGCACTACGATACCC
CCTATACATTTGGCGGCGGCACCAAGCTGGAGATCAAGCGTACGGTGGCAGCCCCATCTGTCT
TCATTTTTCCCCCTAGTGACGAGCAGCTGAAATCCGGAACAGCCTCTGTGGTCTGTCTGCTGAACAAT
TTCTACCCTCGCGAAGCCAAGGTGCAGTGGAAAGTCGATAACGCTCTGCAGAGTGGCAATTCACAGGA
GAGCGTGACTGAACAGGACTCCAAGGATTCTACCTATAGTCTGAGCTCCACTCTGACCCTGTCCAAAG
CAGATTACGAAAAGCACAAAGTGTATGCCTGTGAGGTCACCCACCAGGGGCTGAGTTCTCCAGTCACC
AAATCCTTCAACAGAGGCGAATGT
Amino acid sequence of **19F3H2L3(G1M)** light chain (SEQ ID NO: 28, with non-variable region sequences underlined) 
DIVMTQSPSSLAVSVGERVTISCKSSQSLLNSSNQKNYLAWYQQKPGQAPKLLIYFASTRESGVPDRF
SGSGSGTDFTLTISSLQAEDVAVYYCQQHYDTPYTFGGGTKLEIKRTVAAPSVFIFPPSDEQLKSGTA
SVVCLLNNFYPREAKVQWKVDNALQSGNSQESVTEQDSKDSTYSLSSTLTLSKADYEKHKVYACEVTH
QGLSSPVTKSFNRGEC
Nucleotide sequence of **19F3H2L3(hG1TM)** heavy chain (SEQ ID NO: 29, with non-variable region sequences underlined) 
CAGGTGCAGCTGGTGCAGTCTGGAGCAGAGGTGGTGAAGCCAGGAGCCTCTGTGAAGGT
GAGCTGTAAGGCCAGCGGCTACTCCTTCACCGGCTATACAATGAACTGGGTGAGGCAGGC
ACCAGGACAGAATCTGGAGTGGATCGGCCTGATCAACCCTTACAATGCCGGCACCTCTTA
TAACCAGAAGTTTCAGGGCAAGGTGACCCTGACAGTGGACAAGTCCACCTCTACAGCCTA
CATGGAGCTGAGCTCCCTGCGGAGCGAGGATACAGCCGTGTACTATTGCGCCCGGTCCGA
GTACAGATATGGCGGCGACTACTTTGATTATTGGGGCCAGGGCACCACACTGACCGTGTC
TAGCGCCTCCACAAAG ggG ccC agc gtg ttt cct ctc gcc ccc tcc tcc aaa a
gcaccagcggaggaaccgctgctctcggatgtctggtgaaggactacttccctgaacccgtcaccgtg
agctggaatagcggcgctctgacaagcggagtccatacattccctgctgtgctgcaaagcagcggact
ctattccctgtccagcgtcgtcacagtgcccagcagcagcctgggcacccagacctacatctgtaacg
tcaaccacaagccctccaacaccaaggtggacaagaaagtggagcccaaatcctgcgacaagacacac
 
acctgtcccccctgtcctgctcccgaaGCTGCTggaGCCcctagcgtcttcctctttcctcccaaac
ccaaggacaccctcatgatcagcagaacccctgaagtcacctgtgtcgtcgtggatgtcagccatgag
gaccccgaggtgaaattcaactggtatgtcgatggcgtcgaggtgcacaacgccaaaaccaagcccag
ggaggaacagtacaactccacctacagggtggtgtccgtgctgacagtcctccaccaggactggctga
acggcaaggagtacaagtgcaaggtgtccaacaaggctctccctgcccccattgagaagaccatcagc
aaggccaaaggccaacccagggagccccaggtctatacactgcctccctccagggacgaactcaccaa
gaaccaggtgtccctgacctgcctggtcaagggcttttatcccagcgacatcgccgtcgagtgggagt
ccaacggacagcccgagaataactacaagaccacccctcctgtcctcgactccgacggctccttcttc
ctgtacagcaagctgaccgtggacaaaagcaggtggcagcagggaaacgtgttctcctgcagcgtgat
gcacgaagccctccacaaccactacacccagaaaagcctgtccctgagccccggcaaatga
Amino acid sequence of 19F3H2L3(hG1TM) heavy chain variable region: (SEQ ID NO: 30) 
QVQLVQSGAEVVKPGASVKVSCKASGYSFTGYTMNWVRQAPGQNLEWIGLINPYNAGTSYN
QKFQGKVTLTVDKSTSTAYMELSSLRSEDTAVYYCARSEYRYGGDYFDYWGQGTTLTVSSA
STKGPSVFPLAPSSKSTSGGTAALGCLVKDYFPEPVTVSWNSGALTSGVHTFPAVLQSSGLYS
LSSVVTVPSSSLGTQTYICNVNHKPSNTKVDKKVEPKSCDKTHTCPPCPAPEAAGAPSVFLFPP
KPKDTLMISRTPEVTCVVVDVSHEDPEVKFNWYVDGVEVHNAKTKPREEQYNSTYRVVSVL
TVLHQDWLNGKEYKCKVSNKALPAPIEKTISKAKGQPREPQVYTLPPSRDELTKNQVSLTCLV
KGFYPSDIAVEWESNGQPENNYKTTPPVLDSDGSFFLYSKLTVDKSRWQQGNVFSCSVMHEA
LHNHYTQKSLSLSPGK
**Amino acid sequences of 19F3H2 framework regions:**
   FR-H1: QVQLVQSGAEVVKPGASVKVSCKAS (SEQ ID NO: 31)
   FR-H2: MNWVRQAPGQNLEWIGL (SEQ ID NO: 32)
   FR-H3: SYNQKFQGKVTLTVDKSTSTAYMELSSLRSEDTAVYYC (SEQ ID NO: 33)
   FR-H4: QVQLVQSGAEVVKPGASVKVSCKAS (SEQ ID NO: 34)
**Amino acid sequences of 19F3L2 framework regions:**
   FR-L1: DIVMTQSPSSLAVSVGERVTISCKSS (SEQ ID NO: 35)
   FR-L2: LAWYQQKPGQAPKLLIY (SEQ ID NO: 36)
   FR-L3: TRESGVPDRFSGSGSGTDFTLTISSVQAEDVADYYC (SEQ ID NO: 37)
   FR-L4: FGGGTKLEIK (SEQ ID NO: 38)
**Amino acid sequences of 19F3L3 framework regions:**
   FR-L1: DIVMTQSPSSLAVSVGERVTISCKSS (SEQ ID NO: 39)
   FR-L2: LAWYQQKPGQAPKLLIY (SEQ ID NO: 40)
   FR-L3: TRESGVPDRFSGSGSGTDFTLTISSLQAEDVAVYYC (SEQ ID NO: 41)
   FR-L4: FGGGTKLEIK (SEQ ID NO: 42)
**Nucleotide sequence of 14C12 heavy chain variable region:** (SEQ ID NO: 43) 
GAGGTCAAACTGGTGGAGAGCGGCGGCGGGCTGGTGAAGCCCGGCGGGTCACTGAAACT
GAGCTGCGCCGCTTCCGGCTTCGCCTTTAGCTCCTACGACATGTCATGGGTGAGGCAGAC
CCCTGAGAAGCGCCTGGAATGGGTCGCTACTATCAGCGGAGGCGGGCGATACACCTACTA
TCCTGACTCTGTCAAAGGGAGATTCACAATTAGTCGGGATAACGCCAGAAATACTCTGTA
TCTGCAGATGTCTAGTCTGCGGTCCGAGGATACAGCTCTGTACTATTGTGCAAACCGGTA
CGGCGAAGCATGGTTTGCCTATTGGGGACAGGGCACCCTGGTGACAGTCTCTGCC
**Amino acid sequence of 14C12 heavy chain variable region:** (SEQ ID NO: 44) 
EVKLVESGGGLVKPGGSLKLSCAASGFAFSSYDMSWVRQTPEKRLEWVATISGGGRYTYYPD
SVKGRFTISRDNARNTLYLQMSSLRSEDTALYYCANRYGEAWFAYWGQGTLVTVSA
HCDR1 amino acid sequence of **14C12:** GFAFSSYD
   (SEQ ID NO: 45)
HCDR2 amino acid sequence of **14C12:** ISGGGRYT
   (SEQ ID NO: 46)
HCDR3 amino acid sequence of **14C12:** ANRYGEAWFAY
   (SEQ ID NO: 47)
**Nucleotide sequence of 14C12 light chain variable region:** (SEQ ID NO: 48) 
GACATTAAGATGACACAGTCCCCTTCCTCAATGTACGCTAGCCTGGGCGAGCGAGTGACC
TTCACATGCAAAGCATCCCAGGACATCAACACATACCTGTCTTGGTTTCAGCAGAAGCCA
GGCAAAAGCCCCAAGACCCTGATCTACCGGGCCAATAGACTGGTGGACGGGGTCCCCAG
CAGATTCTCCGGATCTGGCAGTGGGCAGGATTACTCCCTGACCATCAGCTCCCTGGAGTA
TGAAGACATGGGCATCTACTATTGCCTGCAGTATGATGAGTTCCCTCTGACCTTTGGAGCA
GGCACAAAACTGGAACTGAAG
**Amino acid sequence of 14C12 light chain variable region:** (SEQ ID NO: 49) 
DIKMTQSPSSMYASLGERVTFTCKASQDINTYLSWFQQKPGKSPKTLIYRANRLVDGVPSRFS
GSGSGQDYSLTISSLEYEDMGIYYCLQYDEFPLTFGAGTKLELK
LCDR1 **amino acid sequence** of **14C12**: QDINTY (SEQ ID NO: 50)
LCDR2 **amino acid sequence** of **14C12:** RAN (SEQ ID NO: 51)
LCDR3 **amino acid sequence** of **14C12**: LQYDEFPLT (SEQ ID NO: 52)
**Amino acid sequences of 14C12 heavy chain framework regions:**
   FR-H1: EVKLVESGGGLVKPGGSLKLSCAAS (SEQ ID NO: 53)
   FR-H2: MSWVRQTPEKRLEWVAT (SEQ ID NO: 54)
   FR-H3: YYPDSVKGRFTISRDNARNTLYLQMSSLRSEDTALYYC (SEQ ID NO: 55)
   FR-H4: WGQGTLVTVSA (SEQ ID NO: 56)
**Amino acid sequences of 14C12 light chain framework regions:**
   HR-L1: DIKMTQSPSSMYASLGERVTFTCKAS (SEQ ID NO: 57)
   HR-L2: LSWFQQKPGKSPKTLIY (SEQ ID NO: 58)
   HR-L3: RLVDGVPSRFSGSGSGQDYSLTISSLEYEDMGIYYC (SEQ ID NO: 59)
   HR-L4: FGAGTKLELK (SEQ ID NO: 60)
**Nucleotide sequence of 14C12H1:** (SEQ ID NO: 61) 
GAAGTGCAGCTGGTCGAGTCTGGGGGAGGGCTGGTGCAGCCCGGCGGGTCACTGCGACT
GAGCTGCGCAGCTTCCGGATTCGCCTTTAGCTCCTACGACATGTCCTGGGTGCGACAGGC
ACCAGGAAAGGGACTGGATTGGGTCGCTACTATCTCAGGAGGCGGGAGATACACCTACT
ATCCTGACAGCGTCAAGGGCCGGTTCACAATCTCTAGAGATAACAGTAAGAACAATCTGT
ATCTGCAGATGAACAGCCTGAGGGCTGAGGACACCGCACTGTACTATTGTGCCAACCGCT
ACGGGGAAGCATGGTTTGCCTATTGGGGGCAGGGAACCCTGGTGACAGTCTCTAGT
**Amino acid sequence of 14C12H1:** (SEQ ID NO: 62) 
EVQLVESGGGLVQPGGSLRLSCAASGFAFSSYDMSWVRQAPGKGLDWVATISGGGRYTYYP
DSVKGRFTISRDNSKNNLYLQMNSLRAEDTALYYCANRYGEAWFAYWGQGTLVTVSS
**Nucleotide sequence of 14C12L1:** (SEQ ID NO: 63) 
GACATTCAGATGACTCAGAGCCCCTCCTCCATGTCCGCCTCTGTGGGCGACAGGGTCACC
TTCACATGCCGCGCTAGTCAGGATATCAACACCTACCTGAGCTGGTTTCAGCAGAAGCCA
GGGAAAAGCCCCAAGACACTGATCTACCGGGCTAATAGACTGGTGTCTGGAGTCCCAAGT
CGGTTCAGTGGCTCAGGGAGCGGACAGGACTACACTCTGACCATCAGCTCCCTGCAGCCT
GAGGACATGGCAACCTACTATTGCCTGCAGTATGATGAGTTCCCACTGACCTTTGGCGCC
GGGACAAAACTGGAGCTGAAG
**Amino acid sequence of 14C12L1:** (SEQ ID NO: 64) 
DIQMTQSPSSMSASVGDRVTFTCRASQDINTYLSWFQQKPGKSPKTLIYRANRLVSGVPSRFS
GSGSGQDYTLTISSLQPEDMATYYCLQYDEFPLTFGAGTKLELK
**Nucleotide sequence of 14C12H1L1 heavy chain** (SEQ ID NO: 65) 
GAAGTGCAGCTGGTCGAGTCTGGGGGAGGGCTGGTGCAGCCCGGCGGGTCACTGCGACT
GAGCTGCGCAGCTTCCGGATTCGCCTTTAGCTCCTACGACATGTCCTGGGTGCGACAGGC
ACCAGGAAAGGGACTGGATTGGGTCGCTACTATCTCAGGAGGCGGGAGATACACCTACT
ATCCTGACAGCGTCAAGGGCCGGTTCACAATCTCTAGAGATAACAGTAAGAACAATCTGT
ATCTGCAGATGAACAGCCTGAGGGCTGAGGACACCGCACTGTACTATTGTGCCAACCGCT
ACGGGGAAGCATGGTTTGCCTATTGGGGGCAGGGAACCCTGGTGACAGTCTCTAGTGCCA
GCACCAAAGGACCTAGCGTGTTTCCTCTCGCCCCCTCCTCCAAAAGCACCAGCGGAGGAA
CCGCTGCTCTCGGATGTCTGGTGAAGGACTACTTCCCTGAACCCGTCACCGTGAGCTGGA
ATAGCGGCGCTCTGACAAGCGGAGTCCATACATTCCCTGCTGTGCTGCAAAGCAGCGGAC
TCTATTCCCTGTCCAGCGTCGTCACAGTGCCCAGCAGCAGCCTGGGCACCCAGACCTACA
TCTGTAACGTCAACCACAAGCCCTCCAACACCAAGGTGGACAAGAAAGTGGAGCCCAAA
TCCTGCGACAAGACACACACCTGTCCCCCCTGTCCTGCTCCCGAACTCCTCGGAGGCCCTA
GCGTCTTCCTCTTTCCTCCCAAACCCAAGGACACCCTCATGATCAGCAGAACCCCTGAAGT
CACCTGTGTCGTCGTGGATGTCAGCCATGAGGACCCCGAGGTGAAATTCAACTGGTATGT
CGATGGCGTCGAGGTGCACAACGCCAAAACCAAGCCCAGGGAGGAACAGTACAACTCCA
CCTACAGGGTGGTGTCCGTGCTGACAGTCCTCCACCAGGACTGGCTGAACGGCAAGGAGT
ACAAGTGCAAGGTGTCCAACAAGGCTCTCCCTGCCCCCATTGAGAAGACCATCAGCAAGG
CCAAAGGCCAACCCAGGGAGCCCCAGGTCTATACACTGCCTCCCTCCAGGGACGAACTCA
CCAAGAACCAGGTGTCCCTGACCTGCCTGGTCAAGGGCTTTTATCCCAGCGACATCGCCG
TCGAGTGGGAGTCCAACGGACAGCCCGAGAATAACTACAAGACCACCCCTCCTGTCCTCG
ACTCCGACGGCTCCTTCTTCCTGTACAGCAAGCTGACCGTGGACAAAAGCAGGTGGCAGC
AGGGAAACGTGTTCTCCTGCAGCGTGATGCACGAAGCCCTCCACAACCACTACACCCAGA
AAAGCCTGTCCCTGAGCCCCGGCAAA
**Amino acid sequence of 14C12H1L1 heavy chain variable region: (SEQ ID NO: 66)** 
EVQLVESGGGLVQPGGSLRLSCAASGFAFSSYDMSWVRQAPGKGLDWVATISGGGRYTYYP
DSVKGRFTISRDNSKNNLYLQMNSLRAEDTALYYCANRYGEAWFAYWGQGTLVTVSSASTK
GPSVFPLAPSSKSTSGGTAALGCLVKDYFPEPVTVSWNSGALTSGVHTFPAVLQSSGLYSLSSV
VTVPSSSLGTQTYICNVNHKPSNTKVDKKVEPKSCDKTHTCPPCPAPELLGGPSVFLFPPKPKD
TLMISRTPEVTCVVVDVSHEDPEVKFNWYVDGVEVHNAKTKPREEQYNSTYRVVSVLTVLH
QDWLNGKEYKCKVSNKALPAPIEKTISKAKGQPREPQVYTLPPSRDELTKNQVSLTCLVKGF
YPSDIAVEWESNGQPENNYKTTPPVLDSDGSFFLYSKLTVDKSRWQQGNVFSCSVMHEALHN
HYTQKSLSLSPGK
**Nucleotide sequence of 14C12H1L1 light chain (SEQ ID NO: 67)** 
GACATTCAGATGACTCAGAGCCCCTCCTCCATGTCCGCCTCTGTGGGCGACAGGGTCACC
TTCACATGCCGCGCTAGTCAGGATATCAACACCTACCTGAGCTGGTTTCAGCAGAAGCCA
GGGAAAAGCCCCAAGACACTGATCTACCGGGCTAATAGACTGGTGTCTGGAGTCCCAAGT
CGGTTCAGTGGCTCAGGGAGCGGACAGGACTACACTCTGACCATCAGCTCCCTGCAGCCT
GAGGACATGGCAACCTACTATTGCCTGCAGTATGATGAGTTCCCACTGACCTTTGGCGCC
GGGACAAAACTGGAGCTGAAGCGAACTGTGGCCGCTCCCTCCGTCTTCATTTTTCCCCCTT
CTGACGAACAGCTGAAATCAGGCACAGCCAGCGTGGTCTGTCTGCTGAACAATTTCTACC
CTAGAGAGGCAAAAGTGCAGTGGAAGGTCGATAACGCCCTGCAGTCCGGCAACAGCCAG
GAGAGTGTGACTGAACAGGACTCAAAAGATAGCACCTATTCCCTGTCTAGTACACTGACT
CTGTCCAAGGCTGATTACGAGAAGCACAAAGTGTATGCATGCGAAGTGACACATCAGGG
ACTGTCAAGCCCCGTGACTAAGTCTTTTAACCGGGGCGAATGT
**Amino acid sequence of 14C12H1L1 light chain:** (SEQ ID NO: 68) 
DIQMTQSPSSMSASVGDRVTFTCRASQDINTYLSWFQQKPGKSPKTLIYRANRLVSGVPSRFS
GSGSGQDYTLTISSLQPEDMATYYCLQYDEFPLTFGAGTKLELKRTVAAPSVFIFPPSDEQLKS
GTASVVCLLNNFYPREAKVQWKVDNALQSGNSQESVTEQDSKDSTYSLSSTLTLSKADYEKH
KVYACEVTHQGLSSPVTKSFNRGEC
Nucleotide sequence o**f 14C12H1L1(G1TM) heavy chain** (SEQ ID NO: 69) 
GAAGTGCAGCTGGTCGAGTCTGGGGGAGGGCTGGTGCAGCCCGGCGGGTCACTGCGACT
GAGCTGCGCAGCTTCCGGATTCGCCTTTAGCTCCTACGACATGTCCTGGGTGCGACAGGC
ACCAGGAAAGGGACTGGATTGGGTCGCTACTATCTCAGGAGGCGGGAGATACACCTACT
ATCCTGACAGCGTCAAGGGCCGGTTCACAATCTCTAGAGATAACAGTAAGAACAATCTGT
 
ATCTGCAGATGAACAGCCTGAGGGCTGAGGACACCGCACTGTACTATTGTGCCAACCGCT
ACGGGGAAGCATGGTTTGCCTATTGGGGGCAGGGAACCCTGGTGACAGTCTCTAGTGCCA
GCACCAAAGGGCCCAGCGTGTTTCCTCTCGCCCCCTCCTCCAAAAGCACCAGCGGAGGAA
CCGCTGCTCTCGGATGTCTGGTGAAGGACTACTTCCCTGAACCCGTCACCGTGAGCTGGA
ATAGCGGCGCTCTGACAAGCGGAGTCCATACATTCCCTGCTGTGCTGCAAAGCAGCGGAC
TCTATTCCCTGTCCAGCGTCGTCACAGTGCCCAGCAGCAGCCTGGGCACCCAGACCTACA
TCTGTAACGTCAACCACAAGCCCTCCAACACCAAGGTGGACAAGAAAGTGGAGCCCAAA
TCCTGCGACAAGACACACACCTGTCCCCCCTGTCCTGCTCCCGAAGCTGCTGGAGCCCCT
AGCGTCTTCCTCTTTCCTCCCAAACCCAAGGACACCCTCATGATCAGCAGAACCCCTGAA
GTCACCTGTGTCGTCGTGGATGTCAGCCATGAGGACCCCGAGGTGAAATTCAACTGGTAT
GTCGATGGCGTCGAGGTGCACAACGCCAAAACCAAGCCCAGGGAGGAACAGTACAACTC
CACCTACAGGGTGGTGTCCGTGCTGACAGTCCTCCACCAGGACTGGCTGAACGGCAAGGA
GTACAAGTGCAAGGTGTCCAACAAGGCTCTCCCTGCCCCCATTGAGAAGACCATCAGCAA
GGCCAAAGGCCAACCCAGGGAGCCCCAGGTCTATACACTGCCTCCCTCCAGGGACGAACT
CACCAAGAACCAGGTGTCCCTGACCTGCCTGGTCAAGGGCTTTTATCCCAGCGACATCGC
CGTCGAGTGGGAGTCCAACGGACAGCCCGAGAATAACTACAAGACCACCCCTCCTGTCCT
CGACTCCGACGGCTCCTTCTTCCTGTACAGCAAGCTGACCGTGGACAAAAGCAGGTGGCA
GCAGGGAAACGTGTTCTCCTGCAGCGTGATGCACGAAGCCCTCCACAACCACTACACCCA
GAAAAGCCTGTCCCTGAGCCCCGGCAAA
**Amino acid sequence** of 14C12H1L1(G1TM) heavy chain variable region: (SEQ ID NO: 70) 
EVQLVESGGGLVQPGGSLRLSCAASGFAFSSYDMSWVRQAPGKGLDWVATISGGGRYTYYP
DSVKGRFTISRDNSKNNLYLQMNSLRAEDTALYYCANRYGEAWFAYWGQGTLVTVSSASTK
GPSVFPLAPSSKSTSGGTAALGCLVKDYFPEPVTVSWNSGALTSGVHTFPAVLQSSGLYSLSSV
VTVPSSSLGTQTYICNVNHKPSNTKVDKKVEPKSCDKTHTCPPCPAPEAAGAPSVFLFPPKPK
DTLMISRTPEVTCVVVDVSHEDPEVKFNWYVDGVEVHNAKTKPREEQYNSTYRVVSVLTVL
HQDWLNGKEYKCKVSNKALPAPIEKTISKAKGQPREPQVYTLPPSRDELTKNQVSLTCLVKG
FYPSDIAVEWESNGQPENNYKTTPPVLDSDGSFFLYSKLTVDKSRWQQGNVFSCSVMHEALH
NHYTQKSLSLSPGK
**Amino acid sequences of 14C12H1 heavy chain framework regions:**
   FR-H1: EVQLVESGGGLVQPGGSLRLSCAAS (SEQ ID NO: 71)
   FR-H2: MSWVRQAPGKGLDWVAT (SEQ ID NO: 72)
   FR-H3: YYPDSVKGRFTISRDNSKNNLYLQMNSLRAEDTALYYC (SEQ ID NO: 73)
   FR-H4: WGQGTLVTVSS (SEQ ID NO: 74)
**Amino acid sequences of 14C12L1 light chain framework regions:**
   FR-L1: DIQMTQSPSSMSASVGDRVTFTCRAS (SEQ ID NO: 75)
   FR-L2: LSWFQQKPGKSPKTLIY (SEQ ID NO: 76)
   FR-L3: RLVSGVPSRFSGSGSGQDYTLTISSLQPEDMATYYC (SEQ ID NO: 77)
   FR-L4: FGAGTKLELK (SEQ ID NO: 78)
**Amino acid sequence of Linker1: GGGGSGGGGSGGGGSGGGGS** (SEQ ID NO: 79)
   Nucleotide **sequence of Linker1:** (SEQ ID NO: 80) 
GGCGGCGGCGGCAGCGGCGGCGGCGGCTCCGGAGGAGGCGGCTCTGGCGGCGGCGGCAG
C
**Amino acid sequence of Linker2:** GGGGSGGGGSGGGGS (SEQ ID NO: 81)
   Nucleotide **sequence of Linker2:** (SEQ ID NO: 82) GGCGGCGGCGGCTCCGGAGGAGGCGGCTCTGGCGGCGGCGGCAGC
The amino acid sequence of the heavy chain of NTPDV2 and NTPDV4 (SEQ ID NO: 83): wherein the CDR regions of 19F3H2(hG1TM) in the immunoglobulin moiety are marked in **bold underlines,** the CDR regions of 14C12H1V-Linker1-14C12L1V in the scFv part are marked in **bold underlines,** the mutated amino acids in the heavy chain region are marked in ***bold italics***, and the linker regions are marked in **bold:** 
QVQLVQSGAEVVKPGASVKVSCKASGYSFTGYTMNWVRQAPGQNLEWIGLINPYNAGTSY
NQKFQGKVTLTVDKSTSTAYMELSSLRSEDTAVYYCARSEYRYGGDYFDYWGQGTTLTVSS
ASTKGPSVFPLAPSSKSTSGGTAALGCLVKDYFPEPVTVSWNSGALTSGVHTFPAVLQSSGLY
SLSSVVTVPSSSLGTQTYICNVNHKPSNTKVDKKVEPKSCDKTHTCPPCPAPEAAGAPSVFLFP
PKPKDTLMISRTPEVTCVVVDVSHEDPEVKFNWYVDGVEVHNAKTKPREEQYNSTYRVVSV
LTVLHQDWLNGKEYKCKVSNKALPAPIEKTISKAKGQPREPQVYTLPPSRDELTKNQVSLTCL
VKGFYPSDIAVEWESNGQPENNYKTTPPVLDSDGSFFLYSKLTVDKSRWQQGNVFSCSVMHE
ALHNHYTQKSLSLSPGKGGGGSGGGGSGGGGSGGGGSEVQLVESGGGLVQPGGSLRLSCAAS
GFAFSSYDMSWVRQAPGKGLDWVATISGGGRYTYYPDSVKGRFTISRDNSKNNLYLQMNSLRAE
DTALYYCANRYGEAWFAYWGQGTLVTVSSGGGGSGGGGSGGGGSGGGGSDIQMTQSPSSMS
ASVGDRVTFTCRASQDINTYLSWFQQKPGKSPKTLIYRANRLVSGVPSRFSGSGSGQDYTLTISSLQ
PEDMATYYCLQYDEFPLTFGAGTKLELKR
Nucleotide sequence of the heavy chain of NTPDV2 and NTPDV4: (SEQ ID NO: 84) 
CAGGTGCAGCTGGTGCAGTCTGGAGCAGAGGTGGTGAAGCCAGGAGCCTCTGTGAAGGT
GAGCTGTAAGGCCAGCGGCTACTCCTTCACCGGCTATACAATGAACTGGGTGAGGCAGGC
ACCAGGACAGAATCTGGAGTGGATCGGCCTGATCAACCCTTACAATGCCGGCACCTCTTA
TAACCAGAAGTTTCAGGGCAAGGTGACCCTGACAGTGGACAAGTCCACCTCTACAGCCTA
CATGGAGCTGAGCTCCCTGCGGAGCGAGGATACAGCCGTGTACTATTGCGCCCGGTCCGA
GTACAGATATGGCGGCGACTACTTTGATTATTGGGGCCAGGGCACCACACTGACCGTGTC
TAGCgcctccacaaaggggcccagcgtgtttcctctcgccccctcctccaaaagcaccagcggaggaaccgctgctctcggatgtctggtgaa
ggactacttccctgaacccgtcaccgtgagctggaatagcggcgctctgacaagcggagtccatacattccctgctgtgctgcaaagcagcggact
ctattccctgtccagcgtcgtcacagtgcccagcagcagcctgggcacccagacctacatctgtaacgtcaaccacaagccctccaacaccaaggt
ggacaagaaagtggagcccaaatcctgcgacaagacacacacctgtcccccctgtcctgctcccgaaGCTGCTggagCccctagcgtcttcc
tctttcctcccaaacccaaggacaccctcatgatcagcagaacccctgaagtcacctgtgtcgtcgtggatgtcagccatgaggaccccgaggtgaa
attcaactggtatgtcgatggcgtcgaggtgcacaacgccaaaaccaagcccagggaggaacagtacaactccacctacagggtggtgtccgtgct
gacagtcctccaccaggactggctgaacggcaaggagtacaagtgcaaggtgtccaacaaggctctccctgcccccattgagaagaccatcagca
aggccaaaggccaacccagggagccccaggtctatacactgcctccctccagggacgaactcaccaagaaccaggtgtccctgacctgcctggtc
aagggcttttatcccagcgacatcgccgtcgagtgggagtccaacggacagcccgagaataactacaagaccacccctcctgtcctcgactccgac
ggctccttcttcctgtacagcaaactgaccgtcgataaatctaggtggcagcagggcaacgtgttctcttgttccgtgatgcatgaagcactgcacaac
cattatacccagaagtctctgagcctgtcccccggcaagGGCGGCGGCGGCTCTGGAGGAGGAGGCAGCGGCGG
AGGAGGCTCCGGAGGCGGCGGCTCTGAGGTGCAGCTGGTGGAGTCTGGAGGAGGACTGG
TGCAGCCTGGAGGCTCCCTGAGGCTGTCTTGCGCAGCAAGCGGATTCGCCTTTAGCTCCT
ACGACATGAGCTGGGTGCGGCAGGCACCTGGCAAGggtCTGGATTGGGTGGCAACCATCA
GCGGAGGCGGCAGATACACATACTATCCCGACTCCGTGAAGGGCAGGTTCACCATCTCCC
GCGATAACTCTAAGAACAATCTGTATCTGCAGATGAACAGCCTGAGGGCCGAGGACACA
GCCCTGTACTATTGCGCCAACCGCTACGGCGAGGCCTGGTTTGCCTATTGGGGCCAGGGC
ACCCTGGTGACAGTGTCTAGCGGCGGCGGCGGCAGCGGCGGCGGCGGCTCCGGAGGAGG
CGGCTCTGGCGGCGGCGGCAGCGATATCCAGATGACCCAGTCCCCCTCCTCTATGTCTGC
CAGCGTGGGCGACCGGGTGACCTTCACATGTAGAGCCTCCCAGGATATCAACACCTACCT
GTCTTGGTTTCAGCAGAAGCCCGGCAAGAGCCCTAAGACACTGATCTATCGGGCCAATAG
ACTGGTGAGCGGAGTGCCTTCCCGGTTCTCCGGCTCTGGCAGCGGACAGGACTATACCCT
GACAATCAGCTCCCTGCAGCCAGAGGATATGGCCACATACTATTGCCTGCAGTATGACGA
GTTCCCCCTGACCTTCGGGgctGGCACTAAGCTGGAGCTGAAAAGA
The amino acid sequence of the heavy chain of NTPDV1 and NTPDV3 (SEQ ID NO: 85): wherein the CDR regions of 19F3H2(hG1TM) in the immunoglobulin moiety are marked in **bold underlines,** the CDR regions of 14C12H1V-Linker2-14C12L1V in the scFv part are marked in **bold underlines,** the mutated amino acids in the heavy chain region are marked in ***bold italics***, and the linker regions are marked in **bold:** 
QVQLVQSGAEVVKPGASVKVSCKASGYSFTGYTMNWVRQAPGQNLEWIGLINPYNAGTSY
NQKFQGKVTLTVDKSTSTAYMELSSLRSEDTAVYYCARSEYRYGGDYFDYWGQGTTLTVSS
ASTKGPSVFPLAPSSKSTSGGTAALGCLVKDYFPEPVTVSWNSGALTSGVHTFPAVLQSSGLY
SLSSVVTVPSSSLGTQTYICNVNHKPSNTKVDKKVEPKSCDKTHTCPPCPAPEAAGAPSVFLFP
PKPKDTLMISRTPEVTCVVVDVSHEDPEVKFNWYVDGVEVHNAKTKPREEQYNSTYRVVSV
LTVLHQDWLNGKEYKCKVSNKALPAPIEKTISKAKGQPREPQVYTLPPSRDELTKNQVSLTCL
VKGFYPSDIAVEWESNGQPENNYKTTPPVLDSDGSFFLYSKLTVDKSRWQQGNVFSCSVMHE
 
ALHNHYTQKSLSLSPGKGGGGSGGGGSGGGGSGGGGSEVQLVESGGGLVQPGGSLRLSCAAS
GFAFSSYDMSWVRQAPGKGLDWVATISGGGRYTYYPDSVKGRFTISRDNSKNNLYLQMNSLRAE
DTALYYCANRYGEAWFAYWGQGTLVTVSSGGGGSGGGGSGGGGSDIQMTQSPSSMSASVGD
RVTFTCRASQDINTYLSWFQQKPGKSPKTLIYRANRLVSGVPSRFSGSGSGQDYTLTISSLQPEDMA
TYYCLQYDEFPLTFGAGTKLELKR
**Nucleotide sequence of the heavy chain of NTPDV1 and NTPDV3:** (SEQ ID NO: 86) 
CAGGTGCAGCTGGTGCAGTCTGGAGCAGAGGTGGTGAAGCCAGGAGCCTCTGTGAAGGT
GAGCTGTAAGGCCAGCGGCTACTCCTTCACCGGCTATACAATGAACTGGGTGAGGCAGGC
ACCAGGACAGAATCTGGAGTGGATCGGCCTGATCAACCCTTACAATGCCGGCACCTCTTA
TAACCAGAAGTTTCAGGGCAAGGTGACCCTGACAGTGGACAAGTCCACCTCTACAGCCTA
CATGGAGCTGAGCTCCCTGCGGAGCGAGGATACAGCCGTGTACTATTGCGCCCGGTCCGA
GTACAGATATGGCGGCGACTACTTTGATTATTGGGGCCAGGGCACCACACTGACCGTGTC
TAGCGCCTCCACAAaggggcccagcgtgtttcctctcgccccctcctccaaaagcaccagcggaggaaccgctgctctcggatgtctg
gtgaaggactacttccctgaacccgtcaccgtgagctggaatagcggcgctctgacaagcggagtccatacattccctgctgtgctgcaaagcagc
ggactctattccctgtccagcgtcgtcacagtgcccagcagcagcctgggcacccagacctacatctgtaacgtcaaccacaagccctccaacacc
aaggtggacaagaaagtggagcccaaatcctgcgacaagacacacacctgtcccccctgtcctgctcccgaaGCTGCTggagCccctagcg
tcttcctctttcctcccaaacccaaggacaccctcatgatcagcagaaccCCTGAAGTCACCTGTGTCGTCGTGGATGTC
AGCCATGAGGACCCCGAGGTGAAATTCAACTGGTATGTCGATGGCGTCGAGGTGCACAA
CGCCAAAACCAAGCCCAGGGAGGAACAGTACAACTCCACCTACAGGGTGGTGTCCGTGC
TGACAGTCCTCCACCAGGACTGGCTGAACGGCAAGGAGTACAAGTGCAAGGTGTCCAAC
AAGGCTCTCCCTGCCCCCATTGAGAAGACCATCAGCAAGGCCAAAGGCCAACCCAGGGA
GCCCCAGGTCTATACACTGCCTCCCTCCAGGGACGAACTCACCAAGAACCAGGTGTCCCT
GACCTGCCTGGTCAAGGGCTTTTATCCCAGCGACATCGCCGTCGAGTGGGAGTCCAACGG
ACAGCCCGAGAATAACTACAAGACCACCCCTCCTGTCCTCGACTCCGACGGCTCCTTCTTC
CTGTACAGCAAACTGACCGTCGATAAATCTAGGTGGCAGCAGGGCAACGTGTTCTCTTGT
TCCGTGATGCATGAAGCACTGCACAACCATTATACCCAGAAGTCTCTGAGCCTGTCCCCC
GGCAAGGGCGGCGGCGGCTCTGGAGGAGGAGGCAGCGGCGGAGGAGGCTCCGGAGGCG
GCGGCTCTGAGGTGCAGCTGGTGGAGTCTGGAGGAGGACTGGTGCAGCCTGGAGGCTCC
CTGAGGCTGTCTTGCGCAGCAAGCGGATTCGCCTTTAGCTCCTACGACATGAGCTGGGTG
CGGCAGGCACCTGGCAAGGGTCTGGATTGGGTGGCAACCATCAGCGGAGGCGGCAGATA
CACATACTATCCCGACTCCGTGAAGGGCAGGTTCACCATCTCCCGCGATAACTCTAAGAA
CAATCTGTATCTGCAGATGAACAGCCTGAGGGCCGAGGACACAGCCCTGTACTATTGCGC
CAACCGCTACGGCGAGGCCTGGTTTGCCTATTGGGGCCAGGGCACCCTGGTGACAGTGTC
TAGCGGCGGCGGCGGCTCCGGAGGAGGCGGCTCTGGCGGCGGCGGCAGCGATATCCAGA
TGACCCAGTCCCCCTCCTCTATGTCTGCCAGCGTGGGCGACCGGGTGACCTTCACATGTAG
AGCCTCCCAGGATATCAACACCTACCTGTCTTGGTTTCAGCAGAAGCCCGGCAAGAGCCC
TAAGACACTGATCTATCGGGCCAATAGACTGGTGAGCGGAGTGCCTTCCCGGTTCTCCGG
CTCTGGCAGCGGACAGGACTATACCCTGACAATCAGCTCCCTGCAGCCAGAGGATATGGC
CACATACTATTGCCTGCAGTATGACGAGTTCCCCCTGACCTTCGGGGCTGGCACTAAGCT
GGAGCTGAAAAGATGATAAGAATTC
Amino acid sequence of **NTSE(1-552)-his** (SEQ ID NO: 87) 
MCPRAARAPATLLLALGAVLWPAAGAWELTILHTNDVHSRLEQTSEDSSKCVNASRCMGGV
ARLFTKVQQIRRAEPNVLLLDAGDQYQGTIWFTVYKGAEVAHFMNALRYDAMALGNHEFD
NGVEGLIEPLLKEAKFPILSANIKAKGPLASQISGLYLPYKVLPVGDEVVGIVGYTSKETPFLSN
PGTNLVFEDEITALQPEVDKLKTLNVNKIIALGHSGFEMDKLIAQKVRGVDVVVGGHSNTFLY
TGNPPSKEVPAGKYPFIVTSDDGRKVPVVQAYAFGKYLGYLKIEFDERGNVISSHGNPILLNSS
IPEDPSIKADINKWRIKLDNYSTQELGKTIVYLDGSSQSCRFRECNMGNLICDAMINNNLRHTD
EMFWNHVSMCILNGGGIRSPIDERNNGTITWENLAAVLPFGGTFDLVQLKGSTLKKAFEHSV
HRYGQSTGEFLQVGGIHVVYDLSRKPGDRVVKLDVLCTKCRVPSYDPLKMDEVYKVILPNFL
ANGGDGFQMIKDELLRHDSGDQDINVVSTYISKMKVIYPAVEGRIKFSTGSHHHHHH
Nucleotide sequence of **NT5E(1-552)-his** (SEQ ID NO: 88) 
ATGTGTCCTAGAGCCGCCAGAGCTCCAGCTACACTGCTGCTGGCTCTGGGAGCAGTCCTC
TGGCCAGCAGCAGGAGCTTGGGAACTGACCATCCTGCACACCAACGACGTGCACAGCAG
GCTGGAACAGACCAGCGAGGACAGCAGCAAGTGCGTGAACGCCAGTCGCTGTATGGGAG
GAGTGGCAAGGCTGTTCACCAAGGTGCAGCAGATCCGGAGAGCCGAACCTAACGTGCTG
 
CTGCTGGACGCCGGAGATCAGTATCAGGGAACCATCTGGTTCACCGTGTACAAGGGCGCC
GAAGTGGCCCACTTCATGAACGCTCTGCGCTACGACGCTATGGCCCTGGGCAATCACGAG
TTCGATAACGGCGTGGAGGGACTGATCGAGCCTCTGCTGAAGGAGGCCAAGTTCCCCATC
CTGAGCGCCAACATCAAGGCCAAGGGACCTCTGGCTAGCCAGATTAGCGGCCTGTACCTG
CCTTACAAGGTGCTGCCCGTGGGAGACGAAGTGGTGGGAATCGTGGGCTACACCAGCAA
GGAGACCCCTTTCCTGAGCAACCCAGGCACCAACCTGGTGTTCGAGGACGAGATCACCGC
TCTGCAGCCAGAGGTGGACAAGCTGAAGACCCTGAACGTGAACAAGATCATCGCCCTGG
GACACAGCGGCTTCGAGATGGACAAGCTGATCGCCCAGAAAGTGCGAGGAGTGGACGTG
GTCGTGGGCGGACACAGCAACACCTTCCTGTACACCGGCAACCCTCCTTCTAAGGAAGTG
CCAGCCGGCAAGTACCCCTTCATCGTGACCAGCGACGACGGAAGAAAGGTGCCAGTGGT
GCAGGCTTACGCCTTCGGCAAGTACCTGGGCTACCTGAAGATCGAGTTCGACGAGCGGGG
CAACGTGATCTCTAGCCACGGCAACCCCATCCTGCTGAACAGCAGCATCCCAGAGGACCC
CAGCATCAAGGCCGACATCAACAAGTGGCGGATCAAGCTGGACAACTACAGCACCCAGG
AGCTGGGAAAGACCATCGTGTACCTGGACGGCAGCTCTCAGTCTTGCCGGTTCCGCGAGT
GCAACATGGGCAACCTGATTTGCGACGCCATGATCAACAACAACCTGCGGCACACCGAC
GAGATGTTTTGGAACCACGTCAGCATGTGCATCCTGAACGGCGGAGGCATCAGAAGCCCT
ATTGACGAGCGGAACAACGGCACCATCACTTGGGAGAACCTGGCAGCAGTGCTGCCTTTT
GGCGGAACATTCGACCTGGTGCAGCTGAAGGGCAGCACACTGAAGAAGGCCTTCGAGCA
CAGCGTGCACAGATACGGCCAGAGCACAGGCGAGTTCCTGCAGGTCGGAGGAATCCACG
TGGTGTACGACCTGAGCAGGAAGCCAGGAGACAGAGTGGTGAAGCTGGACGTGCTCTGC
ACCAAGTGTCGGGTGCCAAGCTACGACCCCCTGAAGATGGACGAGGTGTACAAGGTCAT
CCTGCCCAACTTCCTGGCTAACGGAGGAGACGGCTTCCAGATGATCAAGGACGAGCTGCT
GAGGCACGACAGCGGAGACCAGGACATCAACGTCGTGTCCACCTACATCAGCAAGATGA
AGGTCATCTACCCCGCCGTGGAAGGCAGGATCAAGTTCAGCACCGGCTCTCACCACCACC
ATCACCAC
Amino acid sequence of **mFc:** (SEQ ID NO: 89) 
PRGPTIKPCPPCKCPAPNLLGGPSVFIFPPKIKDVLMISLSPIVTCVVVDVSEDDPDVQISWFVN
NVEVHTAQTQTHREDYNSTLRVVSALPIQHQDWMSGKEFKCKVNNKDLPAPIERTISKPKGS
VRAPQVYVLPPPEEEMTKKQVTLTCMVTDFMPEDIYVEWTNNGKTELNYKNTEPVLDSDGS
YFMYSKLRVEKKNWVERNSYSCSVVHEGLHNHHTTKSFSRTPGK
Amino acid sequence **of** 19F3H1V-Linker-19F3L2V: (SEQ ID NO: 90) 
QVQLQQSGAEVVKPGASMKMSCKASGYSFTGYTMNWVKQAHGQNLEWIGLINPYNAGTSY
NQKFQGKATLTVDKSTSTAYMELSSLRSEDTAVYYCARSEYRYGGDYFDYWGQGTTLTVSS
GGGGSGGGGSGGGGSDIVMTQSPSSLAVSVGERVTISCKSSQSLLNSSNQKNYLAWYQQKPG
QAPKLLIYFASTRESGVPDRFSGSGSGTDFTLTISSVQAEDVADYYCQQHYDTPYTFGGGTKL
EIK
Nucleotide sequence **of** 19F3H1V-Linker2-19F3L2V: (SEQ ID NO: 91 
CAGGTGCAGCTGCAGCAGTCTGGAGCAGAGGTGGTGAAGCCAGGAGCCTCTATGAAGAT
GAGCTGTAAGGCCAGCGGCTACTCCTTCACCGGCTATACAATGAACTGGGTGAAGCAGGC
CCACGGCCAGAATCTGGAGTGGATCGGCCTGATCAACCCTTACAATGCCGGCACCTCTTA
TAACCAGAAGTTTCAGGGCAAGGCCACCCTGACAGTGGACAAGTCCACCTCTACAGCCTA
CATGGAGCTGAGCTCCCTGCGGAGCGAGGATACAGCCGTGTACTATTGCGCCCGGTCCGA
GTACAGATATGGCGGCGACTACTTTGATTATTGGGGCCAGGGCACCACACTGACCGTGTC
TAGC
GGCCTGTACGGCCTGTACGGCCTGTACGGCCTGTACAGCGAGAGGGGCCTGTACGGCCTG
TACGGCCTGTACGGCCTGTACAGCGAGAGGGGCCTGTACGGCCTGTACGGCCTGTACGGC
CTGTACAGCGAGAGGGACATCGTGATGACCCAGTCCCCAAGCTCCCTGGCCGTGTCTGTG
GGAGAGCGGGTGACAATCTCCTGTAAGTCTAGCCAGTCTCTGCTGAACTCCTCTAATCAG
AAGAACTACCTGGCCTGGTATCAGCAGAAGCCCGGCCAGGCCCCTAAGCTGCTGATCTAC
TTCGCCTCTACCAGGGAGAGCGGAGTGCCAGACAGATTCTCTGGCAGCGGCTCCGGCACA
GACTTCACCCTGACAATCAGCTCCGTGCAGGCAGAGGACGTGGCAGATTACTATTGCCAG
CAGCACTACGATACCCCCTATACATTTGGCGGCGGCACCAAGCTGGAGATCAAG
**Nucleotide sequence of 19F3H1:** (SEQ ID NO: 92) 
CAGGTGCAGCTGCAGCAGTCTGGAGCAGAGGTGGTGAAGCCAGGAGCCTCTATGAAGAT
GAGCTGTAAGGCCAGCGGCTACTCCTTCACCGGCTATACAATGAACTGGGTGAAGCAGGC
CCACGGCCAGAATCTGGAGTGGATCGGCCTGATCAACCCTTACAATGCCGGCACCTCTTA
TAACCAGAAGTTTCAGGGCAAGGCCACCCTGACAGTGGACAAGTCCACCTCTACAGCCTA
CATGGAGCTGAGCTCCCTGCGGAGCGAGGATACAGCCGTGTACTATTGCGCCCGGTCCGA
GTACAGATATGGCGGCGACTACTTTGATTATTGGGGCCAGGGCACCACACTGACCGTGTC
TAGC
**Amino acid sequence of 19F3H1:** (SEQ ID NO: 93) 
QVQLQQSGAEVVKPGASMKMSCKASGYSFTGYTMNWVKQAHGQNLEWIGLINPYNAGTSY
NQKFQGKATLTVDKSTSTAYMELSSLRSEDTAVYYCARSEYRYGGDYFDYWGQGTTLTVSS
**Nucleotide sequence of 19F3L1:** (SEQ ID NO: 94) 
GACATCGTGATGACCCAGTCCCCAAGCTCCCTGGCAATGTCTGTGGGAGAGAGGGTGACA
ATGTCCTGTAAGTCTAGCCAGTCTCTGCTGAACTCCTCTAATCAGAAGAACTACCTGGCCT
GGTATCAGCAGAAGCCCGGCCAGGCCCCTAAGCTGCTGGTGTACTTTGCCTCTACCAGGG
AGAGCGGAGTGCCAGACAGATTCTCTGGCAGCGGCTCCGGCACAGACTTCACCCTGACAA
TCAGCTCCGTGCAGGCAGAGGACCTGGCAGATTATTTCTGCCAGCAGCACTACGATACCC
CCTATACATTTGGCGGCGGCACCAAGCTGGAGATCAAG
**Amino acid sequence of 19F3L1:** (SEQ ID NO: 95) 
DIVMTQSPSSLAMSVGERVTMSCKSSQSLLNSSNQKNYLAWYQQKPGQAPKLLVYFASTRES
GVPDRFSGSGSGTDFTLTISSVQAEDLADYFCQQHYDTPYTFGGGTKLEIK
Amino acid of **19F3L2** light chain (full length) (SEQ ID NO: 96, with non-variable region sequences underlined 
DIVMTQSPSSLAVSVGERVTISCKSSQSLLNSSNQKNYLAWYQQKPGQAPKLLIYFASTRESG
VPDRFSGSGSGTDFTLTISSVQAEDVADYYCQQHYDTPYTFGGGTKLEIKRTVAAPSVFIFPPS
DEQLKSGTASVVCLLNNFYPREAKVQWKVDNALQSGNSQESVTEQDSKDSTYSLSSTLTLSKADYEKH
KVYACEVTHQGLSSPVTKSFNRGEC
**Amino acid sequence of 19F3H2 heavy chain variable region with CDR sequences underlined: (SEQ ID NO: 97)** 
QVQLVQSGAEVVKPGASVKVSCKASGYSFTGYTMNWVRQAPGQNLEWIGLINPYNAGTSYN
QKFQGKVTLTVDKSTSTAYMELSSLRSEDTAVYYCARSEYRYGGDYFDYWGQGTTLTVSS
**Amino acid sequence of 19F3L2 heavy chain variable region with CDR sequences underlined: (SEQ ID NO: 98)** 
DIVMTQSPSSLAVSVGERVTISCKSSQSLLNSSNQKNYLAWYQQKPGQAPKLLIYFASTRESG
VPDRFSGSGSGTDFTLTISSVQAEDVADYYCQQHYDTPYTFGGGTKLEIK
**Amino acid sequence of 19F3L3 heavy chain variable region with CDR sequences underlined: (SEQ ID NO: 99)** 
DIVMTQSPSSLAVSVGERVTISCKSSQSLLNSSNQKNYLAWYQQKPGQAPKLLIYFASTRESG
VPDRFSGSGSGTDFTLTISSLQAEDVAVYYCQQHYDTPYTFGGGTKLEIK
Nucleotide sequence of **19F3L2** light chain (full length) (SEQ ID NO: 100)

GACATCGTGATGACCCAGTCCCCAAGCTCCCTGGCCGTGTCTGTGGGAGAGCGGGTGACAATCTCCTG
TAAGTCTAGCCAGTCTCTGCTGAACTCCTCTAATCAGAAGAACTACCTGGCCTGGTATCAGCAGAAGC
CCGGCCAGGCCCCTAAGCTGCTGATCTACTTCGCCTCTACCAGGGAGAGCGGAGTGCCAGACAGATTC
TCTGGCAGCGGCTCCGGCACAGACTTCACCCTGACAATCAGCTCCGTGCAGGCAGAGGACGTGGCAGA
TTACTATTGCCAGCAGCACTACGATACCCCCTATACATTTGGCGGCGGCACCAAGCTGGAGATCAAGC
GTACGGTGGCAGCCCCATCTGTCTTCATTTTTCCCCCTAGTGACGAGCAGCTGAAATCCGGAACAGCC
TCTGTGGTCTGTCTGCTGAACAATTTCTACCCTCGCGAAGCCAAGGTGCAGTGGAAAGTCGATAACGC
TCTGCAGAGTGGCAATTCACAGGAGAGCGTGACTGAACAGGACTCCAAGGATTCTACCTATAGTCTGA
GCTCCACTCTGACCCTGTCCAAAGCAGATTACGAAAAGCACAAAGTGTATGCCTGTGAGGTCACCCAC
CAGGGGCTGAGTTCTCCAGTCACCAAATCCTTCAACAGAGGCGAATGT

## Claims

1. An anti-CD73/anti-PD-1 bispecific antibody comprising:
a first protein functional region targeting PD-1, and
a second protein functional region targeting CD73,
wherein
the first protein functional region comprises: HCDR1, HCDR2 and HCDR3 contained in a heavy chain variable region having an amino acid sequence set forth in SEQ ID NO: 44,
wherein preferably the amino acid sequences of HCDR1, HCDR2 and HCDR3 are sequences set forth in SEQ ID NOs: 45-47, respectively, or sequences having at least 80%, 81%, 82%, 83%, 84%, 85%, 86%, 87%, 88%, 89% or 90%, preferably at least 91%, 92%, 93%, 94%, 95%, 96%, 97%, 98% or 99% sequence identity to the sequences set forth in SEQ ID NOs: 45-47, or amino acid sequences having one or more (preferably 1, 2 or 3) conservative amino acid mutations (preferably substitutions, insertions or deletions) compared with the sequences set forth in SEQ ID NOs: 45-47; and
LCDR1, LCDR2 and LCDR3 contained in a light chain variable region having an amino acid sequence set forth in SEQ ID NO: 49, wherein preferably the amino acid sequences of LCDR1, LCDR2 and LCDR3 are sequences set forth in SEQ ID NOs: 50-52, respectively, or sequences having at least 80%, 81%, 82%, 83%, 84%, 85%, 86%, 87%, 88%, 89% or 90%, preferably at least 91%, 92%, 93%, 94%, 95%, 96%, 97%, 98% or 99% sequence identity to the sequences set forth in SEQ ID NOs: 50-52, or amino acid sequences having one or more (preferably 1, 2 or 3) conservative amino acid mutations (preferably substitutions, insertions or deletions) compared with the sequences set forth in SEQ ID NOs: 50-52;
or the second protein functional region comprises: HCDR1, HCDR2 and HCDR3 contained in a heavy chain variable region having an amino acid sequence set forth in SEQ ID NO: 2, wherein preferably the amino acid sequences of HCDR1, HCDR2 and HCDR3 are sequences set forth in SEQ ID NOs: 3-5, respectively, or sequences having at least 80%, 81%, 82%, 83%, 84%, 85%, 86%, 87%, 88%, 89% or 90%, preferably at least 91%, 92%, 93%, 94%, 95%, 96%, 97%, 98% or 99% sequence identity to the sequences set forth in SEQ ID NOs: 3-5, or amino acid sequences having one or more (preferably 1, 2 or 3) conservative amino acid mutations (preferably substitutions, insertions or deletions) compared with the sequences set forth in SEQ ID NOs: 3-5; and
LCDR1, LCDR2 and LCDR3 contained in a light chain variable region having an amino acid sequence set forth in SEQ ID NO: 7, wherein preferably the amino acid sequences of LCDR1, LCDR2 and LCDR3 are sequences set forth in SEQ ID NOs: 8-10, respectively, or sequences having at least 80%, 81%, 82%, 83%, 84%, 85%, 86%, 87%, 88%, 89% or 90%, preferably at least 91%, 92%, 93%, 94%, 95%, 96%, 97%, 98% or 99% sequence identity to the sequences set forth in SEQ ID NOs: 8-10, or amino acid sequences having one or more (preferably 1, 2 or 3) conservative amino acid mutations (preferably substitutions, insertions or deletions) compared with the sequences set forth in SEQ ID NOs: 8-10.

2. The anti-CD73/anti-PD-1 bispecific antibody according to claim 1, wherein:
the first protein functional region comprises:
a sequence having an amino acid sequence set forth in SEQ ID NO: 44 or SEQ ID NO: 62, or a sequence having at least 80%, 81%, 82%, 83%, 84%, 85%, 86%, 87%, 88%, 89% or 90%, preferably at least 91%, 92%, 93%, 94%, 95%, 96%, 97%, 98% or 99% sequence identity to the sequence set forth in SEQ ID NO: 44 or 62, or an amino acid sequence having one or more (preferably 1, 2 or 3) conservative amino acid mutations (preferably substitutions, insertions or deletions) compared with the sequences set forth in SEQ ID NO: 44 or 62; and
a sequence having an amino acid sequence correspondingly set forth in SEQ ID NO: 49 or SEQ ID NO: 64, or a sequence having at least 80%, 81%, 82%, 83%, 84%, 85% or 90%, preferably at least 91%, 92%, 93%, 94%, 95%, 96%, 97%, 98% or 99% sequence identity to the sequence set forth in SEQ ID NO: 49 or 64, or an amino acid sequence having one or more (preferably 1, 2 or 3) conservative amino acid mutations (preferably substitutions, insertions or deletions) compared with the sequences set forth in SEQ ID NO: 49 or 64;
and/or,
the second protein functional region comprises a sequence having an amino acid sequence set forth in SEQ ID NO: 2 or SEQ ID NO: 20, or a sequence having at least 80%, 81%, 82%, 83%, 84%, 85%, 86%, 87%, 88%, 89% or 90%, preferably at least 91%, 92%, 93%, 94%, 95%, 96%, 97%, 98% or 99% sequence identity to the sequence set forth in SEQ ID NO: 2 or 20, or an amino acid sequence having one or more (preferably 1, 2 or 3) conservative amino acid mutations (preferably substitutions, insertions or deletions) compared with the sequences set forth in SEQ ID NO: 2 or 20; and
a sequence having an amino acid sequence correspondingly set forth in SEQ ID NO: 7 or SEQ ID NO: 22, or a sequence having at least 80%, 81%, 82%, 83%, 84%, 85%, 86%, 87%, 88%, 89% or 90%, preferably at least 91%, 92%, 93%, 94%, 95%, 96%, 97%, 98% or 99% sequence identity to the sequence set forth in SEQ ID NO: 7 or 22, or an amino acid sequence having one or more (preferably 1, 2 or 3) conservative amino acid mutations (preferably substitutions, insertions or deletions) compared with the sequences set forth in SEQ ID NO: 7 or 22;
or
the second protein functional region comprises a sequence having an amino acid sequence set forth in SEQ ID NO: 20, or a sequence having at least 80%, 81%, 82%, 83%, 84%, 85%, 86%, 87%, 88%, 89% or 90%, preferably at least 91%, 92%, 93%, 94%, 95%, 96%, 97%, 98% or 99% sequence identity to the sequence set forth in SEQ ID NO: 20, or an amino acid sequence having one or more (preferably 1, 2 or 3) conservative amino acid mutations (preferably substitutions, insertions or deletions) compared with the sequences set forth in SEQ ID NO: 20; and
a sequence having an amino acid sequence set forth in SEQ ID NO: 24, or a sequence having at least 80%, 81%, 82%, 83%, 84%, 85%, 86%, 87%, 88%, 89% or 90%, preferably at least 91%, 92%, 93%, 94%, 95%, 96%, 97%, 98% or 99% sequence identity to the sequence set forth in SEQ ID NO: 24, or an amino acid sequence having one or more (preferably 1, 2 or 3) conservative amino acid mutations (preferably substitutions, insertions or deletions) compared with the sequences set forth in SEQ ID NO: 24.

3. The anti-CD73/anti-PD-1 bispecific antibody according to claim 1 or 2, wherein: the numbers of the first protein functional region and the second protein functional region are independently 1, 2 or more.

4. The anti-CD73/anti-PD-1 bispecific antibody according to any one of claims 1-3, wherein: the first protein functional region and the second protein functional region are linked directly or via a linker; preferably, the linker is (GGGGS)n, and n is a positive integer, e.g., 1, 2, 3, 4, 5 or 6.

5. The anti-CD73/anti-PD-1 bispecific antibody according to any one of claims 1-4, wherein: the first protein functional region and the second protein functional region are independently an immunoglobulin or an antigen-binding fragment, such as a half-antibody, Fab, F(ab')₂ or a single chain fragment variable, preferably, the first protein functional region is an immunoglobulin and the second protein functional region is an antigen-binding fragment; or the first protein functional region is an antigen-binding fragment and the second protein functional region is an immunoglobulin.

6. The anti-CD73/anti-PD-1 bispecific antibody according to any one of claims 1-5, wherein: the N terminus of the heavy chain variable region of the antigen-binding fragment is linked directly (or via a linker) to the C terminus of CH1 of the immunoglobulin, and the N terminus of the light chain variable region of the antigen-binding fragment is linked directly (or via a linker) to the C terminus of the light chain variable region CL of the immunoglobulin; or the N terminus of the heavy chain variable region of the antigen-binding fragment is linked directly (or via a linker) to the C terminus of the light chain variable region CL of the immunoglobulin, and the N terminus of the light chain variable region of the antigen-binding fragment is linked directly (or via a linker) to the C terminus of the heavy chain variable region CH1 of the immunoglobulin. or
the C terminus of the heavy chain variable region of the antigen-binding fragment is linked directly (or via a linker) to the N terminus of the heavy chain of the immunoglobulin, and the C terminus of the light chain variable region of the antigen-binding fragment is linked directly (or via a linker) to the N terminus of the light chain of the immunoglobulin; or the C terminus of the heavy chain variable region of the antigen-binding fragment is linked directly (or via a linker) to the N terminus of the light chain of the immunoglobulin, and the C terminus of the light chain variable region of the antigen-binding fragment is linked directly (or via a linker) to the N terminus of the heavy chain of the immunoglobulin.

7. The anti-CD73/anti-PD-1 bispecific antibody according to any one of claims 1-6, wherein: the antigen-binding fragment is a single chain fragment variable; preferably, the first protein functional region is an immunoglobulin and the second protein functional region is a single chain fragment variable; or the first protein functional region is a single chain fragment variable and the second protein functional region is an immunoglobulin.

8. The anti-CD73/anti-PD-1 bispecific antibody according to claim 7, wherein: the single chain fragment variable is a molecule formed by connecting an antibody heavy chain variable region (V_{H}) and an antibody light chain variable region (V_{L}) via a linker; preferably, the single chain fragment variable has the following structure: NH₂-V_{L}-linker-V_{H}-COOH or NH₂-V_{H}-linker-V_{L}-COOH.

9. The anti-CD73/anti-PD-1 bispecific antibody according to claim 7 or 8, wherein: when the single chain fragment variable is linked to the C terminus of the heavy chain of the immunoglobulin (C_{H}) (or the N terminus of the heavy chain, the C terminus of CH1 of the heavy chain variable region) via a linker, the antibody heavy chain variable region (V_{H}) of the single chain fragment variable is firstly linked, or the antibody light chain variable region (V_{L}) of the single chain fragment variable is firstly linked; preferably, the single chain fragment variablemay have the following structure:
linker-V_{H}-linker-V_{L}-COOH, or, linker-V_{L}-linker-V_{H}-COOH,
preferably,
the heavy chain variable region of the immunoglobulin comprises CDRs having amino acid sequences set forth in SEQ ID NOs: 3-5, and the light chain variable region of the immunoglobulin comprises CDRs having amino acid sequences set forth in SEQ ID NOs: 8-10;
the heavy chain variable region of the single chain fragment variable comprises CDRs having amino acid sequences set forth in SEQ ID NOs: 45-47, and the light chain variable region of the single chain fragment variable comprises CDRs having amino acid sequences set forth in SEQ ID NOs: 50-52,
preferably, when the single chain fragment variable (such as NH₂-V_{L}-linker-V_{H}-COOH or NH₂-V_{H}-linker-V_{L}-COOH)is linked to the C terminus of the heavy chain of the immunoglobulin via a linker, the antibody heavy chain variable region (V_{H}) of the single chain fragment variable comprising CDRs having amino acid sequences set forth in SEQ ID NOs: 45-47 may be firstly linked, or the antibody light chain variable region (V_{L}) of the single chain fragment variable comprising CDRs having amino acid sequences set forth in SEQ ID NOs: 50-52 may be firstly linked,
or preferably,
the heavy chain variable region of the immunoglobulin comprises CDRs having amino acid sequences set forth in SEQ ID NOs: 45-47, and the light chain variable region of the immunoglobulin comprises CDRs having amino acid sequences set forth in SEQ ID NOs: 50-52; and/or,
the heavy chain variable region of the single chain fragment variable comprises CDRs having amino acid sequences set forth in SEQ ID NOs: 3-5, and the light chain variable region of the single chain fragment variable comprises CDRs having amino acid sequences set forth in SEQ ID NOs: 8-10,
wherein, when the single chain fragment variable (such as NH₂-V_{L}-linker-V_{H}-COOH or NH₂-V_{H}-linker-V_{L}-COOH)is linked to the C terminus of the heavy chain of the immunoglobulin via a linker, the antibody heavy chain variable region (V_{H}) of the single chain fragment variable comprising CDRs having amino acid sequences set forth in SEQ ID NOs: 3-5 may be firstly linked, or the antibody light chain variable region (V_{L}) of the single chain fragment variable comprising CDRs having amino acid sequences set forth in SEQ ID NOs: 8-10 may be firstly linked,
preferably,
one immunoglobulin molecule is linked to two single chain fragment variable molecules, and more preferably, the two single chain fragment variable molecules are identical.

10. The anti-CD73/anti-PD-1 bispecific antibody according to claim 1 or 2, wherein: the immunoglobulin is IgG, IgA, IgD, IgE or IgM, preferably IgG, e.g., IgG1, IgG2, IgG3 or IgG4.

11. The anti-CD73/anti-PD-1 bispecific antibody according to claim 1 or 2, wherein: the single chain fragment variable is linked to the C terminus of the heavy chain of the immunoglobulin, preferably, one immunoglobulin molecule is linked to two single chain fragment variable molecules, and more preferably, the two single chain fragment variable molecules are identical.

12. The anti-CD73/anti-PD-1 bispecific antibody according to claim 1 or 2, wherein:
the heavy chain variable region of the immunoglobulin comprises CDRs having amino acid sequences set forth in SEQ ID NOs: 45-47, and the light chain variable region of the immunoglobulin comprises CDRs having amino acid sequences set forth in SEQ ID NOs: 50-52;
and/or,
the heavy chain variable region of the single chain fragment variable comprises CDRs having amino acid sequences set forth in SEQ ID NOs: 3-5, and the light chain variable region of the single chain fragment variable comprises CDRs having amino acid sequences set forth in SEQ ID NOs: 8-10,
preferably, when the single chain fragment variable is linked to the C terminus of the heavy chain of the immunoglobulin via a linker, the antibody heavy chain variable region (V_{H}) of the single chain fragment variable comprising CDRs having amino acid sequences set forth in SEQ ID NOs: 3-5 may be firstly linked, or the antibody light chain variable region (V_{L}) of the single chain fragment variable comprising CDRs having amino acid sequences set forth in SEQ ID NOs: 8-10 may be firstly linked.

13. The anti-CD73/anti-PD-1 bispecific antibody according to claim 1 or 2, wherein:
the heavy chain variable region of the immunoglobulin comprises CDRs having amino acid sequences set forth in SEQ ID NOs: 3-5, and the light chain variable region of the immunoglobulin comprises CDRs having amino acid sequences set forth in SEQ ID NOs: 8-10; and/or,
the heavy chain variable region of the single chain fragment variable comprises CDRs having amino acid sequences set forth in SEQ ID NOs: 45-47, and the light chain variable region of the single chain fragment variable comprises CDRs having amino acid sequences set forth in SEQ ID NOs: 50-52,
wherein, when the single chain fragment variable is linked to the C terminus of the heavy chain of the immunoglobulin via a linker, the antibody heavy chain variable region (V_{H}) of the single chain fragment variable comprising CDRs having amino acid sequences set forth in SEQ ID NOs: 45-47 may be firstly linked, or the antibody light chain variable region (V_{L}) of the single chain fragment variable comprising CDRs having amino acid sequences set forth in SEQ ID NOs: 50-52 may be firstly linked.

14. The anti-CD73/anti-PD-1 bispecific antibody according to claim 1 or 2, wherein:
the heavy chain variable region of the immunoglobulin has an amino acid sequence selected from SEQ ID NO: 44 and SEQ ID NO: 62, and the light chain variable region of the immunoglobulin has an amino acid sequence correspondingly selected from SEQ ID NO: 49 and SEQ ID NO: 64;
and/or,
the heavy chain variable region of the single chain fragment variable has an amino acid sequence selected from SEQ ID NO: 2 and SEQ ID NO: 20, and the light chain variable region of the single chain fragment variable has an amino acid sequence correspondingly selected from SEQ ID NO: 7 and SEQ ID NO: 22; or the heavy chain variable region of the single chain fragment variable has an amino acid sequence set forth in SEQ ID NO: 20, and the light chain variable region of the single chain fragment variable has an amino acid sequence set forth in SEQ ID NO: 24;
wherein, when the single chain fragment variable is linked to the C terminus of the heavy chain of the immunoglobulin via a linker, the antibody heavy chain variable region (V_{H}) of the single chain fragment variable may be firstly linked, or the antibody light chain variable region (V_{L}) of the single chain fragment variable may be firstly linked.

15. The anti-CD73/anti-PD-1 bispecific antibody according to claim 1 or 2, wherein:
the heavy chain variable region of the immunoglobulin has an amino acid sequence selected from SEQ ID NO: 2 and SEQ ID NO: 20, and the light chain variable region of the immunoglobulin has an amino acid sequence selected from SEQ ID NO: 7 and SEQ ID NO: 22; or the heavy chain variable region of the single chain fragment variable has an amino acid sequence set forth in SEQ ID NO. 20, and the light chain variable region of the single chain fragment variable has an amino acid sequence set forth in SEQ ID NO. 24;
and/or,
the heavy chain variable region of the single chain fragment variable has an amino acid sequence selected from SEQ ID NO: 44 and SEQ ID NO: 62, and the light chain variable region of the single chain fragment variable has an amino acid sequence selected from SEQ ID NO: 49 and SEQ ID NO: 64,
wherein, when the single chain fragment variable is linked to the C terminus of the heavy chain via a linker, the antibody heavy chain variable region (V_{H}) of the single chain fragment variable may be firstly linked, or, the antibody light chain variable region (V_{L}) may be linked firstly.

16. The anti-CD73/anti-PD-1 bispecific antibody according to any one of claims 5-14, wherein: the immunoglobulin comprises a non-CDR region, and the non-CDR region is derived from a non-murine species, such as from a human antibody; more preferably, the constant region of the immunoglobulin is humanized; for example, the heavy chain constant region is an Ig gamma-1 chain C region, ACCESSION: P01857; and the light chain constant region is an Ig kappa chain C region, ACCESSION: P01834, or
the heavy chain constant region of the immunoglobulin mutates at any 2 or 3 of positions 234, 235 and 237 based on Ig gamma-1 chain C region, ACCESSION: P01857, and after the mutation, the bispecific antibody has a reduced affinity constant to FcyRIa, FcγRIIIa and/or C1q compared with that before the mutation;
more preferably, according to the EU numbering system, the heavy chain constant region has the following mutations at positions 234, 235 and/or 237 based on Ig gamma-1 chain C region, ACCESSION: P01857:
L234A and L235A;
L234A and G237A;
L235A and G237A;
or
L234A, L235A and G237A,
even more preferably, the heavy chain constant region of the immunoglobulin also has one or more mutations selected from the following mutations:
N297A, D265A, D270A, P238D, L328E, E233D, H268D, P271G, A330R, C226S, C229S, E233P, P331S, S267E, L328F, A330L, M252Y, S254T, T256E, N297Q, P238S, P238A, A327Q, A327G, P329A, K322A, T394D, G236R, G236A, L328R, A330S, P331S, H268A, E318A and K320A,
preferably, the anti-CD73/anti-PD-1 bispecific antibody has a structure shown as heavy chain-light chain-linker 1-scFv, and the scFv is selected from 14C12H1V-linker 2-14C12L1V, 14C12H1V-linker 1-14C12L1V, 14C12H1V-linker 2-14C12L1V and 14C12H1V-linker 1-14C12L1V, particularly selected from the group consisting of:
(1) NTPDV1, of which the heavy chain has an amino acid sequence set forth in SEQ ID NO: 85, the light chain has an amino acid sequence set forth in SEQ ID NO: 28, the linker 1 has an amino acid sequence set forth in SEQ ID NO: 79, 14C12H1V has an amino acid sequence set forth in SEQ ID NO: 66, the linker 2 has an amino acid sequence set forth in SEQ ID NO: 81, and 14C12L1V has an amino acid sequence set forth in SEQ ID NO: 68;
(2) NTPDV2, of which the heavy chain has an amino acid sequence set forth in SEQ ID NO: 85, the light chain has an amino acid sequence set forth in SEQ ID NO: 28, the linker 1 has an amino acid sequence set forth in SEQ ID NO: 79, 14C12H1V has an amino acid sequence set forth in SEQ ID NO: 66, the linker 1 has an amino acid sequence set forth in SEQ ID NO: 79, and 14C12L1V has an amino acid sequence set forth in SEQ ID NO: 68;
(3) NTPDV3, of which the heavy chain has an amino acid sequence set forth in SEQ ID NO: 85, the light chain has an amino acid sequence set forth in SEQ ID NO: 96, the linker 1 has an amino acid sequence set forth in SEQ ID NO: 79, 14C12H1V has an amino acid sequence set forth in SEQ ID NO: 66, the linker 2 has an amino acid sequence set forth in SEQ ID NO: 81, and 14C12L1V has an amino acid sequence set forth in SEQ ID NO: 68; and
(4) NTPDV4, of which the heavy chain has an amino acid sequence set forth in SEQ ID NO: 85, the light chain has an amino acid sequence set forth in SEQ ID NO: 96, the linker 1 has an amino acid sequence set forth in SEQ ID NO: 79, 14C12H1V has an amino acid sequence set forth in SEQ ID NO: 66, the linker 1 has an amino acid sequence set forth in SEQ ID NO: 79, and 14C12L1V has an amino acid sequence set forth in SEQ ID NO: 68.

17. The anti-CD73/anti-PD-1 bispecific antibody according to any one of claims 1-16, wherein: the anti-CD73/anti-PD-1 bispecific antibody binds to CD73 protein and /or PD-1 protein with a K_{D} of less than about 10⁻⁵ M, e.g., less than about 10⁻⁶ M, 10⁻⁷ M, 10⁻⁸ M, 10⁻⁹ M, 10⁻¹⁰ M or less.

18. An isolated nucleic acid molecule comprising a nucleotide sequence capable of encoding a heavy chain variable region of a bispecific antibody, wherein
the heavy chain variable region of the antibody comprises:
a CDR having an amino acid sequence of SEQ ID NOs: 3-5, a CDR having an amino acid sequence of SEQ ID NOs: 45-47, and a CDR having an amino acid sequence of SEQ ID NOs: 50-52;
or
a CDR having an amino acid sequence of SEQ ID NOs: 45-47, a CDR having an amino acid sequence of SEQ ID NOs: 3-5, and a CDR having an amino acid sequence of SEQ ID NOs: 8-10;
and the heavy chain variable region of the bispecific antibody specifically binds to CD73 and PD-1 antigens as a part of the bispecific antibody, and the bispecific antibody further comprises a light chain variable region comprising:
a CDR having an amino acid sequence of SEQ ID NOs: 8-10;
or, a CDR having an amino acid sequence of SEQ ID NOs: 50-52, preferably, the CDRs of the light chain variable region are different from the CDRs of the heavy chain variable region.

19. A vector comprising the isolated nucleic acid molecule according to claim 18.

20. A host cell comprising the isolated nucleic acid molecule c claim 18 or the vector according to claim 19.

21. A method for preparing the anti-CD73/anti-PD-1 bispecific antibody according to any one of claims 1-17, comprising: culturing the host cell according to claim 20 in a suitable condition and isolating the bispecific antibody from the cell cultures.

22. A conjugate comprising the anti-CD73/anti-PD-1 bispecific antibody according to any one of claims 1-17 and a conjugated moiety, wherein the conjugated moiety is a detectable label; specifically, the conjugated moiety is a radioisotope, a fluorescent substance, a chemiluminescent substance, a colored substance or an enzyme.

23. A kit comprising the anti-CD73/anti-PD-1 bispecific antibody according to any one of claims 1-17 or the conjugate according to claim 22, wherein: preferably, the kit further comprises a secondary antibody that specifically recognizes the bispecific antibody; optionally, the secondary antibody further comprises a detectable label, e.g., a radioisotope, a fluorescent substance, a chemiluminescent substance, a colored substance or an enzyme.

24. Use of the anti-CD73/anti-PD-1 bispecific antibody according to any one of claims 1-17 in preparing a kit for detecting the presence or level of CD73 and/or PD-1 in a sample.

25. A pharmaceutical composition comprising the anti-CD73/anti-PD-1 bispecific antibody according to any one of claims 1-17 or the conjugate according to claim 22, wherein: optionally, the pharmaceutical composition further comprises a pharmaceutically acceptable carrier and/or excipient.

26. Use of the anti-CD73/anti-PD-1 bispecific antibody according to any one of claims 1-17 or the conjugate according to claim 22 in preventing and/or treating a tumor or anemia, or in diagnosing a tumor or anemia.

27. Use of the anti-CD73/anti-PD-1 bispecific antibody according to any one of claims 1-17 or the conjugate according to claim 22 in preparing a medicament for preventing and/or treating a tumor or anemia, or in preparing a medicament for diagnosing a tumor or anemia.

28. Use of the anti-CD73/anti-PD-1 bispecific antibody according to any one of claims 1-17 or the conjugate according to claim 22 in preparing the following medicaments:
a medicament for detecting the level of CD73 in a sample,
a medicament for inhibiting the enzyme activity reaction of CD73;
and/or
a medicament for blocking the binding of PD-1 to PD-L1,
a medicament for down-regulating (e.g., down-regulating) the activity or level of PD-1,
a medicament for relieving the immunosuppression of PD-1 in an organism,
a medicament for elevating IL-2 expression in T lymphocytes, or
a medicament for elevating IFN-yexpression in T lymphocytes.

29. An *in vivo* or *in vitro* method comprising: administering to a cell or administering to a subject in need thereof an effective amount of the anti-CD73/anti-PD-1 bispecific antibody according to any one of claims 1-17 or the conjugate according to claim 22.

30. A hybridoma cell line, selected from:
hybridoma cell line LT014 (also called CD73-19F3) deposited at China Center for Type Culture Collection (CCTCC) on June 19, 2018 with an accession number of CCTCC NO: C2018137; or
hybridoma cell line LT003 (also called PD-1-14C12) deposited at China Center for Type Culture Collection (CCTCC) on June 16, 2015 with an accession number of CCTCC NO: C2015105.

31. An anti-CD73 monoclonal antibody, wherein the antibody is 19F3H2L3(hGlTM), and has a heavy chain amino acid sequence set forth in SEQ ID NO: 30 and a light chain amino acid sequence set forth in SEQ ID NO: 28.
